# EUROPEAN PATENT SPECIFICATION

(11) **EP 2 863 736 B1**
(45) Date of publication and mention of the grant of the patent: **11.12.2019**
(21) Application number: 13806511.5
(22) Date of filing: 19.06.2013
(51) Int. Cl.: A01K 67/027, C12N 15/63, C12N 15/85, C12N 5/073, C12N 9/22, C12N 15/877, C12N 15/90

(54) **GENETICALLY EDITED ANIMALS AND METHODS FOR MAKING THE SAME**
GENETISCH MODIFIZIERTE NUTZTIERE UND VERFAHREN ZUR HERSTELLUNG DAVON
ANIMAUX D'ÉLEVAGE GÉNÉTIQUEMENT MODIFIÉS ET PROCÉDÉS DE PRODUCTION ASSOCIÉS

(30) Priority: 21.06.2012 US 201261662767 P; 24.08.2012 US 201213594694
(43) Date of publication of application: 29.04.2015
(73) Proprietor: Recombinetics, Inc., Saint Paul, MN 55104 (US)
(72) Inventor: FAHRENKRUG, Scott, C., Minneapolis, MN 55406 (US); CARLSON, Daniel F., Woodbury, MN 55125 (US)
(74) Representative: dompatent von Kreisler Selting Werner - Partnerschaft von Patent- und Rechtsanwälten mbB
(86) International application number: PCT/US2013/046590
(87) International publication number: WO 2013/192316

(56) References cited:
- WO-A1-2011/002988
- WO-A1-2011/019385
- WO-A1-2011/078665
- WO-A1-2011/154393
- WO-A1-2011/154393
- WO-A2-2012/116274
- US-A1- 2011 023 158
- US-A1- 2011 145 940
- US-A1- 2011 301 073
- US-A1- 2012 030 778
- US-A1- 2012 222 143
- LAURENT TESSON ET AL: "Knockout rats generated by embryo microinjection of TALENs", NATURE BIOTECHNOLOGY, vol. 29, no. 8, 31 March 2011 (2011-03-31) , pages 695-696, XP055145023, ISSN: 1087-0156, DOI: 10.1038/nbt.1940 -& Laurent Tesson ET AL: "Knockout rats generated by embryo microinjection of TALENs", Nature Biotechnology, 1 August 2011 (2011-08-01), pages 1-13, XP055244375, DOI: 10.1038/nbt.1940 Retrieved from the Internet: URL:http://www.nature.com/nbt/journal/v29/ n8/extref/nbt.1940-S1.pdf [retrieved on 2016-01-25]
- MELANIE MEYER ET AL: "Modeling disease mutations by gene targeting in one-cell mouse embryos", PROCEEDINGS OF THE NATIONAL ACADEMY OF SCIENCES, NATIONAL ACADEMY OF SCIENCES, US, vol. 109, no. 24, 12 June 2012 (2012-06-12), pages 9354-9359, XP002681302, ISSN: 0027-8424, DOI: 10.1073/PNAS.1121203109 [retrieved on 2012-06-01]
- FUQIANG CHEN ET AL: "High-frequency genome editing using ssDNA oligonucleotides with zinc-finger nucleases", NATURE METHODS, NATURE PUBLISHING GROUP, GB , vol. 8, no. 9 1 September 2011 (2011-09-01), XP002681301, ISSN: 1548-7091, DOI: 10.1038/NMETH.1653 Retrieved from the Internet: URL:http://www.nature.com/nmeth/journal/v8 /n9/full/nmeth.1653.html [retrieved on 2011-07-17]
- WENFANG (SPRING) TAN ET AL: "Chapter Two - Precision Editing of Large Animal Genomes", ADVANCES IN GENETICS, ACADEMIC PRESS, vol. 80, 1 January 2012 (2012-01-01), pages 37-97, XP009170794, ISSN: 0065-2660, DOI: 10.1016/B978-0-12-404742-6.00002-8
- GEURTS ARON M ET AL: "Knockout Rats via Embryo Microinjection of Zinc-Finger Nucleases", SCIENCE, AMERICAN ASSOCIATION FOR THE ADVANCEMENT OF SCIENCE, US, vol. 325, no. 5939, 24 July 2009 (2009-07-24), page 433, XP002580718, ISSN: 0036-8075, DOI: 10.1126/SCIENCE.1172447
- CARLSON ET AL: "VERSATILE TRANSCRIPTION ACTIVATOR-LIKE EFFECTOR NUCLEASE (TALEN)-MEDIATED ENGINEERING OF OSSABAW MINIATURE SWINE", REPRODUCTION FERTILITY AND DEVELOPMENT, C S I R O PUBLISHING, AU, vol. 25, no. 1, 1 December 2012 (2012-12-01), page 312, XP009188104, ISSN: 1031-3613
- DANIEL F CARLSON ET AL: "Efficient TALEN-mediated gene knockout in livestock", PROCEEDINGS OF THE NATIONAL ACADEMY OF SCIENCES, NATIONAL ACADEMY OF SCIENCES, US, vol. 109, no. 43, 1 October 2012 (2012-10-01), pages 17382-17387, XP002716843, ISSN: 0027-8424, DOI: 10.1073/PNAS.1211446109
- CARLSON, DANIEL F. ET AL.: 'Targeting DNA With Fingers and TALENs' MOLECULAR THERAPY-NUCLEIC ACID vol. 1, 24 January 2012, page E3, XP055182775
- BEDELL, VIVTORIA M. ET AL.: 'In vivo genome editing using a high-efficiency TALEN system' NATURE vol. 491, no. 7422, 23 September 2012, pages 114 - 118, XP055048281

### SEQUENCE LISTING

<110> Recombinetics Inc.
<120> GENETICALLY MODIFIED ANIMALS AND METHODS FOR MAKING THE SAME
<130> 5054.16US02
<140> 13/594,694
   <141> 2012-08-24
<150> 61/446,651
   <151> 2011-02-25
<150> 13/404,662
   <151> 2012-02-24
<160> 148
<170> PatentIn version 3.5
<210> 1
   <211> 71
   <212> DNA
   <213> Bos taurus
<400> 1
   ccctcttcct ttcctccagg gatccctcct acatggcctc ccactggaga agcaacagag 60
   gagcacacag a 71
<210> 2
   <211> 71
   <212> DNA
   <213> Artificial Sequence
<220>
   <223> Indel of sequence 1
<400> 2
   ccctcttcct ttcctccagg gatccctcct acagggactc ccactggaga agcaacagag 60
   gagcacacag a 71
<210> 3
   <211> 66
   <212> DNA
   <213> Artificial Sequence
<220>
   <223> Indel of sequence 1
<400> 3
   ccctcttcct ttcctccagg gatccctcct acctcccact ggagaagcaa cagaggagca 60
   cacaga 66
<210> 4
   <211> 71
   <212> DNA
   <213> Bos taurus
<400> 4
   ccctcttcct ttcctccagg gatccctcct acatggcctc ccactggaga agcaacagag 60
   gagcacacag a 71
<210> 5
   <211> 59
   <212> DNA
   <213> Artificial Sequence
<220>
   <223> Indel of sequence 4
<400> 5
   ccttcttcct ttcctccagg gatccctccc actggagaag caacagagga gcacacaga 59
<210> 6
   <211> 66
   <212> DNA
   <213> Artificial Sequence
<220>
   <223> Indel of sequence 4
<400> 6
   ccctcttcct ttcctccagg gatccctcct acttcccact ggagaagcaa cagaggagca 60
   cacaga 66
<210> 7
   <211> 70
   <212> DNA
   <213> Sus
<400> 7
   gcccccccac acagctgagc ccatgctgat ggagtaccct gaggctataa ctcgcttggt 60
   gacagggtcc 70
<210> 8
   <211> 91
   <212> DNA
   <213> Artificial Sequence
<220>
   <223> Indel of sequence 7
<400> 8
   gcccccccac acagctgagc cacaagtcga gggtctcaga gtcactgagt actgagtgct 60
   gatggagtac cctgaggcta taactcgctt g 91
<210> 9
   <211> 66
   <212> DNA
   <213> Artificial Sequence
<220>
   <223> Indel of sequence 7
<400> 9
   gcccccccac acagctgagc cctgatggag taccctgagg ctataactcg cttggtgaca 60
   gggtcc 66
<210> 10
   <211> 69
   <212> DNA
   <213> Artificial Sequence
<220>
   <223> Indel of sequence 7
<400> 10
   gcccccccac acagctgagc catgctgatg gagtaccctg aggctataac tcgcttggtg 60
   acagggtcc 69
<210> 11
   <211> 65
   <212> DNA
   <213> Artificial Sequence
<220>
   <223> Indel of sequence 7
<400> 11
   gcccccccac acagctgagc ccaatggagt accctgaggc tataactcgc ttggtgacag 60
   ggtcc 65
<210> 12
   <211> 65
   <212> DNA
   <213> Artificial Sequence
<220>
   <223> Indel of sequence 7
<400> 12
   gcccccccac acagctgagc acacaggagt accctgaggc tataactcgc ttggtgacag        60
   ggtcc                                                                    65
<210> 13
   <211> 70
   <212> DNA
   <213> Sus
<400> 13
   gcccccccac acagctgagc ccatgctgat ggagtaccct gaggctataa ctcgcttggt        60
   gacagggtcc                                                               70
<210> 14
   <211> 69
   <212> DNA
   <213> Artificial Sequence
<220>
   <223> Indel of sequence 13
<400> 14
   gcccccccac acagctgacc catgctgatg gagtaccctg aggctataac tcgcttggtg        60
   acagggtcc                                                                69
<210> 15
   <211> 71
   <212> DNA
   <213> Artificial Sequence
<220>
   <223> Indel of sequence 13
<400> 15
   gcccccccac acagctgagc ccattgctga tggagtaccc tgaggctata actcgcttgg        60
   tgacagggtc c                                                             71
<210> 16
   <211> 69
   <212> DNA
   <213> Artificial Sequence
<220>
   <223> Indel of sequence 13
<400> 16
   gcccccccac acagctgagc ccagctgatg gagtaccctg aggctataac tcgcttggtg 60
   acagggtcc 69
<210> 17
   <211> 67
   <212> DNA
   <213> Artificial Sequence
<220>
   <223> Indel of sequence 13
<400> 17
   gcccccccac acagctgagc ccatgatgga gtaccctgag gctataactc gcttggtgac 60
   agggtcc 67
<210> 18
   <211> 69
   <212> DNA
   <213> Artificial Sequence
<220>
   <223> Indel of sequence 13
<400> 18
   gcccccccac acagctgagc ccagctgatg gagtaccctg aggctataac tcgcttggtg 60
   acagggtcc 69
<210> 19
   <211> 65
   <212> DNA
   <213> Artificial Sequence
<220>
   <223> Indel of sequence 13
<400> 19
   gcccccccac acagctgagc ccaatggagt accctgaggc tataactcgc ttggtgacag 60
   ggtcc 65
<210> 20
   <211> 69
   <212> DNA
   <213> Artificial Sequence
<220>
   <223> Indel of sequence 13
<400> 20
   gcccccccac acagctgagc catgctgatg gagtaccctg aggctataac tcgcttggtg 60
   acagggtcc 69
<210> 21
   <211> 69
   <212> DNA
   <213> Artificial Sequence
<220>
   <223> Indel of sequence 13
<400> 21
   gcccccccac acagctgagc catgctgatg gagtaccctg aggctataac tcgcttggtg 60
   acagggtcc 69
<210> 22
   <211> 67
   <212> DNA
   <213> Artificial Sequence
<220>
   <223> Indel of sequence 13
<400> 22
   gcccccccac acagctgagc ccatgatgga gtaccctgag gctataactc gcttggtgac 60
   agggtcc 67
<210> 23
   <211> 69
   <212> DNA
   <213> Artificial Sequence
<220>
   <223> Indel of sequence 13
<400> 23
   gcccccccac acagctgagc ccatgtgatg gagtaccctg aggctataac tcgcttggtg 60
   acagggtcc 69
<210> 24
   <211> 70
   <212> DNA
   <213> Artificial Sequence
<220>
   <223> Indel of sequence 13
<400> 24
   gcccccccac acagctgagc catgcctgat ggagtaccct gaggctataa ctcgcttggt 60
   gacagggtcc 70
<210> 25
   <211> 66
   <212> DNA
   <213> Artificial Sequence
<220>
   <223> Indel of sequence 13
<400> 25
   gcccccccac acagctgagc cctgatggag taccctgagg ctataactcg cttggtgaca 60
   gggtcc 66
<210> 26
   <211> 97
   <212> DNA
   <213> Bos taurus
<400> 26
   tcgccttccc tcttcctttc ctccagggat ccctcctaca tggcctccca ctggagaagc 60
   aacagaggag cacacagaag gcccttctgc aacggaa 97
<210> 27
   <211> 97
   <212> DNA
   <213> Artificial Sequence
<220>
   <223> Indel of sequence 26
<400> 27
   tcgccttccc tcttcctttc ctccagggat ccctcctaca tggcttggcc tcccactgga 60
   gaagcaacag aggagcacac agaaggccct tctgcaa 97
<210> 28
   <211> 106
   <212> DNA
   <213> Artificial Sequence
<220>
   <223> Indel of sequence 26
<400> 28
   tcgccttccc tcttcctttc ctccagggat ccctcctaca tggctcccac tggctcccac 60
   tggagaagca acagaggagc acacagaagg cccttctgca acggaa 106
<210> 29
   <211> 96
   <212> DNA
   <213> Artificial Sequence
<220>
   <223> Indel of sequence 26
<400> 29
   tcgccttccc tcttcctttc ctccagggat ccctcctaca tggctggcct cccactggag 60
   aagcaacaga ggagcacaca gaaggccctt ctgcaa 96
<210> 30
   <211> 92
   <212> DNA
   <213> Artificial Sequence
<220>
   <223> Indel of sequence 26
<400> 30
   tcgccttccc tcttcctttc ctccagggat ccctcctacg cctcccactg gagagcaaca 60
   gaggagcaca cagaggccct tctgcaacgg aa 92
<210> 31
   <211> 91
   <212> DNA
   <213> Artificial Sequence
<220>
   <223> Indel of sequence 26
<400> 31
   tcgccttccc tcttcctttc ctccagggat ccctcctact tcccactgga gaagcaacag 60
   aggagcacac agaggccctt ctgcaacgga a 91
<210> 32
   <211> 89
   <212> DNA
   <213> Artificial Sequence
<220>
   <223> Indel of sequence 26
<400> 32
   tcgccttccc tcttcctttc ctccagggat ccctcctact cccactggag aagcaacaga      60
   ggagcacaca gaggcccttc tgcacggaa                                          89
<210> 33
   <211> 91
   <212> DNA
   <213> Artificial Sequence
<220>
   <223> Indel of sequence 26
<400> 33
   tcgccttccc tcttcctttc ctccagggat ccctcctgct cccactggag aagcaacaga      60
   ggagcacaca gaaggccctt ctgcaacgga a                                       91
<210> 34
   <211> 79
   <212> DNA
   <213> Artificial Sequence
<220>
   <223> Indel of sequence 26
<400> 34
   tcaccttcct tcttcctttc ctccagggat cccactggag aagcaacaga ggagcacaca      60
   gaaggccctt ctgcacgga                                                     79
<210> 35
   <211> 81
   <212> DNA
   <213> Artificial Sequence
<220>
   <223> Indel of sequence 26
<400> 35
   tcgccttccc tcttcctttc ctccaggcct cccactggag aagcaacaga ggagcacaca      60
   gaaggccctt ctgcaacgga a                                                  81
<210> 36
   <211> 71
   <212> DNA
   <213> Artificial Sequence
<220>
   <223> Indel of sequence 26
<400> 36
   tcgccttccc tcttcctttc ctccagggat ccctcacaga ggagcacaca gaaggccctt 60
   ctgcaacgga a 71
<210> 37
   <211> 97
   <212> DNA
   <213> Artificial Sequence
<220>
   <223> Indel of sequence 26
<400> 37
   tcgccttccc tcttcctttc ctccagggat ccctcctaca ttgcctccca ctggagaagc 60
   aacagaggag cacacagaag gcccttctgc aacggaa 97
<210> 38
   <211> 97
   <212> DNA
   <213> Artificial Sequence
<220>
   <223> Indel of sequence 26
<400> 38
   tcgccttccc tcttcctttc ctccagggat ccctcctaca gggatcttcc cactggagaa 60
   gcacagagga gcacacagaa ggcccttctg caacgga 97
<210> 39
   <211> 17
   <212> DNA
   <213> Sus
<400> 39
   ctcctacaag tggattt 17
<210> 40
   <211> 53
   <212> DNA
   <213> Sus
<400> 40
   gacgggaaat gcatctccta caagtgcacg aacagtccct ataaacccct gga 53
<210> 41
   <211> 53
   <212> DNA
   <213> Sus
<400> 41
   gacgggaaat gcatctccta caagtgcacg aacagtccct ataaacccct gga 53
<210> 42
   <211> 26
   <212> DNA
   <213> Sus
<400> 42
   gacgggaaat gcatctccta caagtg 26
<210> 43
   <211> 53
   <212> DNA
   <213> Artificial Sequence
<220>
   <223> Indel of sequence 40
<400> 43
   aaatgcatct cctacaagtg catttttgtt taaattaaga tgtgggagga gaa 53
<210> 44
   <211> 54
   <212> DNA
   <213> Sus
<400> 44
   aaatgcatct cctacaagtg catttttgtt taaattaaga tgtgggaagg agaa 54
<210> 45
   <211> 25
   <212> DNA
   <213> Artificial Sequence
<220>
   <223> Indel of sequence 40
<400> 45
   aaatgcatct cctacaagtg gattt 25
<210> 46
   <211> 29
   <212> DNA
   <213> Artificial Sequence
<220>
   <223> Indel of sequence 40
<400> 46
   catctcctac aagtgcaagg acgggtccg 29
<210> 47
   <211> 29
   <212> DNA
   <213> Artificial Sequence
<220>
   <223> Indel of sequence 40
<400> 47
   catctcctac aagtgcaagg acgggtccg 29
<210> 48
   <211> 53
   <212> DNA
   <213> Artificial Sequence
<220>
   <223> Indel of sequence 40
<400> 48
   catctcctac aagtggattt gtgatgggaa caccgagtgc aaggacgggt ccg 53
<210> 49
   <211> 23
   <212> DNA
   <213> Artificial Sequence
<220>
   <223> Indel of sequence 40
<400> 49
   ctcctacaag gacgggtccg atg 23
<210> 50
   <211> 23
   <212> DNA
   <213> Artificial Sequence
<220>
   <223> Indel of sequence 40
<400> 50
   ctcctacaag gacgggtccg atg 23
<210> 51
   <211> 53
   <212> DNA
   <213> Artificial sequence
<220>
   <223> Indel of sequence 40
<400> 51
   ctcctacaag tggatttgtg atgggaacac cgagtgcaag gacgggtccg atg 53
<210> 52
   <211> 40
   <212> DNA
   <213> Artificial Sequence
<220>
   <223> Indel of sequence 40
<400> 52
   cctacaagtg gtttgtgagt gcaaggacgg gtccgatgag 40
<210> 53
   <211> 40
   <212> DNA
   <213> Artificial Sequence
<220>
   <223> Indel of sequence 40
<400> 53
   cctacaagtg gtttgtgagt gcaaggacgg gtccgatgag 40
<210> 54
   <211> 53
   <212> DNA
   <213> Artificial Sequence
<220>
   <223> Indel of sequence 40
<400> 54
   cctacaagtg gatttgtgat gggaacaccg agtgcaagga cgggtccgat gag 53
<210> 55
   <211> 81
   <212> DNA
   <213> Sus
<400> 55
   wtgccacaca acgactggaa catgcattca acatcgccaa gtatcagtta ggcatagaga 60
   aactactgga tcctgaaggt t 81
<210> 56
   <211> 75
   <212> DNA
   <213> Artificial Sequence
<220>
   <223> Indel of sequence 55
<400> 56
   gccacacaac gactggaaca tgcattcaac atcgccatca gttaggcata gagaaactac 60
   tggatcctga aggtt 75
<210> 57
   <211> 72
   <212> DNA
   <213> Artificial Sequence
<220>
   <223> Indel of sequence 55
<400> 57
   gccacacaac gactggaaca tgcattcaac atcgccagtt aggcatagag aaactactgg 60
   atcctgaagg tt 72
<210> 58
   <211> 69
   <212> DNA
   <213> Artificial Sequence
<220>
   <223> Indel of sequence 55
<400> 58
   gccacacaac gactggaaca tgcattcaac atcagttagg catagagaaa ctactggatc 60
   ctgaaggtt 69
<210> 59
   <211> 74
   <212> DNA
   <213> Artificial Sequence
<220>
   <223> Indel of sequence 55
<400> 59
   gccacacaac gactggaaca tgcattcaac atcgccacag ttaggcatag agaaactact 60
   ggatcctgaa ggtt 74
<210> 60
   <211> 79
   <212> DNA
   <213> Artificial Sequence
<220>
   <223> Indel of sequence 55
<400> 60
   gccacacaac gactggaaca tgcattcaac atcgccaagt atcagttagg catagagaaa 60
   ctactggatc ctgaaggtt 79
<210> 61
   <211> 79
   <212> DNA
   <213> Sus
<400> 61
   gccacacaac gactggaaca tgcattcaac atcgccaagt atcagttagg catagagaaa 60
   ctactggatc ctgaaggtt 79
<210> 62
   <211> 81
   <212> DNA
   <213> Artificial Sequence
<220>
   <223> Indel of sequence 61
<400> 62
   cccacacaac gactggaaca tgcattcaac atcgcataat atatcagtta ggcatagaga 60
   aactactgga tcctgaaggt t 81
<210> 63
   <211> 77
   <212> DNA
   <213> Artificial Sequence
<220>
   <223> Indel of sequence 61
<400> 63
   gccacacaac gactggaaca tgcattcaac atcgccaaat cagttaggca tagagaaact 60
   actggatcct gaaggtt 77
<210> 64
   <211> 69
   <212> DNA
   <213> Artificial Sequence
<220>
   <223> Indel of sequence 61
<400> 64
   gccacacaac gactggaaca tgcattcaac atcagttagg catagagaaa ctactggatc 60
   ctgaaggtt 69
<210> 65
   <211> 79
   <212> DNA
   <213> Artificial Sequence
<220>
   <223> Indel of sequence 61
<400> 65
   gccacacaac gactggaaca tgcattcaac atcgccaagt atcagttagg catagagaaa 60
   ctactggatc ctgaaggtt 79
<210> 66
   <211> 76
   <212> DNA
   <213> Artificial Sequence
<220>
   <223> Indel of sequence 61
<400> 66
   gccacacaac gactggaaca tgcattcaac atcgccaatc agttaggcat agagaaacta 60
   ctggatcctg aaggtt 76
<210> 67
   <211> 75
   <212> DNA
   <213> Artificial Sequence
<220>
   <223> Indel of sequence 61
<400> 67
   gccacacaac gactggaaca tgcattcaac atcgccaagt attaggcata gagaaactac 60
   tggatcctga aggtt 75
<210> 68
   <211> 78
   <212> DNA
   <213> Sus
<400> 68
   gggaaatgca tctcctacaa gtggatttgt gatgggaaca ccgagtgcaa ggacgggtcc 60
   gatgagtccc tggagacg 78
<210> 69
   <211> 82
   <212> DNA
   <213> Artificial Sequence
<220>
   <223> Indel of sequence 68
<400> 69
   gggaaatgca tctcctacaa gtggatttgt gatgggggaa caccgagtgc agggacgggt 60
   cataagagtc cctggagacg tg 82
<210> 70
   <211> 74
   <212> DNA
   <213> Artificial Sequence
<220>
   <223> Indel of sequence 68
<400> 70
   gggaaatgca tctcctacaa gtggatttgt ggaacaccga gtgcaaggac gggtccgatg 60
   agtccctgga gacg 74
<210> 71
   <211> 74
   <212> DNA
   <213> Artificial Sequence
<220>
   <223> Indel of sequence 68
<400> 71
   gggaaatgca tctcctacaa gtggatttgt ggaacaccga gtgcaaggac gggtccgatg 60
   agtccctgga gacg 74
<210> 72
   <211> 74
   <212> DNA
   <213> Artificial Sequence
<220>
   <223> Indel of sequence 68
<400> 72
   gggaaatgca tctcctacaa gtggatttgt ggaacaccga gtgcaaggac gggtccgatg 60
   agtccctgga gacg 74
<210> 73
   <211> 69
   <212> DNA
   <213> Artificial Sequence
<220>
   <223> Indel of sequence 68
<400> 73
   gggaaatgca tctcctacaa gtggatttgc accgagtgca aggacgggtc cgatgagtcc 60
   ctggagacg 69
<210> 74
   <211> 69
   <212> DNA
   <213> Artificial Sequence
<220>
   <223> Indel of sequence 68
<400> 74
   gggaaatgca tctcctacaa gtggacttgc accgagtgca aggacgggtc cgatgagtcc 60
   ctggagacg 69
<210> 75
   <211> 66
   <212> DNA
   <213> Artificial Sequence
<220>
   <223> Indel of sequence 68
<400> 75
   gggaaatgca tctcctacaa gtggatttgt gagtgcaagg acgggtccga tgagtccctg 60
   gagacg 66
<210> 76
   <211> 66
   <212> DNA
   <213> Artificial Sequence
<220>
   <223> Indel of sequence 68
<400> 76
   gggaaatgca tctcctacaa gtggatttgt gagtgcaagg acgggtccga tgagtccctg 60
   gagacg 66
<210> 77
   <211> 54
   <212> DNA
   <213> Artificial Sequence
<220>
   <223> Indel of sequence 68
<400> 77
   gggaaatgca tctcctacaa gtgcaaggac gggtccgatg agtccctgga gacg 54
<210> 78
   <211> 48
   <212> DNA
   <213> Artificial Sequence
<220>
   <223> Indel of sequence 68
<400> 78
   gggaaatgca tctcctacaa ggacgggtcc gatgagtccc tggagaag 48
<210> 79
   <211> 50
   <212> DNA
   <213> sus
<400> 79
   gggaaatgca tctcctacaa gtggacgggt ccgatgagtc cctggagacg 50
<210> 80
   <211> 50
   <212> DNA
   <213> Artificial Sequence
<220>
   <223> Indel of sequence 79
<400> 80
   tgctgatcct ggcactgatc aaggacgggt ccgatgagtc cctggagacg 50
<210> 81
   <211> 50
   <212> DNA
   <213> Artificial Sequence
<220>
   <223> Indel of sequence 79
<400> 81
   gggaaatgca tctcctacaa gtggacgggt ccgatgagtc cctggagacg 50
<210> 82
   <211> 50
   <212> DNA
   <213> Artificial Sequence
<220>
   <223> Indel of sequence 79
<400> 82
   tgctgatcct ggcactgatc aaggacgggt ccgatgagtc cctggagacg 50
<210> 83
   <211> 41
   <212> DNA
   <213> Artificial Sequence
<220>
   <223> Indel of sequence 79
<400> 83
   tagacacagg gagtatggtc acttgctgat tcccaccgag t 41
<210> 84
   <211> 69
   <212> DNA
   <213> Artificial Sequence
<220>
   <223> Indel of sequence 79
<400> 84
   gggaaatgca tctcctacaa gtggatttgc accgagtgca aggacgggtc cgatgagtcc 60
   ctggagacg 69
<210> 85
   <211> 70
   <212> DNA
   <213> Artificial Sequence
<220>
   <223> Indel of sequence 79
<400> 85
   gggaaatgca tctcctacaa gtggatttgt gatgggaaca ccgagtgcaa ggacgggtcc 60
   gatgagtccc 70
<210> 86
   <211> 77
   <212> DNA
   <213> Artificial Sequence
<220>
   <223> Indel of sequence 79
<400> 86
   gggaaatgca tctcctacaa gtgcaaggag tgcaaggacg gaatgcaccg agtgcaagga 60
   cgggtccgat gagtccc 77
<210> 87
   <211> 56
   <212> DNA
   <213> Artificial Sequence
<220>
   <223> Indel of sequence 79
<400> 87
   gggaaatgca tctcctacaa gttgcattcc gctgtgaatt aggatgtggg cggaga 56
<210> 88
   <211> 75
   <212> DNA
   <213> Sus
<400> 88
   gggaaatgca tctcctacaa gtggatttgt gatgacaccg agtgcaagga cgggtccgat 60
   gagtccctgg agacg 75
<210> 89
   <211> 54
   <212> DNA
   <213> Artificial Sequence
<220>
   <223> Indel of sequence 89
<400> 89
   ggctcatttc tcagcttgca gtgcaaggac gggtccggtg agtccctgga gacg 54
<210> 90
   <211> 76
   <212> DNA
   <213> Artificial Sequence
<220>
   <223> Indel of sequence 89
<400> 90
   gggaaatgca tctcctacaa gtggatttgt gatacacacc gagtgcaagg acgggtccga 60
   tgagtccctg gagacg 76
<210> 91
   <211> 62
   <212> DNA
   <213> Sus
<400> 91
   tctataccta ggtcaaaaat gtaatgaaga aagtatcagt taggcataga gaaactactg 60
   ga 62
<210> 92
   <211> 52
   <212> DNA
   <213> Artificial Sequence
<220>
   <223> Indel of sequence 91
<400> 92
   tctataccta ggtcaaaaat gtaattcagt taggcataga gaaactactg ga 52
<210> 93
   <211> 54
   <212> DNA
   <213> Artificial Sequence
<220>
   <223> Indel of sequence 91
<400> 93
   tctataccta ggtcaaaaat gtaatgatca gttaggcata gagaaactac tgga 54
<210> 94
   <211> 62
   <212> DNA
   <213> Artificial Sequence
<220>
   <223> Indel of sequence 91
<400> 94
   tctataccta ggtcaaaaat gtaatgagac aggtatcagt taggcataga gaaactactg 60
   ga 62
<210> 95
   <211> 44
   <212> DNA
   <213> Artificial Sequence
<220>
   <223> Indel of sequence 91
<400> 95
   tctataccta ggtcaaaaat gttaggcata gagaaactac tgga 44
<210> 96
   <211> 43
   <212> DNA
   <213> Artificial Sequence
<220>
   <223> Indel of sequence 91
<400> 96
   tctataccta ggtcaaaaat gtaggcatag agaaactact gga 43
<210> 97
   <211> 51
   <212> DNA
   <213> Artificial Sequence
<220>
   <223> Indel of sequence 91
<400> 97
   tctataccta ggtcaaaaat gtaatcagtt aggcatagag aaactactgg a 51
<210> 98
   <211> 62
   <212> DNA
   <213> Artificial Sequence
<220>
   <223> Indel of sequence 91
<400> 98
   tctataccta ggtcaaaaat gtaatgaaga aagtatcagt taggcataga gaaactactg       60
   ga 62
<210> 99
   <211> 61
   <212> DNA
   <213> Artificial Sequence
<220>
   <223> Indel of sequence 91
<400> 99
   tctataccta ggtcaaaaat gtaatgaaga aatatcagtt aggcatagag aaactactgg 60
   a 61
<210> 100
   <211> 62
   <212> DNA
   <213> Artificial Sequence
<220>
   <223> Indel of sequence 91
<400> 100
   tctataccta ggtcaaaaat gtaatgaatt aaatatcagt taggcataga gaaactactg 60
   ga 62
<210> 101
   <211> 60
   <212> DNA
   <213> Artificial Sequence
<220>
   <223> Indel of sequence 91
<400> 101
   tctataccta ggtcaaaaat gtaatgaaga gtatcagtta ggcatagaga aactactgga 60
<210> 102
   <211> 56
   <212> DNA
   <213> Artificial Sequence
<220>
   <223> Indel of sequence 91
<400> 102
   tctataccta ggtcaaaaat gtaaaggtat cagttaggca tagagaaact actgga 56
<210> 103
   <211> 63
   <212> DNA
   <213> Artificial Sequence
<220>
   <223> Indel of sequence 91
<400> 103
   tctataccta ggtcaaaaat gtaaagaaaa ggggtatcag ttaggcatag agaaactact 60
   gga 63
<210> 104
   <211> 49
   <212> DNA
   <213> Artificial Sequence
<220>
   <223> Indel of sequence 91
<400> 104
   tctataccta ggtcaaaaat tatcagttag gcatagagaa actactgga 49
<210> 105
   <211> 54
   <212> DNA
   <213> Artificial Sequence
<220>
   <223> Indel of sequence 91
<400> 105
   tctataccta ggtcaaaaat gtaagaatca gttaggcata gagaaactac tgga 54
<210> 106
   <211> 81
   <212> DNA
   <213> Sus
<400> 106
   gaaattttct ttctatacct atgtcaaaaa tgtaatgaaa cttggcgatg ttgaatgcat 60
   gttccagtcg ttgtgtggct g 81
<210> 107
   <211> 77
   <212> DNA
   <213> Artificial Sequence
<220>
   <223> Indel of sequence 106
<400> 107
   gaaattttct ttctatacct atgtcaaaaa tgtaatgatg gcgatgttga atgcatgttc 60
   cagtcgttgt gtggctg 77
<210> 108
   <211> 60
   <212> DNA
   <213> Artificial Sequence
<220>
   <223> Indel of sequence 106
<400> 108
   gaaattttct ttctatacct agggcgatgt tgaatgcatg ttccagtcgt tgtgtggctg 60
<210> 109
   <211> 73
   <212> DNA
   <213> Artificial Sequence
<220>
   <223> Indel of sequence 106
<400> 109
   gaaattttct ttctatacct aggtcaaaaa tgtatggcga tgttgaatgc atgttccagt 60
   cgttgtgtgg ctg 73
<210> 110
   <211> 82
   <212> DNA
   <213> Artificial Sequence
<220>
   <223> Indel of sequence 106
<400> 110
   gaaattttct ttctatacct aggtcaaaaa tgtaatgaag acttggcgat gttgaatgca 60
   tgttccagtc gctgtgtggc tg 82
<210> 111
   <211> 86
   <212> DNA
   <213> Artificial Sequence
<220>
   <223> Indel of sequence 106
<400> 111
   gaaattttct ttcgatacct aggtcaaaaa tgtaatgaag atctacttgg cgatgttgaa 60
   tgcatgttcc agtcgttgtg tggctg 86
<210> 112
   <211> 87
   <212> DNA
   <213> Artificial Sequence
<220>
   <223> Indel of sequence 106
<400> 112
   gaaattttct ttctatacct aggtcaaaaa tgtaatgaag atgatacttg gcgatgttga 60
   atgcatgttc cagtcgttgt gtggctg 87
<210> 113
   <211> 79
   <212> DNA
   <213> Artificial Sequence
<220>
   <223> Indel of sequence 106
<400> 113
   ttttcactgt tagtttgttg caatccagcc atgatattca tcagttaggc atagagaaac 60
   tactggatcc tgaaggtta 79
<210> 114
   <211> 84
   <212> DNA
   <213> Artificial Sequence
<220>
   <223> Indel of sequence 106
<400> 114
   ttttcactgt tagtttgttg caatccagcc atgatattct tagtatcagt taggcataga 60
   gaaactactg gatcctgaag gtta 84
<210> 115
   <211> 79
   <212> DNA
   <213> Artificial Sequence
<220>
   <223> Indel of sequence 106
<400> 115
   ttttcactgt tggtttgttg caatccagcc aagatatata tcagttaggc atagagcaac 60
   tactggatcc tgaaggtta 79
<210> 116
   <211> 59
   <212> DNA
   <213> Artificial Sequence
<220>
   <223> Indel of sequence 106
<400> 116
   tgtgatgaac actccacaga atctcgatgc tgtcgttacc ctctaactgt ggattttga 59
<210> 117
   <211> 48
   <212> DNA
   <213> Artificial Sequence
<220>
   <223> Indel of sequence 106
<400> 117
   tgtgacagaa tctcgatgct gtcgttaccc tctaactgtg gattttga 48
<210> 118
   <211> 59
   <212> DNA
   <213> Artificial Sequence
<220>
   <223> Indel of sequence 106
<400> 118
   ttgggcttga ttgtgacaga atctcgatgc tgtcgttacc ctctaactgt ggattttga 59
<210> 119
   <211> 11
   <212> DNA
   <213> Sus scrofa
<400> 119
   atgaacactc c 11
<210> 120
   <211> 33
   <212> DNA
   <213> Sus
<400> 120
   cttgattgtg atgaacactc cacagaatct cga 33
<210> 121
   <211> 22
   <212> DNA
   <213> Artificial Sequence
<220>
   <223> Indel of sequence 120
<400> 121
   cttgattgtg acagaatctc ga 22
<210> 122
   <211> 22
   <212> DNA
   <213> Artificial Sequence
<220>
   <223> Indel of sequence 120
<400> 122
   cttgattgtg acagaatctc ga 22
<210> 123
   <211> 22
   <212> DNA
   <213> Artificial Sequence
<220>
   <223> Indel of sequence 120
<400> 123
   cttgattgtg acagaatctc ga 22
<210> 124
   <211> 22
   <212> DNA
   <213> Artificial Sequence
<220>
   <223> Indel of sequence 120
<400> 124
   cttgattgtg acagaatctc ga 22
<210> 125
   <211> 22
   <212> DNA
   <213> Artificial Sequence
<220>
   <223> Indel of sequence 120
<400> 125
   cttgattgtg acagaatctc ga 22
<210> 126
   <211> 22
   <212> DNA
   <213> Artificial Sequence
<220>
   <223> Indel of sequence 120
<400> 126
   cttgattgtg acagaatctc ga 22
<210> 127
   <211> 22
   <212> DNA
   <213> Artificial Sequence
<220>
   <223> Indel of sequence 120
<400> 127
   cttgattgtg acagaatctc ga 22
<210> 128
   <211> 22
   <212> DNA
   <213> Artificial Sequence
<220>
   <223> Indel of sequence 120
<400> 128
   cttgattgtg acagaatctc ga 22
<210> 129
   <211> 440
   <212> PRT
   <213> Artificial Sequence
<220>
   <223> TALEN
<400> 129
   Met Ala Ser Ser Pro Pro Lys Lys Lys Arg Lys Val Ser Trp Lys Asp
   1               5                   10                  15
   Ala Ser Gly Trp Ser Arg Val Asp Leu Arg Thr Leu Gly Tyr Ser Gln
               20                  25                  30
   Gln Gln Gln Glu Lys Ile Lys Pro Lys Val Arg Ser Thr Val Ala Gln
           35                  40                  45
   His His Glu Ala Leu Val Gly His Gly Phe Thr His Ala His Ile Val
       50                  55                  60
   Ala Leu Ser Gln His Pro Ala Ala Leu Gly Thr Val Ala Val Thr Tyr
   65                  70                  75                  80
   Gln His Ile Ile Thr Ala Leu Pro Glu Ala Thr His Glu Asp Ile Val
                   85                  90                  95
   Gly Val Gly Lys Gln Trp Ser Gly Ala Arg Ala Leu Glu Ala Leu Leu
               100                 105                 110
   Thr Asp Ala Gly Glu Leu Arg Gly Pro Pro Leu Gln Leu Asp Thr Gly
           115                 120                 125
   Gln Leu Val Lys Ile Ala Lys Arg Gly Gly Val Thr Ala Met Glu Ala
       130                 135                 140
   Val His Ala Ser Arg Asn Ala Leu Thr Gly Ala Pro Leu Asn Leu Thr
   145                 150                 155                 160
   Pro Asp Gln Val Val Ala Ile Ala Ser Asn Gly Gly Gly Lys Gln Ala
                   165                 170                 175
   Leu Glu Ser Ile Val Ala Gln Leu Ser Arg Pro Asp Pro Ala Leu Ala
               180                 185                 190
   Ala Leu Thr Asn Asp His Leu Val Ala Leu Ala Cys Leu Gly Gly Arg
           195                 200                 205
   Pro Ala Met Asp Ala Val Lys Lys Gly Leu Pro His Ala Pro Glu Leu
       210                 215                 220
   Ile Arg Arg Val Asn Arg Arg Ile Gly Glu Arg Thr Ser His Arg Val
   225                 230                 235                 240
   Ala Ser Arg Ser Gln Leu Val Lys Ser Phe Leu Glu Glu Lys Lys Ser
                   245                 250                 255
   Glu Leu Arg His Lys Leu Lys Tyr Val Pro His Glu Tyr Ile Glu Leu
               260                 265                 270
   Ile Glu Ile Ala Arg Asn Ser Thr Gln Asp Arg Ile Leu Glu Met Lys
           275                 280                 285
   Val Met Glu Phe Phe Met Lys Val Tyr Gly Tyr Arg Gly Lys His Leu
       290                 295                 300
   Gly Gly Ser Arg Lys Pro Asp Gly Ala Ile Tyr Thr Val Gly Ser Pro
   305                 310                 315                 320
   Ile Asp Tyr Gly Val Ile Val Asp Thr Lys Ala Tyr Ser Gly Gly Tyr
                   325                 330                 335
   Asn Leu Pro Ile Gly Gln Ala Asp Glu Met Gln Arg Tyr Val Glu Glu
               340                 345                 350
   Asn Gln Thr Arg Asn Lys His Ile Asn Pro Asn Glu Trp Trp Lys Val
           355                 360                 365
   Tyr Pro Ser Ser Val Thr Glu Phe Lys Phe Leu Phe Val Ser Gly His
       370                 375                 380
   Phe Lys Gly Asn Tyr Lys Ala Gln Leu Thr Arg Leu Asn His Ile Thr
   385                 390                 395                 400
   Asn Cys Asn Gly Ala Val Leu Ser Val Glu Glu Leu Leu Ile Gly Gly
                   405                 410                 415
   Glu Met Ile Lys Ala Gly Thr Leu Thr Leu Glu Glu Val Arg Arg Lys
               420                 425                 430
   Phe Asn Asn Gly Glu Ile Asn Phe
           435                 440
<210> 130
   <211> 459
   <212> PRT
   <213> Artificial Sequence
<220>
   <223> TALEN
<400> 130
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Arg Gly Val Pro Met Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145 150 155 160
   Glu Ala Val His Ala Trp Arg Asn Ala Leu Thr Gly Ala Pro Leu Asn
   165 170 175
   Leu Thr Pro Glu Gln Val Val Ala Ile Ala Ser Asn Gly Gly Gly Arg
   180 185 190
   Pro Ala Leu Glu Ser Ile Val Ala Gln Leu Ser Arg Pro Asp Pro Ala
   195 200 205
   Leu Ala Ala Leu Thr Asn Asp His Leu Val Ala Leu Ala Cys Leu Gly
   210 215 220
   Gly Arg Pro Ala Leu Asp Ala Val Lys Lys Gly Leu Pro His Ala Pro
   225 230 235 240
   Glu Leu Ile Lys Arg Val Asn Arg Arg Ile Pro Glu Arg Thr Ser His
   245 250 255
   Arg Val Ala Gly Ser Gln Leu Val Lys Ser Phe Leu Glu Glu Lys Lys
   260 265 270
   Ser Glu Leu Arg His Lys Leu Lys Tyr Val Pro His Glu Tyr Ile Glu
   275 280 285
   Leu Ile Glu Ile Ala Arg Asn Ser Thr Gln Asp Arg Ile Leu Glu Met
   290 295 300
   Lys Val Met Glu Phe Phe Met Lys Val Tyr Gly Tyr Arg Gly Lys His
   305 310 315 320
   Leu Gly Gly Ser Arg Lys Pro Asp Gly Ala Ile Tyr Thr Val Gly Ser
   325 330 335
   Pro Ile Asp Tyr Gly Val Ile Val Asp Thr Lys Ala Tyr Ser Gly Gly
   340 345 350
   Tyr Asn Leu Pro Ile Gly Gln Ala Asp Glu Met Gln Arg Tyr Val Glu
   355 360 365
   Glu Asn Gln Thr Arg Asn Lys His Ile Asn Pro Asn Glu Trp Trp Lys
   370 375 380
   Val Tyr Pro Ser Ser Val Thr Glu Phe Lys Phe Leu Phe Val Ser Gly
   385 390 395 400
   His Phe Lys Gly Asn Tyr Lys Ala Gln Leu Thr Arg Leu Asn His Ile
                   405                 410                 415
   Thr Asn Cys Asn Gly Ala Val Leu Ser Val Glu Glu Leu Leu Ile Gly
               420                 425                 430
   Gly Glu Met Ile Lys Ala Gly Thr Leu Thr Leu Glu Glu Val Arg Arg
           435                 440                 445
   Lys Phe Asn Asn Gly Glu Ile Asn Phe Arg Ser
       450                 455
<210> 131
   <211> 4651
   <212> DNA
   <213> Artificial Sequence
<220>
   <223> TALEN
<400> 131
   catgttcttt cctgcgttat cccctgattc tgtggataac cgtattaccg cctttgagtg 60
   agctgatacc gctcgccgca gccgaacgac cgagcgcagc gagtcagtga gcgaggaagc 120
   ggaagagcgc ccaatacgca aaccgaatta accctcacta aagggaagct tgcttgttct 180
   ttttgcagaa gctcagaata aacgctcaac tttggcagat ctaactcgag aaagatattg 240
   tatatatcgt aacaatagga ggttcaacaa tggcttcctc ccctccaaag aaaaagagaa 300
   aggttagttg gaaggacgca agtggttggt ctagagtgga tctacgcacg ctcggctaca 360
   gtcagcagca gcaagagaag atcaaaccga aggtgcgttc gacagtggcg cagcaccacg 420
   aggcactggt gggccatggg tttacacacg cgcacatcgt tgcgctcagc caacacccgg 480
   cagcgttagg gaccgtcgct gtcacgtatc agcacataat cacggcgttg ccagaggcga 540
   cacacgaaga catcgttggc gtcggcaaac agtggtccgg cgcacgcgcc ctggaggcct 600
   tgctcacgga tgcgggggag ttgagaggtc cgccgttaca gttggacaca ggccaacttg 660
   tgaagattgc aaaacgtggc ggcgtgaccg caatggaggc agtgcatgca tcgcgcaatg 720
   cactgacggg tgcccccctg gagacgggcg ccgctacagg gcgcgtccca ttcgccattc 780
   aggctgcgca actgttggga agggcgatcg gtgcgggcct cttcgctatt acgccagctg 840
   gcgaaagggg gatgtgctgc aaggcgatta agttgggtaa cgccagggtt ttcccagtca 900
   cgacgttgta aaacgacggc cagtgagcgc gcgtaatacg actcactata gggcgaattg 960
   ggtaccgggc cccccctcga ggtcctccag cttttgttcc ctttagtgag ggttaattgc 1020
   gcgcttggcg taatcatggt catagctgtt tcctgtgtga aattgttatc cgctcacaat 1080
   tccacacaac atacgagccg gaagcataaa gtgtaaagcc tggggtgcct aatgagtgag 1140
   ctaactcaca ttaattgcgt tgcgctcact gcccgctttc caccggtcgt ctccaacgac 1200
   cacctcgtcg ccttggcctg cctcggcgga cgtcctgcca tggatgcagt gaaaaaggga 1260
   ttgccgcacg cgccggaatt gatcagaaga gtcaatcgcc gtattggcga acgcacgtcc 1320
   catcgcgttg cctctagatc ccagctagtg aaatctgaat tggaagagaa gaaatctgaa 1380
   cttagacata aattgaaata tgtgccacat gaatatattg aattgattga aatcgcaaga 1440
   aattcaactc aggatagaat ccttgaaatg aaggtgatgg agttctttat gaaggtttat 1500
   ggttatcgtg gtaaacattt gggtggatca aggaaaccag acggagcaat ttatactgtc 1560
   ggatctccta ttgattacgg tgtgatcgtt gatactaagg catattcagg aggttataat 1620
   cttccaattg gtcaagcaga tgaaatgcaa agatatgtcg aagagaatca aacaagaaac 1680
   aagcatatca accctaatga atggtggaaa gtctatccat cttcagtaac agaatttaag 1740
   ttcttgtttg tgagtggtca tttcaaagga aactacaaag ctcagcttac aagattgaat 1800
   catatcacta attgtaatgg agctgttctt agtgtagaag agcttttgat tggtggagaa 1860
   atgattaaag ctggtacatt gacacttgag gaagtgagaa ggaaatttaa taacggtgag 1920
   ataaactttt aataggctag tgactgacta ggatctggtt accactaaac cagcctcaag 1980
   aacacccgaa tggagtctct aagctacata ataccaactt acacttacaa aatgttgtcc 2040
   cccaaaatgt agccattcgt atctgctcct aataaaaaga aagtttcttc acattctaaa 2100
   aaaaaaaaaa aaaaaaaaaa aaaaaaaccc cccccccccc cccccccccc cccccccgca 2160
   tgcctgcagg tcgactagga tccccgggta ccgagctcca attcgcccta tagtgagtcg 2220
   tattacaatt cactggccgt cgttttacaa cgtcgtgact gggaaaaccc tggcgttacc 2280
   caacttaatc gccttgcagc acatccccct ttcgccagct ggcgtaatag cgaagaggcc 2340
   cgcaccgatc gcccttccca acagttgcgc agcctgaatg gcgaatggaa attgtaagcg 2400
   ttaatatttt gttaaaattc gcgttaaatt tttgttaaat cagctcattt tttaaccaat 2460
   aggccgaaat cggcaaaatc ccttataaat caaaagaata gaccgagata gggttgagtg 2520
   ttgttccagt ttggaacaag agtccactat taaagaacgt ggactccaac gtcaaagggc 2580
   gaaaaaccgt ctatcagggc gatggcccac tacgtgaacc atcaccctaa tcaagttttt 2640
   tggggtcgag gtgccgtaaa gcactaaatc ggaaccctaa agggagcccc cgatttagag 2700
   cttgacgggg aaagccggcg aacgtggcga gaaaggaagg gaagaaagcg aaaggagcgg 2760
   gcgctagggc gctggcaagt gtagcggtca cgctgcgcgt aaccaccaca cccgccgcgc 2820
   ttaatgcgcc gctacagggc gcgtcaggtg gcacttttcg gggaaatgtg cgcggaaccc 2880
   ctatttgttt atttttctaa atacattcaa atatgtatcc gctcatgaga caataaccct 2940
   gataaatgct tcaataatat tgaaaaagga agagtatgag tattcaacat ttccgtgtcg 3000
   cccttattcc cttttttgcg gcattttgcc ttcctgtttt tgctcaccca gaaacgctgg 3060
   tgaaagtaaa agatgctgaa gatcagttgg gtgcacgagt gggttacatc gaactggatc 3120
   tcaacagcgg taagatcctt gagagttttc gccccgaaga acgttttcca atgatgagca 3180
   cttttaaagt tctgctatgt ggcgcggtat tatcccgtat tgacgccggg caagagcaac 3240
   tcggtcgccg catacactat tctcagaatg acttggttga gtactcacca gtcacagaaa 3300
   agcatcttac ggatggcatg acagtaagag aattatgcag tgctgccata accatgagtg 3360
   ataacactgc ggccaactta cttctgacaa cgatcggagg accgaaggag ctaaccgctt 3420
   ttttgcacaa catgggggat catgtaactc gccttgatcg ttgggaaccg gagctgaatg 3480
   aagccatacc aaacgacgag cgtgacacca cgatgcctgt agcaatggca acaacgttgc 3540
   gcaaactatt aactggcgaa ctacttactc tagcttcccg gcaacaatta atagactgga 3600
   tggaggcgga taaagttgca ggaccacttc tgcgctcggc ccttccggct ggctggttta 3660
   ttgctgataa atctggagcc ggtgagcgtg ggtctcgcgg tatcattgca gcactggggc 3720
   cagatggtaa gccctcccgt atcgtagtta tctacacgac ggggagtcag gcaactatgg 3780
   atgaacgaaa tagacagatc gctgagatag gtgcctcact gattaagcat tggtaactgt 3840
   cagaccaagt ttactcatat atactttaga ttgatttaaa acttcatttt taatttaaaa 3900
   ggatctaggt gaagatcctt tttgataatc tcatgaccaa aatcccttaa cgtgagtttt 3960
   cgttccactg agcgtcagac cccgtagaaa agatcaaagg atcttcttga gatccttttt 4020
   ttctgcgcgt aatctgctgc ttgcaaacaa aaaaaccacc gctaccagcg gtggtttgtt 4080
   tgccggatca agagctacca actctttttc cgaaggtaac tggcttcagc agagcgcaga 4140
   taccaaatac tgtccttcta gtgtagccgt agttaggcca ccacttcaag aactctgtag 4200
   caccgcctac atacctcgct ctgctaatcc tgttaccagt ggctgctgcc agtggcgata 4260
   agtcgtgtct taccgggttg gactcaagac gatagttacc ggataaggcg cagcggtcgg 4320
   gctgaacggg gggttcgtgc acacagccca gcttggagcg aacgacctac accgaactga 4380
   gatacctaca gcgtgagcta tgagaaagcg ccacgcttcc cgaagggaga aaggcggaca 4440
   ggtatccggt aagcggcagg gtcggaacag gagagcgcac gagggagctt ccagggggaa 4500
   acgcctggta tctttatagt cctgtcgggt ttcgccacct ctgacttgag cgtcgatttt 4560
   tgtgatgctc gtcagggggg cggagcctat ggaaaaacgc cagcaacgcg gcctttttac 4620
   ggttcctggc cttttgctgg ccttttgctc a 4651
<210> 132
   <211> 22
   <212> PRT
   <213> Artificial Sequence
<220>
   <223> TALEN N-terminal leader sequence
<400> 132
   Met Ala Ser Ser Pro Pro Lys Lys Lys Arg Lys Val Ser Trp Lys Asp
   1               5                   10                  15
   Ala Ser Gly Trp Ser Arg
               20
<210> 133
   <211> 75
   <212> DNA
   <213> Bos taurus
<400> 133
   ggagagattt tgggcttgat tgtgacagaa tctgatgctg tcgttaccct ctaactgtgg       60
   attttgaagc ttttg                                                        75
<210> 134
   <211> 76
   <212> DNA
   <213> Bos taurus
<400> 134
   caaaagcttc aaaatccaca gttagagggt aacgacagca tcgagattct gtcacaatca       60
   agcccaaaat ctctcc                                                       76
<210> 135
   <211> 34
   <212> DNA
   <213> Bos taurus
<400> 135
   actccacaga atctcgatgc tctaactgtg gatt 34
<210> 136
   <211> 39
   <212> DNA
   <213> Bos taurus
<400> 136
   actccacaga atctcgatgc taccctctaa ctgtggatt 39
<210> 137
   <211> 28
   <212> DNA
   <213> Bos taurus
<400> 137
   actccacaga atcctctaac tgtggatt 28
<210> 138
   <211> 41
   <212> DNA
   <213> Bos taurus
<400> 138
   actccacaga atctcgatgc tgttacctct aactgtggat t 41
<210> 139
   <211> 44
   <212> DNA
   <213> Bos taurus
<400> 139
   actccacaga atctcgatgc ttcgttaccc tctaactgtg gatt 44
<210> 140
   <211> 49
   <212> DNA
   <213> Sus scrofa
<400> 140
   ctcctacaag tggattgtga tgggaacacc gagtgcaagg acgggtccg 49
<210> 141
   <211> 50
   <212> DNA
   <213> Sus scrofa
<400> 141
   ctcctacaag tggatttgtg atgggaacac cgagtgcaag gacgggtccg 50
<210> 142
   <211> 47
   <212> DNA
   <213> Sus scrofa
<400> 142
   ctcctacaag tggatttgtg ggaacaccga gtgcaaggac gggtccg 47
<210> 143
   <211> 37
   <212> DNA
   <213> Sus scrofa
<400> 143
   agggagtatg gtcacaccga gtgcaaggac gggtccg 37
<210> 144
   <211> 40
   <212> DNA
   <213> Sus scrofa
<400> 144
   ctcctacaag tggatttgtg atggggcaag gacgggtccg 40
<210> 145
   <211> 51
   <212> DNA
   <213> Sus scrofa
<400> 145
   ctcctacaag tggatttgtg atgggaaaca ccgagtgcaa ggacgggtcc g 51
<210> 146
   <211> 65
   <212> DNA
   <213> Bos taurus
<400> 146
   ggccaattac tgctctggag agtatgaatt cgtattttta caaaaatacc ctcacactca 60
   tcttg 65
<210> 147
   <211> 45
   <212> DNA
   <213> Bos taurus
<400> 147
   actccacaga atctcgatgc tgtcgttacc ctctaactgt ggatt 45
<210> 148
   <211> 42
   <212> DNA
   <213> Bos taurus
<400> 148
   actccacaga atctcgatgc tgttaccctc taactgtgga tt 42

## Description

### CROSS REFERENCE TO RELATED APPLICATIONS

This patent application claims priority to U.S. Patent Application No. 13/594,694 filed August 24, 2012 and U.S. Provisional No. 61/662,767 filed June 21, 2012.

### STATEMENT AS TO FEDERALLY SPONSORED RESEARCH

Portions of certain inventive embodiments were made with government support under grant nos. 1R41HL108440-01 "Development of Porcine Genetic Models of Atherosclerosis" awarded by the National Institutes of Health. The United States government has certain rights in the invention.

### TECHNICAL FIELD

The technical field relates to methods of making genetically modified non-human animals, for example, livestock animals with functional traits, and is also directed to *in vitro* methods for making modified cells.

### BACKGROUND

Transcription activator-like (TAL) effector sequences can be assembled to bind DNA targets with specificity by assembling sequences of repeat variable-diresidue (RVDs). Fusion proteins of TAL effectors and nucleases (TALENs) can make targeted double-stranded breaks in cellular DNA that can be used to make specific genetic modifications to cells. TALENs have been reported as useful to generate stably modified human embryonic stem cell and induced pluripotent stem cell clones, to generate knockout *C*. *elegans,* and knockout zebrafish.

### SUMMARY

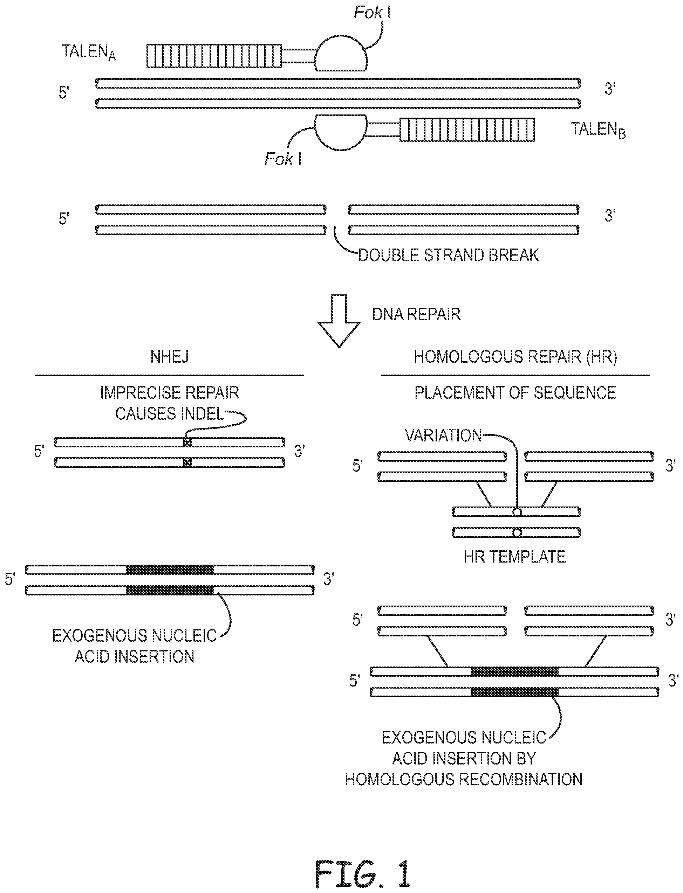

Transcription Activator-Like (TAL) effectors (TALEs) fused with nucleases (TALENs) have been reported for use in genetic modification of cells. These reports have generally focused on plant cells, transformed (immortalized) animal cell lines, or mouse embryonic stem cells. Fig. 1 depicts some of the features of a TALEN-based genetic engineering system.

A first embodiment of the invention is a method of making a genetically edited livestock animal, said method comprising exposing embryos, primary somatic cells, stem cells, germ cells to a single-stranded DNA (ssDNA) homology-dependent repair (HDR) template and an mRNA encoding a TALEN, with the TALEN specifically binding to a target chromosomal site in the embryos or cells, cloning the cells in a surrogate mother or using the embryo for creating a genetically edited animal, with the surrogate mother gestating an animal that is genetically edited at the target chromosomal site, the method being performed without a reporter gene.

An second embodiment of the invention is a method of making a genetically edited non-human animal cell or genetically edited non-human animal embryo comprising exposing embryos or cells of the animal in vitro to a single-stranded DNA (ssDNA) homology-dependent repair (HDR) template and an mRNA encoding a TALEN, with the TALEN specifically binding to a targeted chromosomal site in the embryos or cells, with the cells or embryos being genetically modified only at the targeted chromosomal site and with the method being performed without a reporter gene.

A disclosure is a method of creating a genetic modification comprising exposing a non-human primary cell in an *in vitro* culture or a non-human embryo to a nucleic acid encoding a TALEN, wherein the nucleic acid encodes an N-terminal leader portion having at least 80% homology to SEQ ID NO:132.

Further disclosures are directed to one of more of these methods, or other materials and methods herein comprising one or more of the following: Exposing the embryos to the TALEN without a reporter gene, with more than about 1% of the embryos incorporating the modification at the targeted chromosomal site; providing embryos having genetics known to be capable of expressing a set of traits and exposing the embryos to the TALEN without a reporter gene and screening the gestated animal for the modification and for expression of the set of traits; comprising exposing the cells to the TALEN without a reporter gene, and cloning the cells, with more than 1% of the cloned cells providing animals incorporating the modification at the targeted chromosomal site comprising exposing the cells to the TALEN without a reporter gene, creating colonies of clonal cells, and testing a subset of members of the colonies to identify colonies incorporating the modification at the targeted chromosomal site; wherein testing the subset of members of the colonies is a destructive process; wherein the testing process is chosen from the group consisting of a nucleolytic assay, sequencing, PAGE, PCR, primer extension, or hybridization; wherein the genetic modification is chosen from the group consisting of an insertion, deletion, inversion or translocation; wherein the TALEN is a first TALEN and the targeted chromosomal site is a first site, with the method further comprising a second TALEN directed to a second targeted chromosomal site; comprising exposing the embryos or cells to single stranded DNA (ssDNA) that contains an exogenous sequence, with the genetic modification comprising the exogenous sequence; wherein the exogenous sequence comprises an alternative allele for the TALEN targeted chromosomal site; wherein the alternative allele is linked to a quantitative trait or qualitative trait.; wherein the alternative allele comprises a myostatin allele present in *Belgian Blue* cattle; wherein the cell or embryo belongs to a first breed and the allele belongs to a second breed of the animal; wherein the first breed is *Wagyu or Nelore* cattle and the second breed is *Belgian Blue* cattle, with the offspring being a *Wagyu* or *Nelore* calf; wherein the allele is chosen from the group consisting of an insertion, a deletion, a polymorphism, and a single nucleotide polymorphism; wherein the targeted chromosomal site is chosen for a disruption of a gene & the disruption of the gene comprises an insertion, deletion, or substitution of one or more bases in a sequence encoding the gene and/or a cis-regulatory element thereof; wherein the genetic modification is chosen from the group consisting of an insertion, a deletion, a change to an exogenous nucleic acid sequence, an inversion, a translocation, a gene conversion to natural allele, a gene conversion to a synthetic allele, interspecies allele migration, intraspecies allele migration, and a gene conversion to a novel allele; comprising delivering a recombinase to the cell or embryo; wherein the TALEN mRNA is directly introduced into the cell as mRNA; wherein the direct introduction into the cell comprises a method chosen from the group consisting of electroporation, transfection, lipofection, liposome, nucleofection, biolistic particles, nanoparticles, lipid transfection, electrofusion, and direct injection; wherein the TALEN mRNA is introduced into the cell as a plasmid that encodes the mRNA; wherein the ssDNA is introduced into the cell after a vector encoding a TALEN is introduced into the cell; wherein ssDNA is introduced into the cell between about 8 hours and about 3 days after the vector expressing a TALEN is introduced into the cell; wherein TALEN mRNA is directly introduced into the cell at about the same time as the ssDNA; wherein the cell is a primary cell or stem cell and the method is performed without a selection step that requires either a positive or a negative survival selection criterion; wherein the gestated animal is chosen from the group consisting of swine, cows, sheep, goats, chickens, rabbits, fish, zebrafish, dog, mouse, cat, mouse, rat, and laboratory animal; wherein the cell is chosen from the group consisting of a livestock cell, an artiodactyl cell, a cultured cell, a primary cell, a primary somatic cell, a zygote, a primordial germ cell, a stem cell, and a zygote, or wherein the embryo is a blastocyst; wherein the TALEN is a right TALEN and further comprising a left TALEN that is introduced with the right TALEN; with the cells being primary somatic cells or stem cells; with the cells being cloned by somatic cell nuclear transfer or chromatin transfer; and wherein the gestated animal is homozygous for the modification; wherein the gestated animal is a founder animal.

Further disclosures are directed to an organism (a genetically modified animal, a genetically modified founder animal, or a genetically modified cell) prepared according to one or more of these methods.

The following patent applications explain the technology: US 2010/0146655, US 2010/0105140, US 2011/0059160, and US 2011/0197290.
Laurent Tesson et al., Nature Biotechnology 29 (2011) 695 discloses Talen generated knockout rats.

### BRIEF DESCRIPTION OF THE DRAWINGS

Fig. 1 is an illustration of a TALEN and genetic modifications caused by the same.

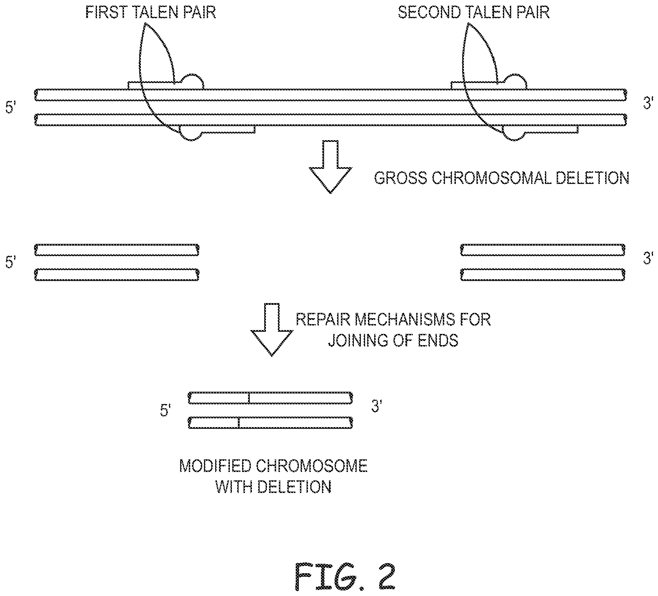

Fig. 2 is an illustration of TALENs operating at a plurality of DNA loci.

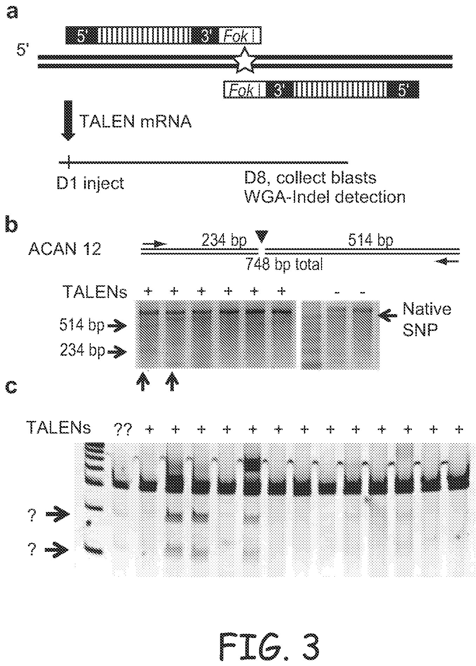

Fig. 3. TALEN activity in bovine embryos. An experimental overview is given in panel (a). TALENs are designed to opposing strands of the DNA target such that the *Fok*I nuclease homodimeric monomers are able to dimerize and cleave DNA between the two monomers. Bovine *in vitro*-produced zygotes are injected with TALEN mRNA on day 1 (D1) and cultured *in vitro* to blastocyst formation. Individual blastocysts (blasts) are collected on day 8, subjected to whole genome amplification (WGA) and analyzed for indels by PCR amplification and Cel-I (SURVEYOR Nuclease, Transgenomics) treatment. Panel b) SURVEYOR Nuclease treatment for analysis of indels in bovine embryos mediated by ACAN12 TALENs. The amplicon length and predicted SURVEYOR cleavage products that are indicative of indels, is shown above. Panel c) SURVEYOR Nuclease treatment for analysis of indels in porcine zygotes mediated by p65 TALENs.

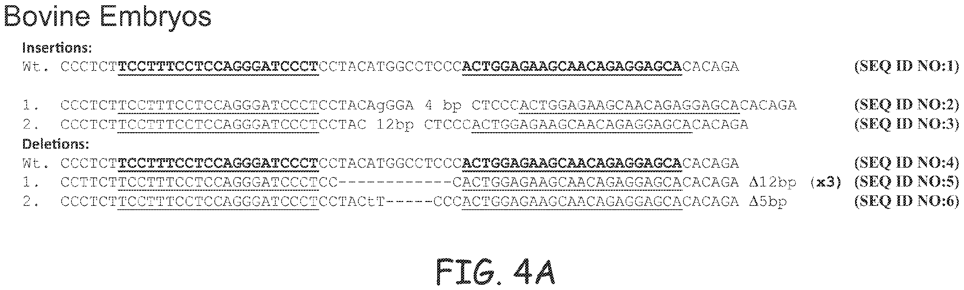

Fig. 4A. Deletions and insertions sequenced from bovine embryos treated with ACAN12 TALENs. The wild-type sequence is shown with TALEN binding sites underlined. Both deletion and insertion events were identified.

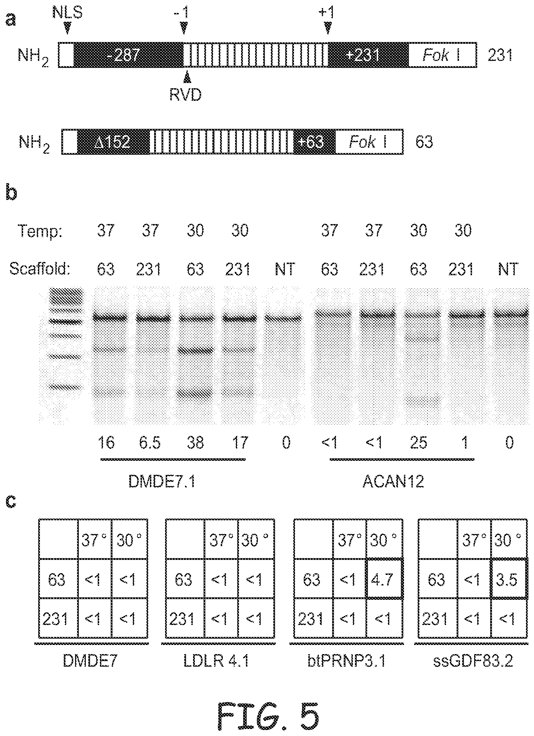

Fig. 5. Comparison of TALEN scaffold for gene editing in livestock fibroblasts Panel a) A diagram of TALEN scaffolds tested in this experiment. Each scaffold (+231, Christian *et. al.* 2010 and Carlson +63, (compare to: Miller *et. al.* 2011)) contains a SV40 nuclear localization signal (NLS) and has a C-terminal fusion of the *Fok*I homodimer domain. Numbering is relative to the DNA binding domain. The amino acid prior to the first repeat variable diresidue repeat (RVD) is labeled "-1" and the amino acid following the last RVD repeat is labeled "+1". Panel b) The SURVEYOR assay was conducted on fibroblasts transfected with either DMDE7.1 or ACAN12 TALEN pairs. Scaffold and temperature treatment is indicated above the gel and percent NHEJ is indicated below. Abbreviations, NT = not treated. Panel c) Activity of four additional TALEN pairs with either the +231 or Carlson +63 scaffold.

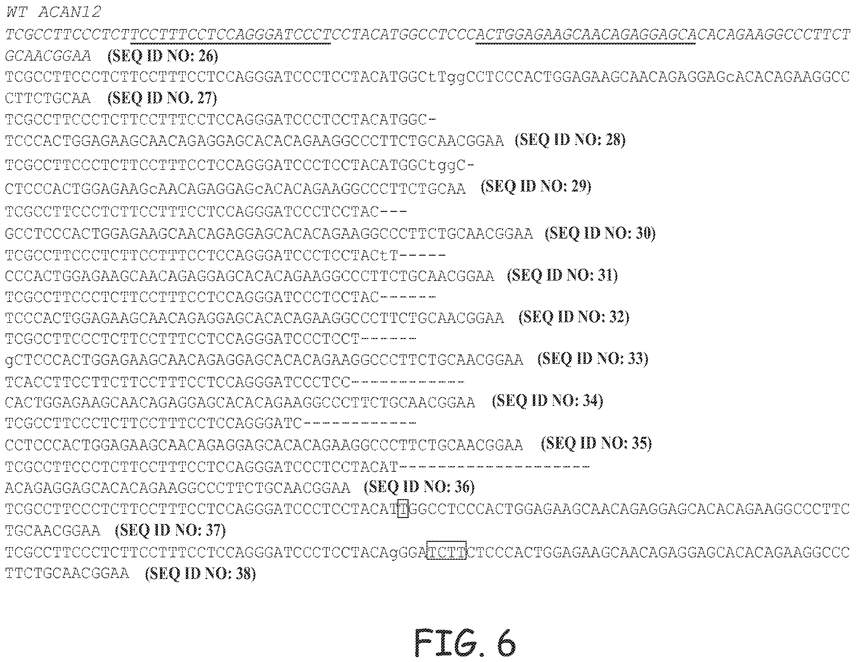

Fig.6. Deletions and insertions sequenced from cells treated with ACAN12 TALENs. The wild-type ACAN12 sequence is displayed in italics and the left and right (complimentary) TALEN-recognition sequences are underlined. Inserted nucleotides are highlighted in grey and mismatch nucleotides are denoted by lower-case text.

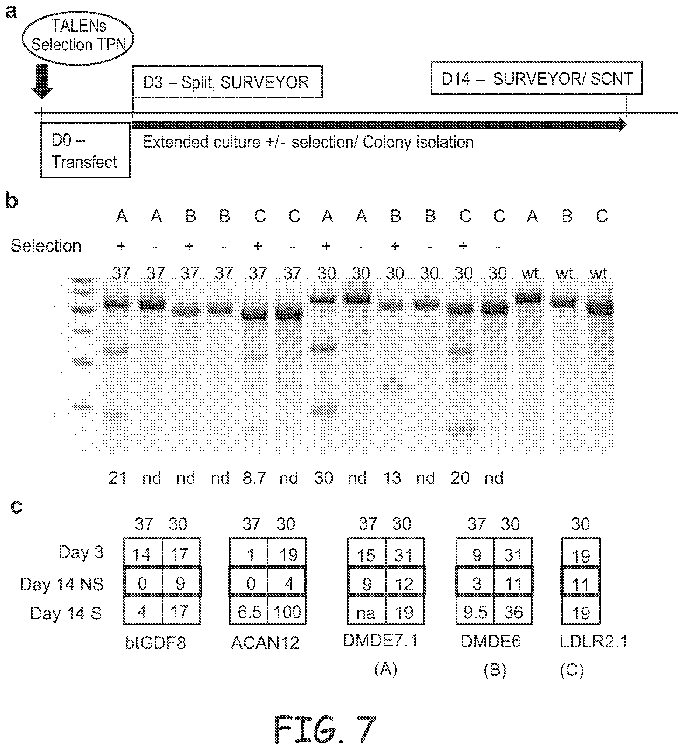

Fig. 7. Transposon co-selection for indel enrichment. An experimental timeline is shown in panel (a). Day zero (D0), cells are transfected with a mixture of plasmids including an expression cassette for each TALEN, a transposon encoding a selection marker, and a transposase-expression cassette. The TALEN plasmid is the major component (4-fold excess by mass) of each transfection. Transfected cells are cultured for 3 days at either 30 or 37 degrees Celsius prior to splitting, collection of a sample for SURVEYOR assay and re-plating for extended culture +/- selection for transposon integration. All cells are cultured at 37 degrees Celsius after day 3. Cells cultured for 14+ days are collected for SURVEYOR assay and cryopreserved for downstream applications, i.e., Single-Cell Nuclear Transfer. Panel b) Fibroblasts were transfected using cationic-lipids. No activity was observed at day 3 (due to low transfection efficiency) so only data for day 14+ populations. Temperature treatment, selection and TALEN id (identified by letters A-C as indicated in panel (c)) are shown above the gel. Panel c) Fibroblasts were transfected by Nucleofection and percent NHEJ was measured at day 3, and in day 14+ non-selected (NS) and selected (S) populations. Temperature treatment is indicated above each matrix. Abbreviations: nd = not detected; wt = wild type amplicon, SURVEYOR treated.

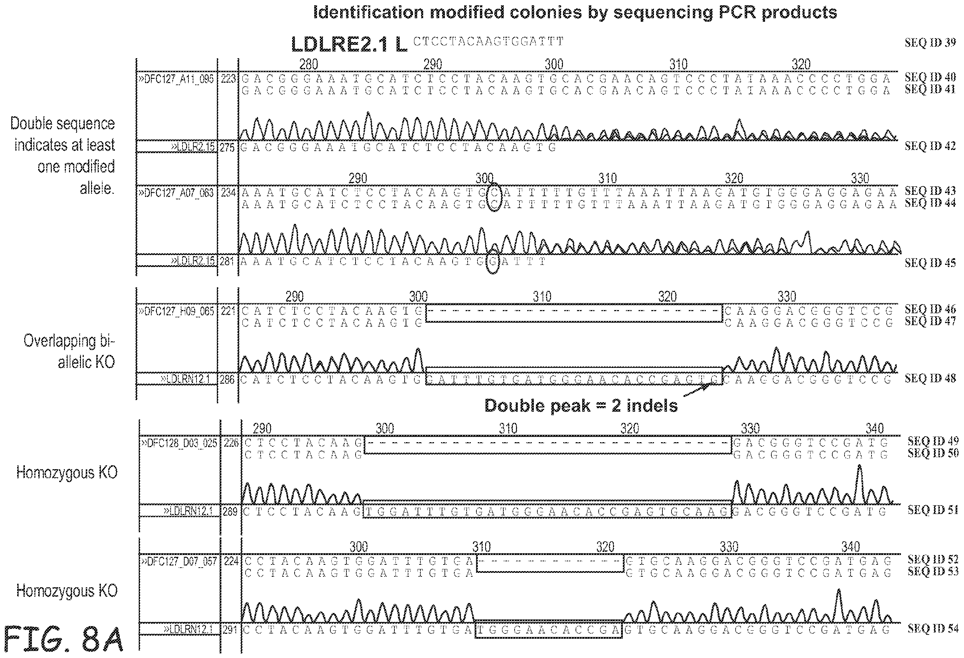

Fig. 8A. Direct PCR sequencing for identification of indels. PCR amplicons from individual fibroblast colonies were purified, sequenced and compared to the wild-type sequence. Mutation of one allele, or, non-overlapping mutations of both alleles will result in double sequence near the TALEN recognition sites (top). Overlapping bi-allelic mutations can be identified where differences between each allele can be identified by double peaks flanking the mutation site. Colonies with homozygous mutations do not display double peaks near the indel site.

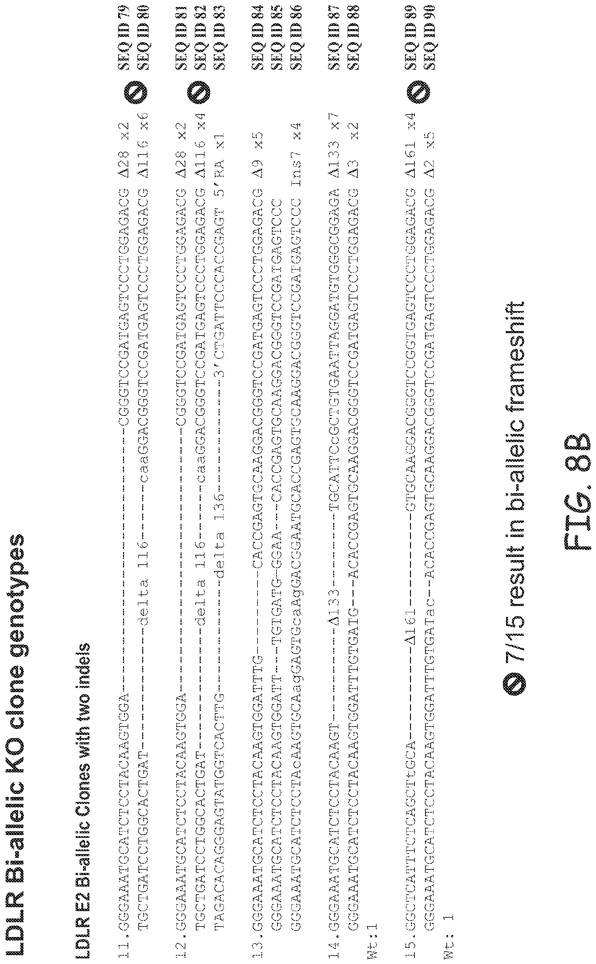

Fig. 8B. Sequence comparisons of wild-type and bi-allelic clones with homozygous indels, as in Fig. 8A.

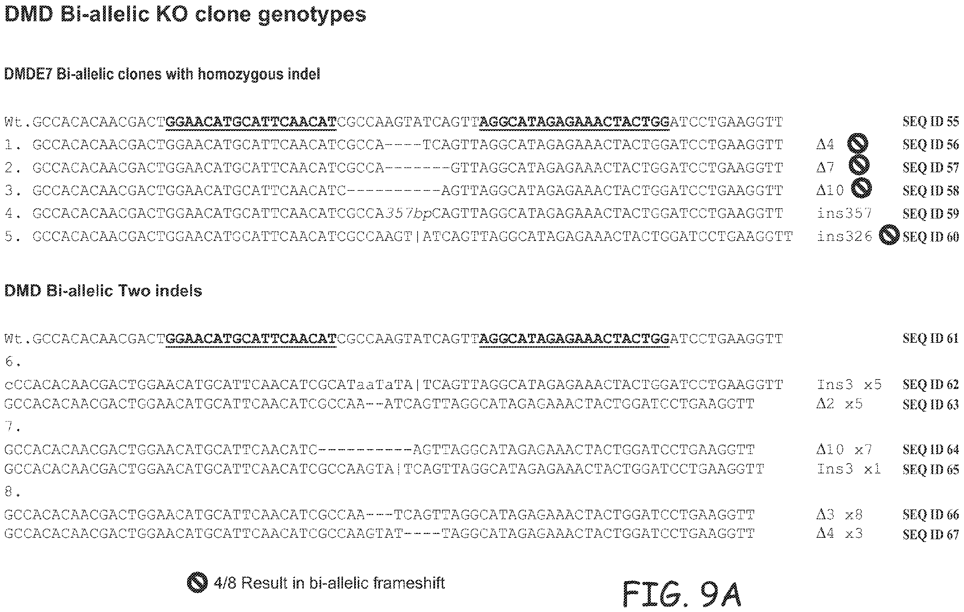

Fig. 9A. DMD Bi-allelic modification alleles. Colonies with either homozygous modification alleles (i.e., both alleles harbor the same mutation) or bi-allelic mutation with different mutations on each allele are displayed. For colonies with two indels, the number of times each allele was sequenced is displayed on the right. In some cases, a third mutation or single wild-type allele was sequenced, indicating that not all colonies are 100% clonal. Frame-shift alleles are indicated and mismatch nucleotides are denoted by lower-case text.

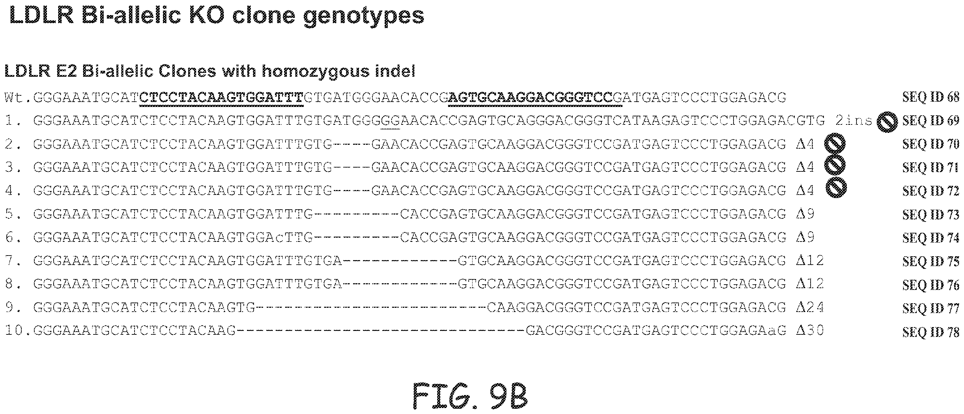

Fig. 9B. LDLR Bi-allelic modification alleles, with notations as in Fig. 9A.

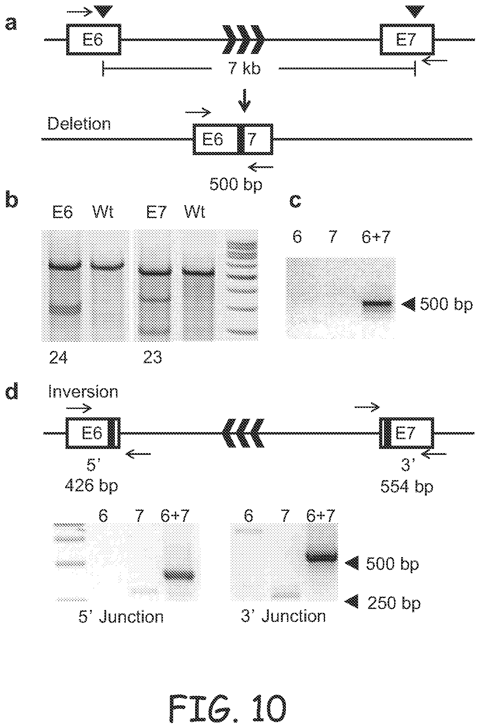

Fig. 10. TALEN-induced deletions and inversions. A schematic of the DMD locus is shown in panel (a). DNA orientation is denoted by black chevrons. TALENs targeted to exons 6 and 7 (black arrowheads) co-transfected into male pig fibroblasts could result in a NHEJ fusion event between exons 6 and 7. This could be identified using primers (black arrows) resulting in ∼500 bp amplicon. Panel b) SURVEYOR assay of cells transfected simultaneously with TALENs targeted to exons 6 and 7 reveal NHEJ indels at both sites. Percent NHEJ is displayed below. Panel c) PCR with primers flanking the presumptive deletion site yield a ∼500 basepair product when both exon-6 and exon-7 TALENs are introduced simultaneously, but not when transfected singly. Panel d) The predicted outcome of an inversion event of the sequence between the TALEN target sites is shown. DNA orientation is denoted by black chevrons. Primers outside the presumptive flanking sites at the 5' and 3' end of the inversion locus are shown (black arrows) along with predicted product size. PCR products were observed at both 5' and 3' junctions only when both exon-6 and exon-7 TALENs are introduced simultaneously.

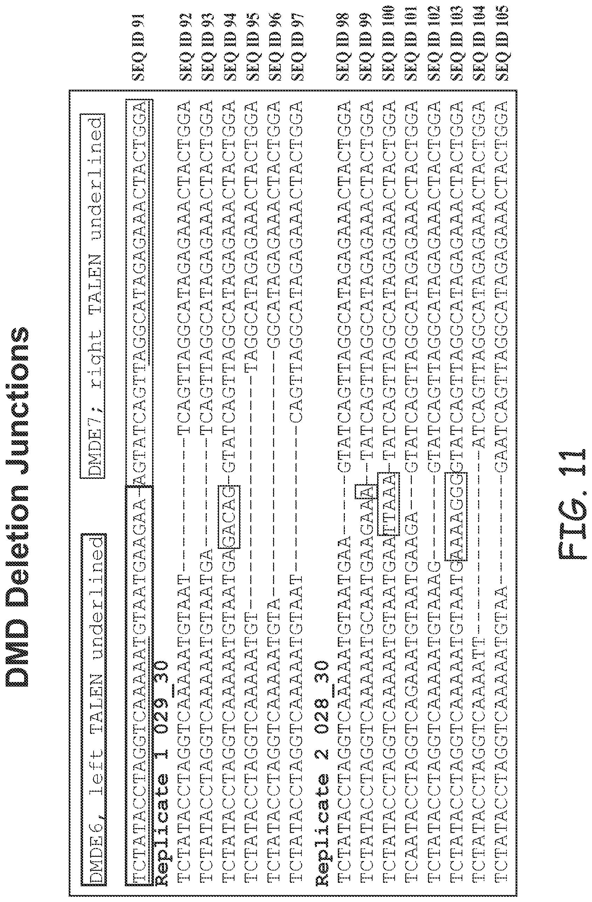

Fig. 11. DMD deletion sequences. DMD deletion junctions from replicate transfections are displayed. Above, exons 6 and 7 sequences are shaded, and TALEN-recognition sites are underlined. Inserted nucleotides are shaded.

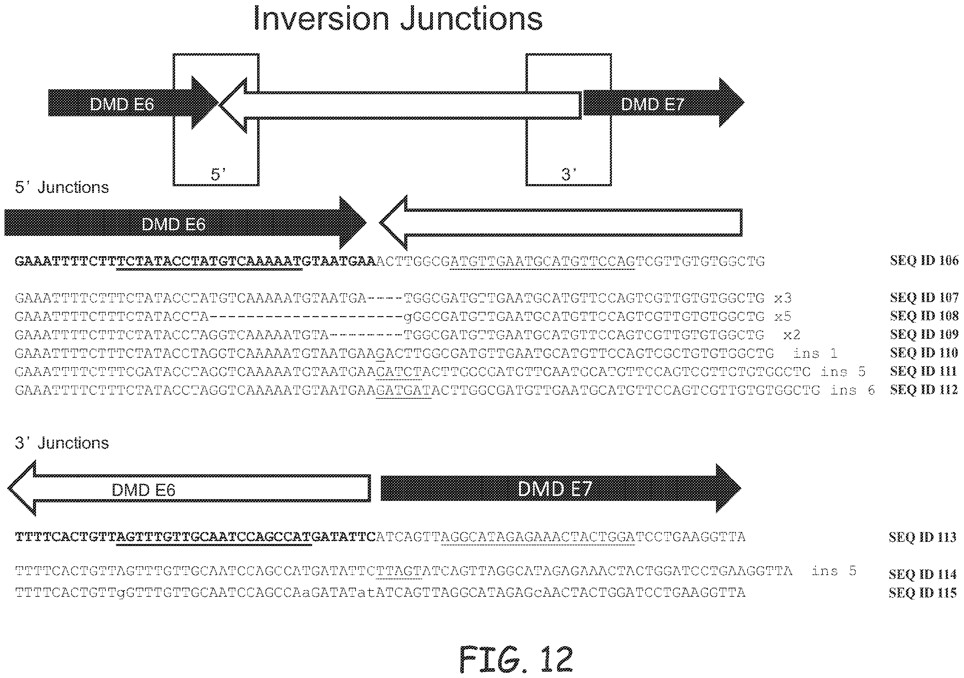

Fig. 12. DMD inversion sequences. A schematic of the DMD inversion allele is shown with the 5' and 3' junctions (boxed) that were analyzed by sequencing. Below, the predicted sequence for each fusion is shown corresponding fusion at the center of each spacer for the TALEN pairs. TALEN-recognition sites are underlined. Sequenced inversion alleles from a transfected population are shown. The number of times each allele was sequenced is indicated at the right and inserted nucleotides are underlined. Mismatched nucleotides are denoted as lower-case text.

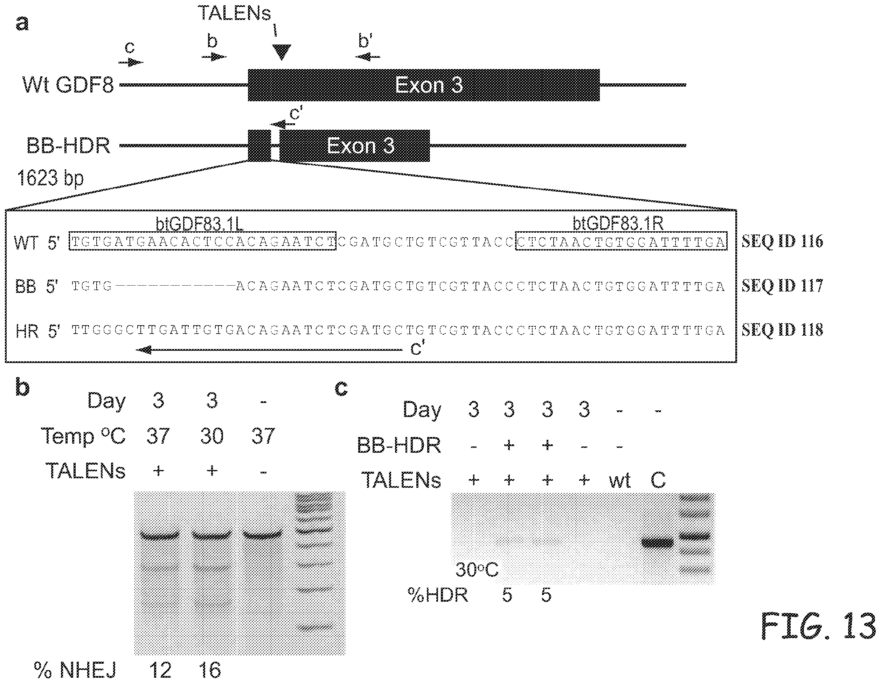

Fig. 13. HDR induction in bovine fibroblasts. Panel a) TALENs (btGDF83.1, arrow) and a dsDNA template (BB-HDR) were designed to introduce an 11-basepair deletion into exon-3 of bovine GDF8 (Belgium Blue mutation) by Double-Strand Break-induced homologous recombination. Half of the binding site for the left TALEN is missing in the BB-HDR template and thus should be resistant to TALEN cleavage. Panel b) SURVEYOR assay demonstrates activity of btGDF83.1 TALENs at both 37 and 30° Celsius. The PCR product used for this assay was generated using primers b and b' (shown in panel a). The BB-HDR template was not included in these replicates since it would confound estimates of btGDF83.1 activity. Panel c) Allele-specific PCR demonstrates that HDR induction is dependent on co-transfection of TALENs and the BB-HDR template. The PCR assay was developed to specifically detect HDR modified GDF8 alleles using primers c and c' (shown panel a). The 3' end of primer c' spans the 11-basepair deletion, and cannot amplify the wild type allele (wt). Five hundred cell equivalents were included in each PCR reaction including the positive control "C". Percent HDR was determined by comparative densitometry between experimental and control reactions.

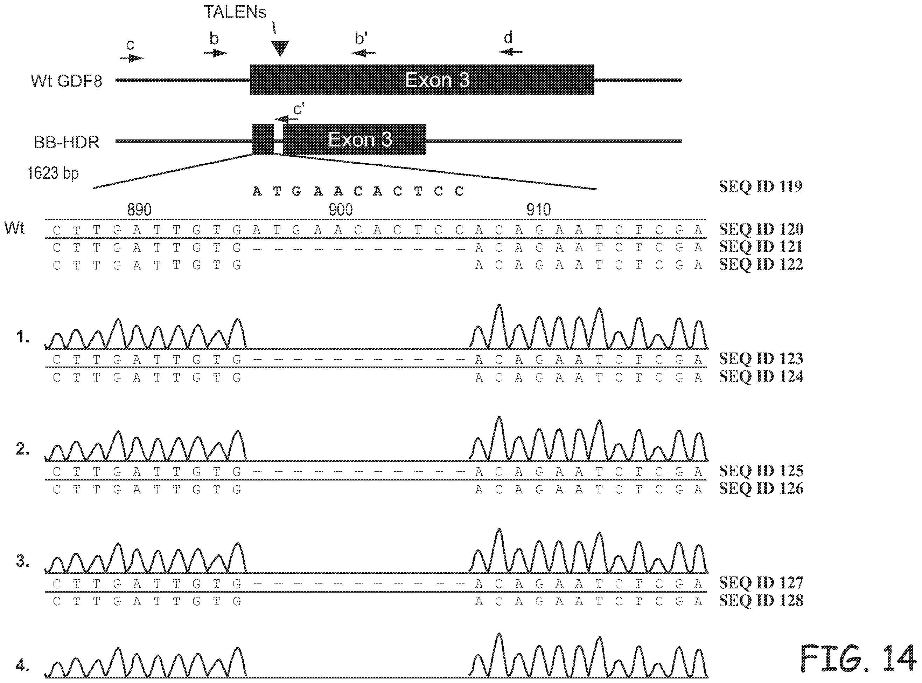

Fig. 14. Confirmation of Belgian Blue introgression by sequencing. The schematics of Wagyu wild-type GDF8 and the Belgian Blue template (BB-HDR) are shown. PCR was conducted using primers located outside of the homology arms (c and d) on five PCR positive colonies followed by cloning and sequencing with primer b'. Comparison to the wild-type sequence reveals the expected 11-basepair deletion characteristic the Belgian Blue allele (heterozygous) in 4 of 5 colonies.

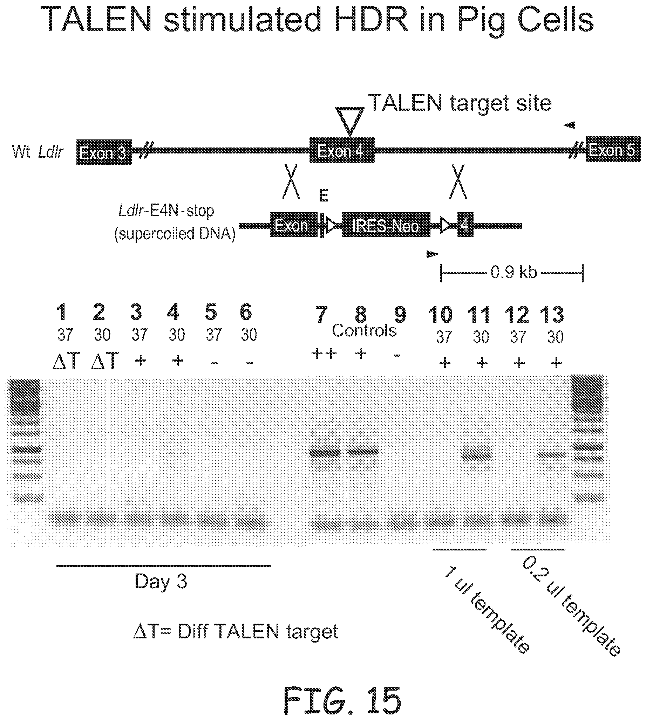

Fig. 15. Schematic and gel for TALEN-mediated HDR. A TALEN pair (LDLR2.1) targeted to the fourth exon of the swine low density lipoprotein receptor (LDLR) gene was co-transfected with the supercoiled plasmid *Ldlr*-E4N-stop, which contains homology arms corresponding to the swine LDLR gene and a gene-trap enabling expression of Neomycin phosphotransferase upon HDR.

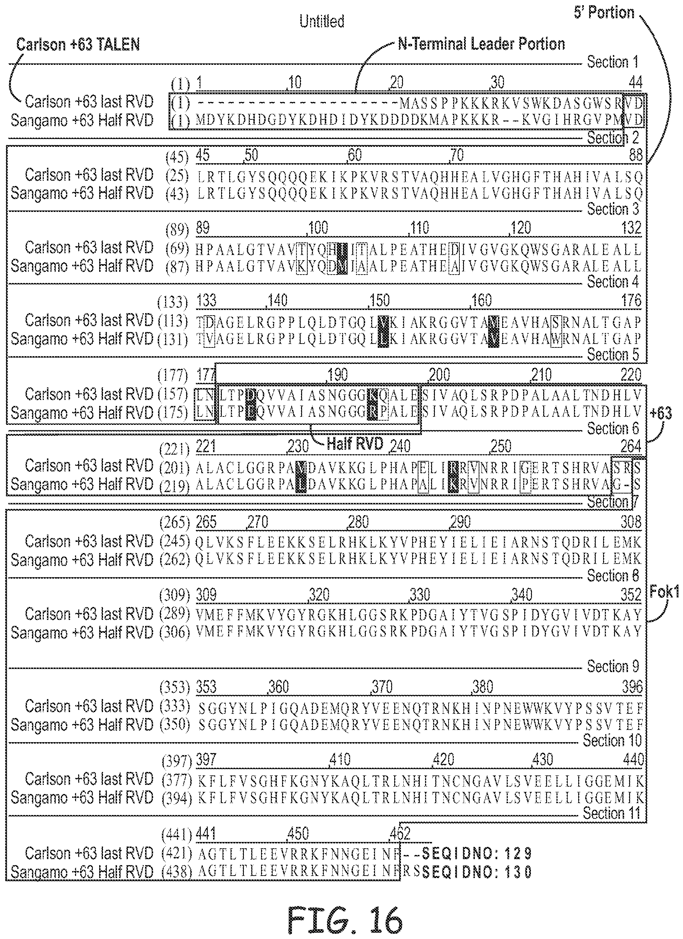

Fig. 16. Detailed sequence information for the Carlson +63 scaffold of Figure 5 and comparison to an alternative scaffold used by Sangamo Biosciences.

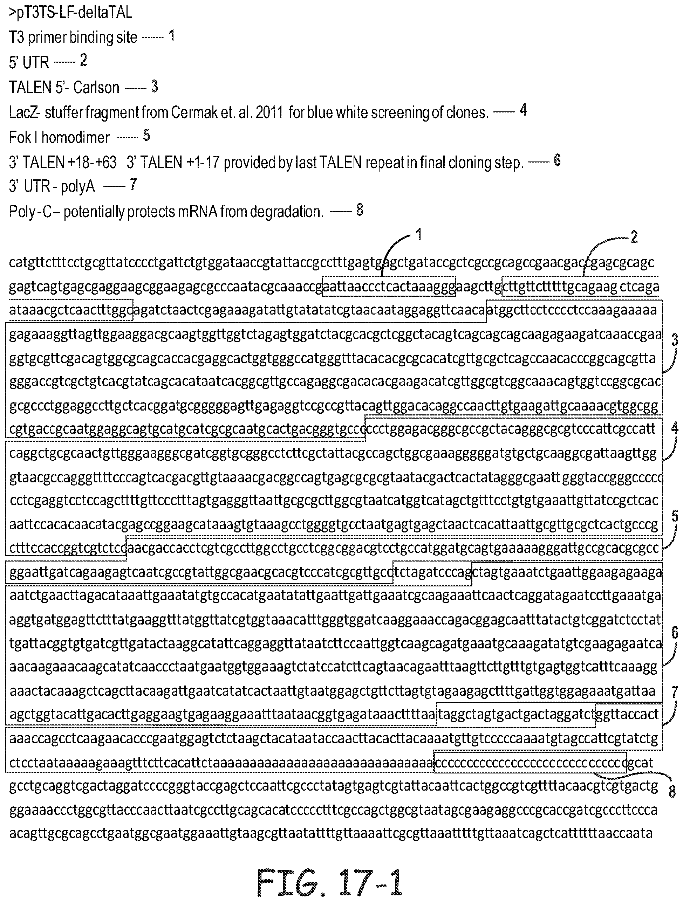

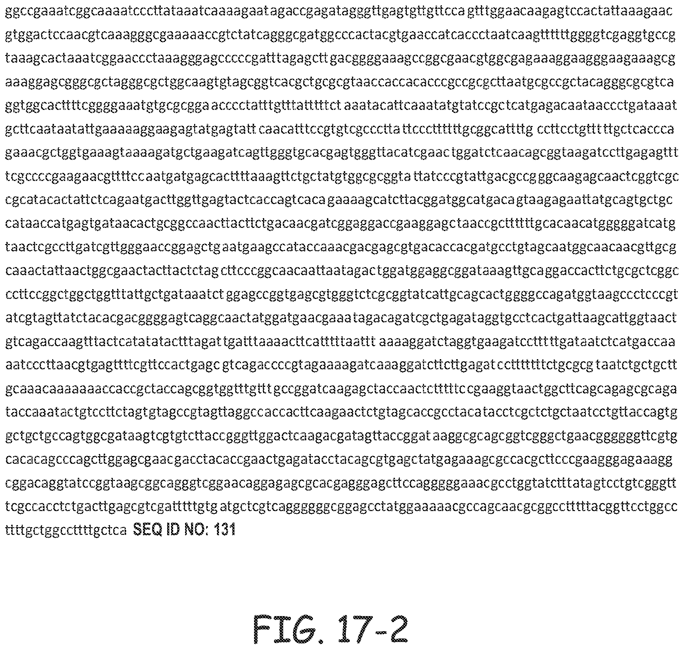

Fig. 17. Detailed nucleic acid sequence for the vector used to make the Carlson +63 scaffold of Figure 5, including non-translated portions.

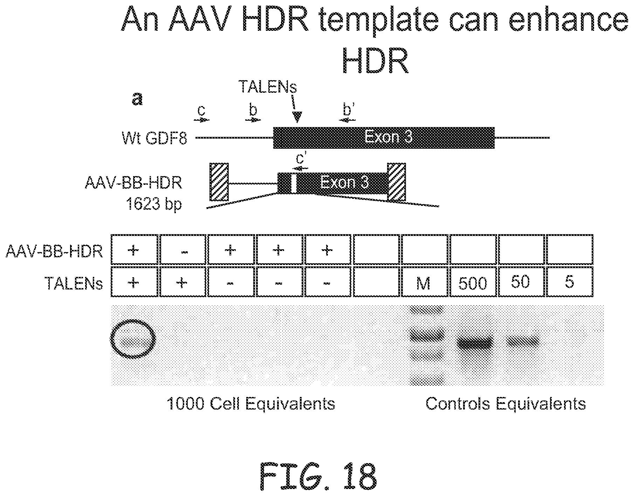

Fig. 18. Use of an AAV-delivered single stranded DNA template emplate for homologous recombination at the bovine GDF8 locus. a) TALENs (btGDF83.1, blue arrow) and a rAAV homologous recombination template (AAV-BB-HDR) were designed to introduce an 11 bp deletion into exon-3 of the bovine GDF8 gene (Belgium Blue mutation) by homologous recombination. b) Allele-specific PCR demonstrates that HR induction is dependent on transfection btGDF83.1 TALENs and infection with defective AAV containing the AAV-BB-HDR template. The PCR assay was developed to specifically detect HDR modified GDF8 alleles using primers c and c' (shown panel a). The 3' end of primer c' spans the 11 bp deletion, and cannot amplify the wild type allele "WT". 1,000 cell equivalents were included in each PCR reaction and positive control reactions with the indicated copy number of a control template were used for comparative quantification of homologous recombination.

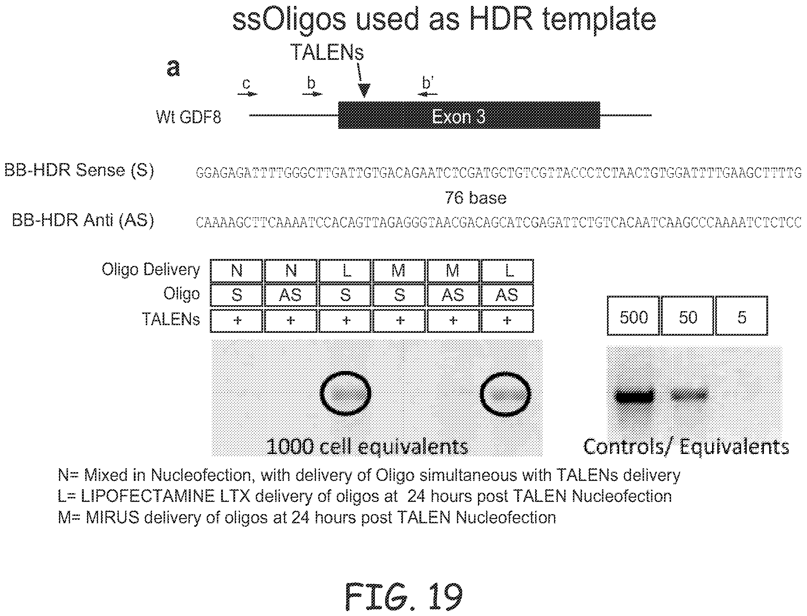

Fig. 19. Use of single stranded oligonucleotides (ssOligos) as a template for homologous recombination at the bovine GDF8 locus. TALENs (btGDF83.1, arrow) and two ssODNs were designed to introduce an 11 bp deletion into exon-3 of the bovine GDF8 gene (Belgium Blue mutation) by homologous recombination. Each ssODN was 76 base pairs in length and were sense and antisense strands of the same target site Allele-specific PCR demonstrates that HDR induction is dependent on transfection btGDF83.1 TALENs and subsequent transfection of ssODNs using Lipofectamine LTX 24 hours later. The PCR assay was developed to specifically detect HDR modified GDF8 alleles using primers *c* and *c'* (shown panel a). The 3' end of primer *c*' spans the 11 bp deletion, and cannot amplify the wild type allele "WT". 1,000 cell equivalents were included in each PCR reaction and positive control reactions with the indicated copy number of a control template were used for comparative quantification of homologous recombination. BB-HDR Sense (S) has SEQ ID NO:133 and BB-HDR Anti (AS) has SEQ ID NO:134.

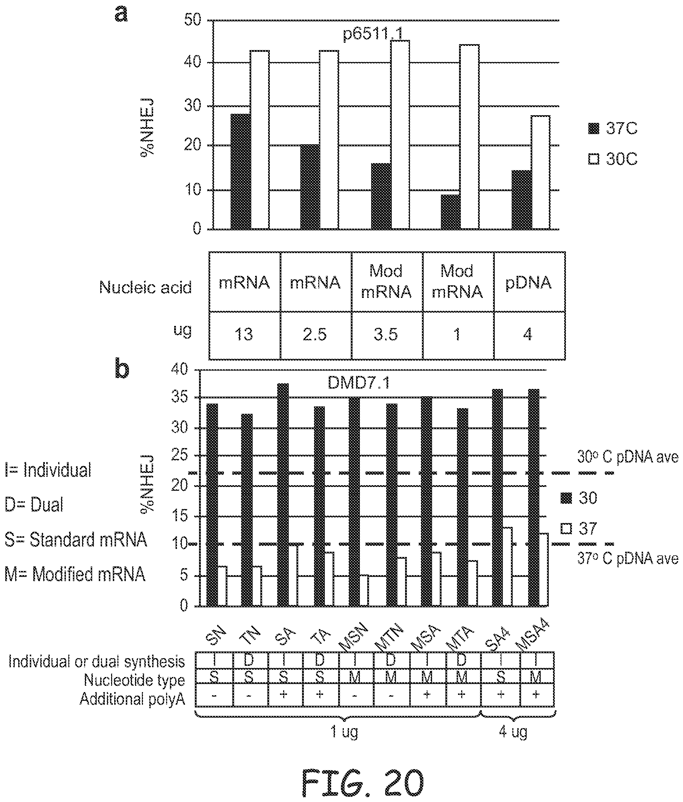

Fig. 20. Transfection of TALEN encoding mRNAs into livestock cells results in efficient target cleavage. Panel a: The indicated quantity of mRNA was transfected into pig fibroblasts and transfected cells were cultured at either 30 or 37 degrees Celsius for three days prior to indel analysis. Panel b: Percent NHEJ was determined. The average percent NHEJ for by transfection of 4 micrograms of plasmid DNA encoding the DMD7.1 TALENs is displayed by dashed lines for cells cultured at 30 or 37 degrees Celsius.

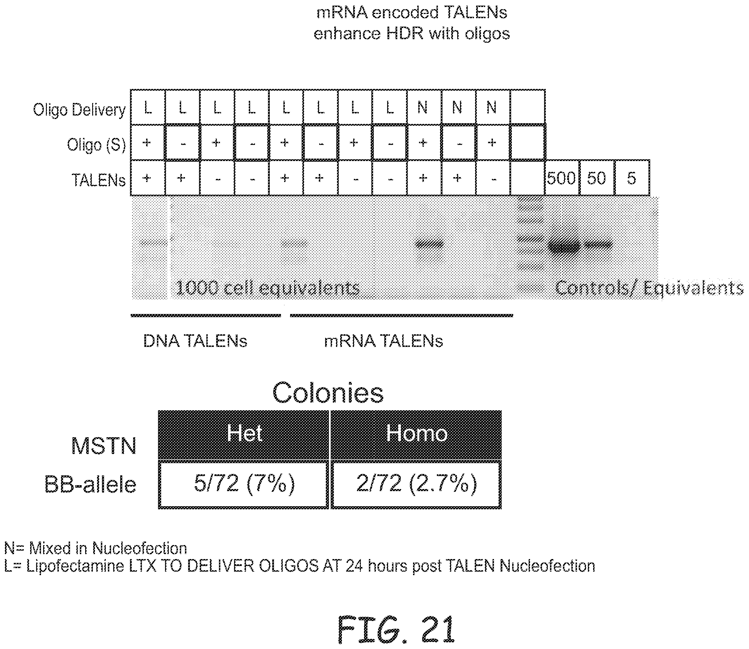

Fig. 21. Transfection of mRNA encoded TALENs enhances ssODN HDR. btGDF83.1 TALEN mRNA and BB-HDR sense ssODN (SEQ ID NO:133) were introduced into Wagyu cells by the specified mechanism and HDR was measured by PCR assay described above. Colonies were isolated from the population of cells where both TALEN mRNA and the ssODNs were delivered simultaneously by nucleofection.

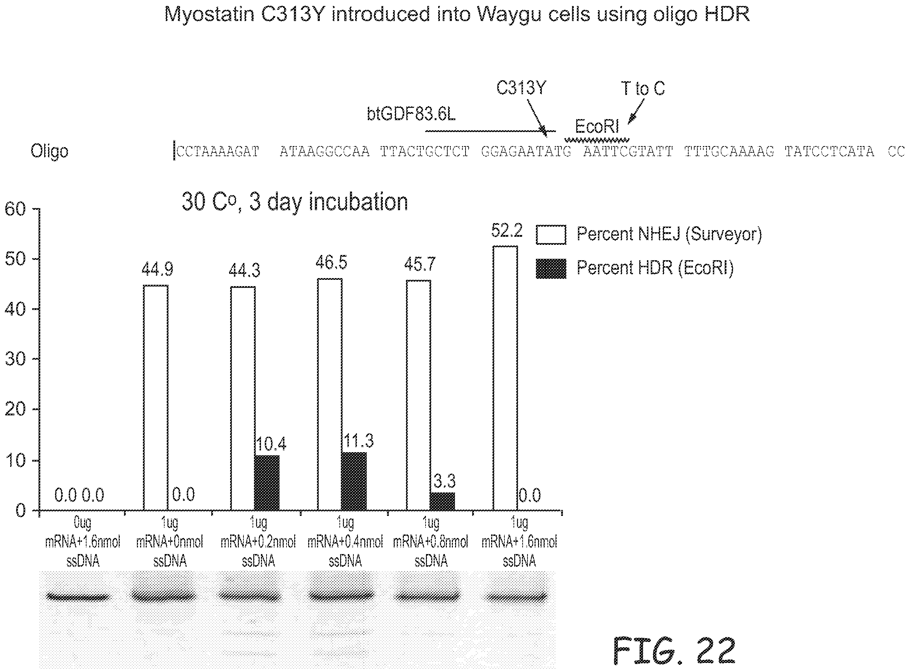

Fig. 22. Introgression of naturally occurring alleles within a species using mRNA encoded TALENs and ssODNs. The Piedmontese Myostatin allele C313Y was introgressed into Waygu fibroblasts by the methods of Figure 21. The sequence labeled "oligo" is has SEQ ID NO:135.

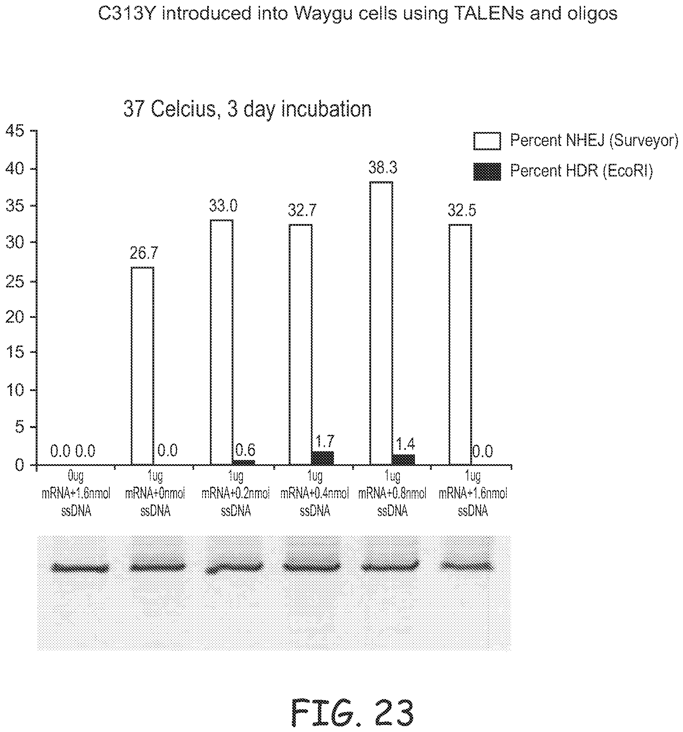

Fig. 23. The process of Fig. 22 was repeated at a different temperature (37°C).

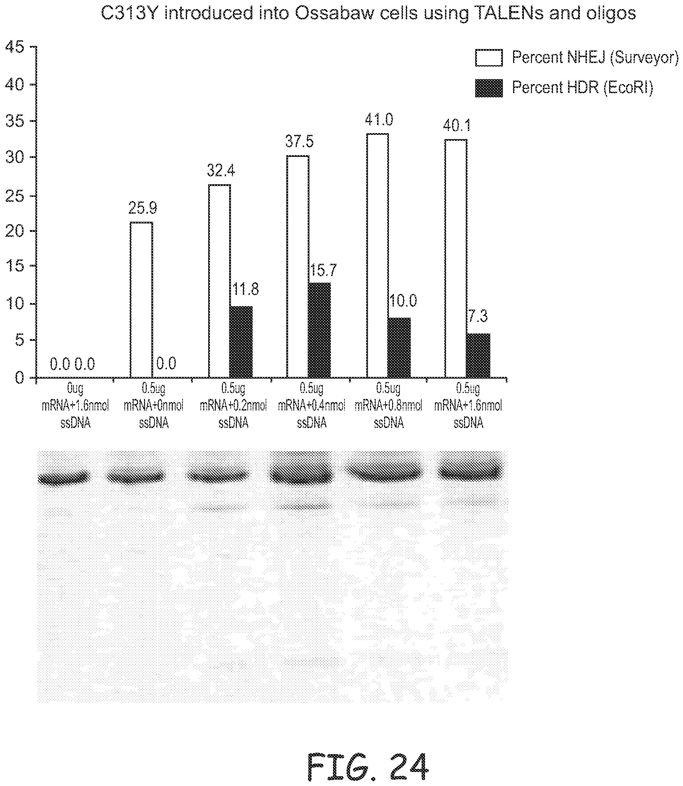

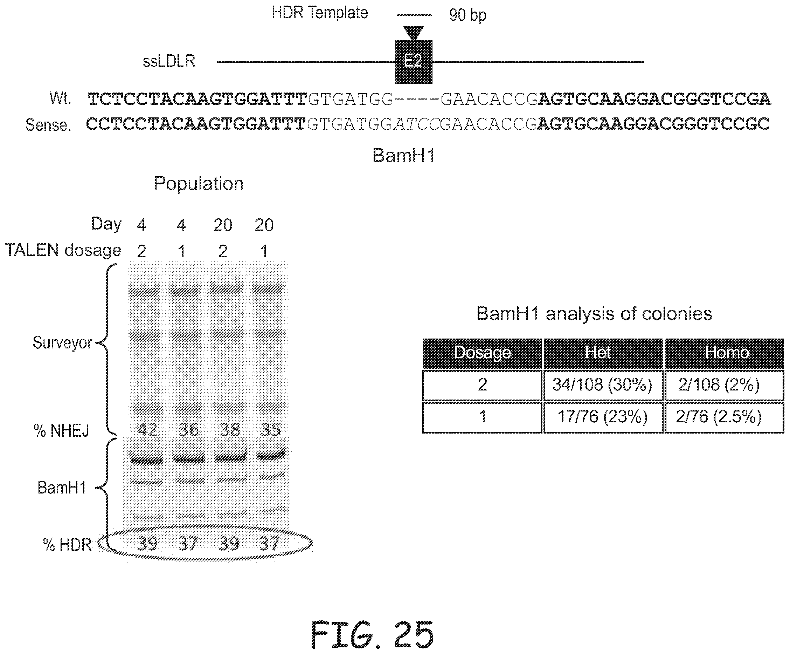

Fig. 24. Introgression of naturally occurring alleles from one species to another using mRNA encoded TALENs and ssODNs. The Piedmontese Myostatin allele C313Y was introgressed into Ossabaw fibroblasts by the methods of Figure 25. The following ssODN was used ggccaattactgctctggagagtatgaattcgtatttttacaaaaataccctcacactcatcttg (SEQ ID NO:146)
Fig. 25. Introduction of a particular frameshift allele into porcine LDLR using mRNA encoded TALENs and ssODNs. A 90-bp oligo was created to introduce a 4 base pair insertion into exon 2 of the porcine LDLR gene. The insertion creates a novel BamH1 site and is predicted to cause a frameshift allele. After co-transfection of ssLDLR2.1 TALEN mRNA (at indicated dosage in micrograms) and 0.3 nMol of ssODN cells were cultured at 30°C for 3 days, followed by an additional day at 37°C. NHEJ was measured by SURVEYOR assay at days 4 and 20. Percent HDR was determined by BamH1 digest of PCR products that include exon 2 of porcine LDLR and quantification of restriction fragments (indicative of HDR) and comparison to wild type products (top product, no HDR) by densitometry. Colonies were isolated from each treatment and analyzed by PCR and BamH1 digest. The sequence labeled "Wt" has SEQ ID NO:136 and the sequence labeled "Sense" has SEQ ID NO:137.

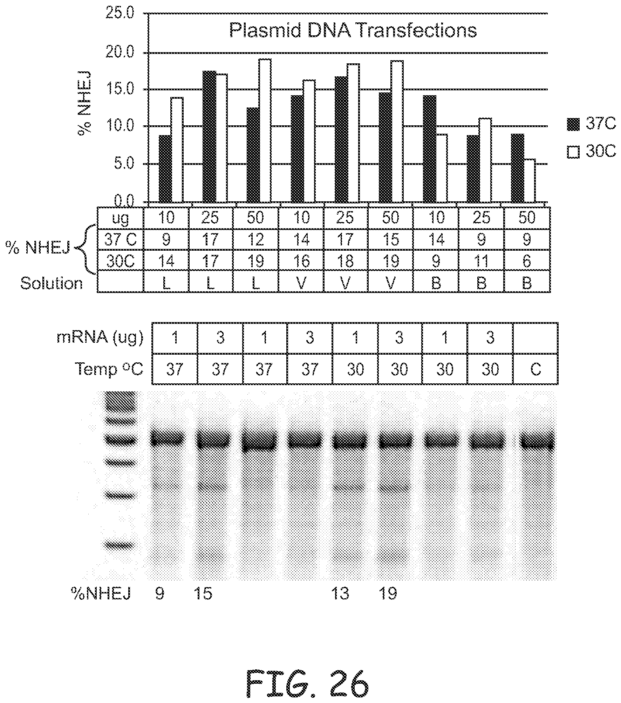

Fig. 26. DNA and mRNA encoded TALENs are active in stem cells. The top panel shows percent NHEJ of DMD7.1 TALENs transfected as plasmid DNA into porcine male germ-line stem cells (GSCs). Nucleofection solutions L, V or B were evaluated. The lower panel shows SURVEYOR assay results of porcine GSCs transfected with mRNA encoding DMD7.1 TALENs. The quantity of mRNA (micrograms) is indicated.

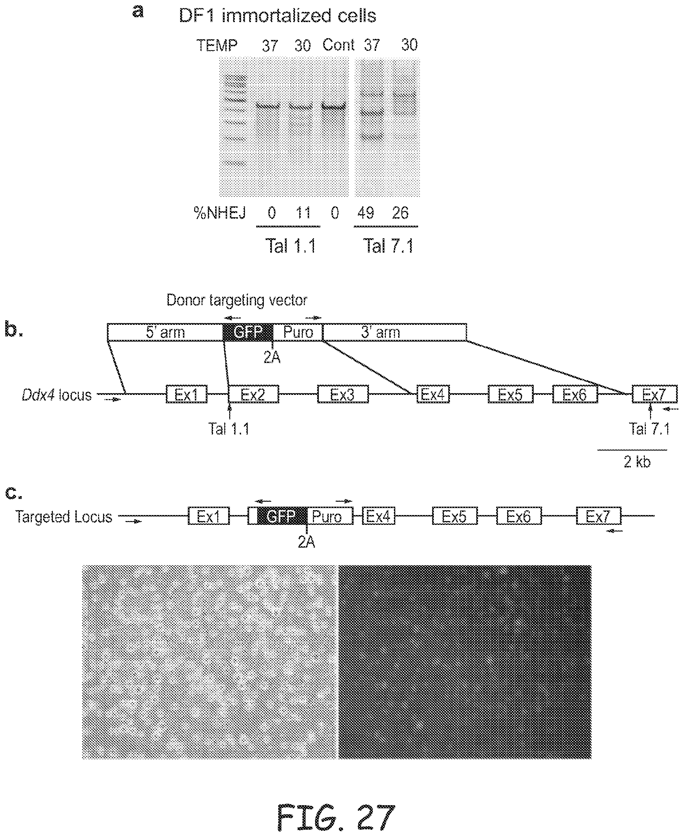

Fig. 27. TALENs mediate DSB in chicken cells and can stimulate homologous recombination in chicken primordial germ cells (PGCs). Panel a) TALEN activity was first determined in DF1 immortalized chicken cells line transfection and SURVEYOR assay. Panel b) Schematic depiction of the targeting strategy of the chicken *ddx4* locus. The GFP/Puromycin reporter gene will replace the endogenous coding sequence in the second exon of targeted cells. Penal c) PGCs were transfected with the homologous recombination construct, TALENs (either Tal 1.1 or Tal 7.1, empty vector) and a puromycin selection transposon. After selection in puromycin, GFP+ cells could be isolated when Tal 1.1 TALENs were used (right picture, left is bright field) but not with Tal7.1 TALENs or empty vector transfections.

### DETAILED DESCRIPTION

The invention is defined in the appended claims and any other aspects or embodiments set forth herein are for information only. The term "animal" as used herein does not include humans. Traditional breeding programmes based on animal mating or artificial reproductive techniques involve mixing many genes in the hope of ultimately producing a good combination of genes that create or combine desirable traits. Transgenic techniques hold out a promise of accelerating traditional breeding processes. Some drawbacks of transgenic processes are that the processes, while an improvement, are nonetheless slow, costly and labor-intensive. Low efficiencies and unpredictability in results are normal. Further, processes that make a change only at an intended genomic site are not available.

Disclosed herein are processes to make transgenic animals that have changes only at an intended site. Additionally, the processes can make specifically intended changes at the intended site. It is not necessary to remove other changes resulting from problems like the use of linked-reporter genes, or linked positive and negative selection genes, or random transgene integration are bypassed. Moreover, the processes can be used in the founder generation to make genetically modified animals that have only the intended change at the intended site. Other processes are also disclosed that involve unlinked marker genes and the like. The techniques use TALENs.

The successful use of TALENs to make genetic modifications in higher animals such as swine or cows presents considerable challenges. Compositions and methods of making such animals are set forth herein. Some of these methods involve cloning from primary artiodactyl or other livestock cells, which also presents considerable challenges. Further, methods for identifying cells or embryos that have been modified with TALENs are presented, as well as processes for enriching the percentage of TALEN-treated cells or embryos. Unexpectedly, it was observed that a genetic modification of one chromosome by a TALEN often caused the complementary locus of the other chromosome to also be modified by cellular machinery.

Further, it was also discovered that TALENs could be used to make gross chromosomal deletions (GCDs) at a plurality of sites. Fig. 2 illustrates this approach, which involves a first TALEN pair directed to a first locus and a second TALEN pair directed to a second locus. It was also surprisingly discovered that inversions of large chromosomal sequences could be created using pairs of TALENs. One use of the inversions is the creation of artiodactyls or other founder animals with fixed genetic traits, or the creation of deletion strains.

### Creation of genetically modified livestock via TALEN technologies; verification of TALEN modification; co-transfectional co-selection for selection of modified cells; elimination of reporter genes from genetically modified animals

One of the barriers to making TALEN-modified livestock or other animals is that the efficiency of making a modification to an animal cell is only a few percent with conventional best practices. Achievement of a deletion or an insertion at an intended site does not necessarily mean success because it may not actually create the intended effect, such as expressing an exogenous protein or stopping expression of an endogenous protein. Even a low efficiency can be useful for the creation of genetically modified lower animals such as fruit flies or mice because they have short and prolific reproductive cycles that provide for the creating, testing, and screening of hundreds of animals to determine if there are a few that have been successfully modified. These levels of efficiency that are conventionally achieved, however, are not suited to livestock artiodactyls that have much longer gestational times and comparatively few progeny per pregnancy.

Another barrier to using TALENs to modify livestock or other animals is that TALEN-mediated modification of DNA in primary cells is difficult because the cells are unstable. Indeed, it is shown herein that frequency of TALEN-modified cells decreases significantly over time in the absence of enrichment or selection methods. Without being bound to a particular theory, it is theorized that DNA cleavage at non-intended sites can compromise the stability of the cell by inducing apoptosis or disabling non-target genes. The term primary cell means a cell isolated from a living animal, wherein the cell has undergone between 0 and 2 replications since its isolation from the tissue.

As a result, techniques customarily used to create and test transformed cells for successful genetic modification can not be used in primary cells due to their propensity to senesce. TALEN-modified cells are customarily destroyed to assay their genetic modification, or isolated to grow clonal lines with many identical cells from one parent. However, primary cells are inherently unstable and typically undergo genetic changes, senescence, and/or cell death when attempts are made to genetically modify and clonally expand them. TALEN-modified cells are even less stable, as documented herein for the first time. As a result, it is unreasonable to expect high rates of success when using conventional approaches that involve modifying a primary cell for somatic cell nuclear transfer or other animal cloning technique. As reported herein, however, TALENs have been used to make genetically modified artiodactyl primary cells. These modifications are suited to making founders of genetically modified animal lines by cloning. Also described herein are direct-embryonic injections that were used to modify zygotes, with the modified zygotes being suited to implant into surrogate females for gestation and delivery of founder animal lines.

A typical approach to testing for an actual TALEN-mediated insertion/deletion event is to sequence the modified cell or zygote, which is a destructive process. Thus when a zygote or embryo is modified before implantation to a surrogate, its modification can not be verified with any degree of convenience until the animal is born. It is not conventionally appreciated that an actual production process for making genetically modified animals by cloning will benefit from a process for testing for the presence of a genetic modification. There are inventions presented herein that provide for an indication of genetic modification at the single cell, zygote, or oocyte stage. As shown herein, expression of a reporter gene that is not coupled to TALEN modification is, despite not being part of the reporter gene expression cassette, nonetheless generally predictive of a desired genetic modification. More specifically, the expression of the reporter gene indicates that the nucleic acids were effectively delivered and being expressed in a cell or embryo; a reporter-expressing cell or embryo is more likely to have undergone TALEN-based modification.

Another technique for making modified organisms was the use of a co-transfection, co-selection technique. The cells that express the reporter are selected for, and may be used for making genetically modified animals. The reporter may be chosen to require transposase activity. Without being bound to a specific theory, it is theorized that cells that have undergone transposition have 1) been transfected and 2) been competent for double stranded DNA repair, thus increasing the likelihood of TALEN-based modification in selected clones. This also facilitates enrichment/selection for transposed cells (and by extension TALEN-modified cells). The fact that the transposon is operably but not physically linked to the TALEN modification permits their segregation away from each other by breeding. A benefit of a co-transfection strategy is that the reporter, or reporters, may be placed on a chromosome that is not the same chromosome that is modified by TALENs. This process provides for the creation of founder animals that have no reporter genes. For example, some animals were made by using plasmids carrying reporter genes that were independent of the genetic modification, which was orchestrated separately in the cells. This scheme was based on a theory of operation that cells that incorporate new reporter genes will also incorporate genetic modifications. For instance, data provided herein shows that cells can be transfected with four independent plasmids and the successful incorporation of the gene product of one plasmid is predictive of successful incorporation of the other plasmid gene products and also for the success of TALEN-mediated changes. Conventional wisdom is that transfection with so many plasmids would not be successful and would yield unhealthy cells. Unexpectedly, however, these techniques were effective.

TALEN function in livestock embryos was investigated using *in vitro* prepared (IVP) bovine and porcine embryos. Example 1 describes direct injection of TALENs (a left TALEN and a right TALEN) into bovine embryos to produce genetically modified animals with a modification at the site where the TALENs specifically bound. The modifications included homozygous and heterozygous (bi-allelic) modifications. TALEN mRNAs were directly injected into the embryos and successful genetic modifications were observed. Direct embryo modification using TALENs was thus shown to be a viable approach to livestock genome modification. Embryos may thus be prepared and implanted into surrogate females for gestation and delivery of animal founder lines using well known processes. Moreover, it is possible to use a reporter to select cells (e.g., primary cells, zygotes, oocytes, blastocysts) for further use in cloning or other processes.

Methods for TALEN-mediated genetic modification of livestock (or zebrafish, dogs, mice, rats, avian, chicken, or a laboratory animal) by cloning were also developed. Example 3 describes development of suitable TALENs and TALEN modification of somatic primary cells of swine and cows. The efficiency of successful modification was somewhat low and no reporters for measuring success of the modification were used. Nucleofection is a means for introducing foreign nucleic acids into a cell with high efficiency, but it is expensive, results in high levels of cytotoxicity, and is not available to many researchers. Therefore, a common cationic lipid transfection reagent was used as a vehicle for genetic modification. As shown in Example 4, despite a less than 5% transfection efficiency with cationic lipids, modification levels were significantly enriched by transposon co-selection. Whereas gene modification was below detection in day 3 populations (data not shown) and day 14 populations without transposon-mediated selection, modification levels in selected populations reached 31, 13 and 20 percent for DMD7.1, DMD6 and LDLR2.1 respectively (Fig. 7). Transposon co-selection was then applied to cells transfected by nucleofection where >90% transfection efficiency is routine. Transposon co-selection was effective for maintenance modified cells transfected by Nucleofection, however, with the exception of ACAN12, nucleofection did not significantly enrich for modified cells over day 3 levels (Fig. 7). Thus, transposon co-selection is an effective enrichment method when transfection efficiency is low and an effective maintenance method when transfection efficiency is high. Co-selection processes were also effective when feeder cells were used, as demonstrated in Example 5. An unexpectedly high proportion of bi-allelic modifications (about 17% to about 35% depending on the TALEN-pair) were observed.

An embodiment of the invention is a method for using TALENs to genetically modify livestock such as artiodactyls or zebrafish, dogs, mice, rats, fish, avian, chicken, or a laboratory animal. Many of the problems making these animals using conventional processes have been discussed above. The genetic modification may be, for example, chosen from the list consisting of an insertion, a deletion, insertion of or change to an exogenous nucleic acid fragment, an inversion, a translocation, interspecies allele migration, intraspecies allele migration, gene conversion to a natural, synthetic, or a novel allele. For instance, an undesired mutation in a chromosome or chromosome pair may be replaced with a normal sequence. In general, a target DNA site is identified and a TALEN-pair is created that will specifically bind to the site. The TALEN is delivered to the cell or embryo, e.g., as a protein, mRNA or by a vector that encodes the TALEN. The TALEN cleaves the DNA to make a double-strand break that is then repaired, often resulting in the creation of an indel, or incorporating sequences or polymorphisms contained in an accompanying exogenous nucleic acid that is either inserted or serves as a template for repair of the break with a modified sequence. The term exogenous nucleic acid means a nucleic acid that is added to the cell or embryo, regardless of whether the nucleic acid is the same or distinct from nucleic acid sequences naturally in the cell. An exogenous sequence refers to a sequence used to change the target cell, regardless of whether the sequence is actually a nucleic acid inserted into chromosomal DNA or if the sequence is used as a template to change the cellular DNA. The term nucleic acid fragment is broad and includes a chromosome, expression cassette, gene, DNA, RNA, mRNA, or portion thereof. The term ssDNA includes ss-oligonucleotides. The cell or embryo may be, for instance, chosen from the group consisting of livestock, an artiodactyl, a cow, a swine, a sheep, a goat, a bird, a chicken, a rabbit, and a fish. The term livestock means domesticated animals that are raised as commodities for food or biological material. The term artiodactyl means a hoofed mammal of the order *Artiodactyla,* which includes cattle, deer, camels, hippopotamuses, sheep, and goats, that have an even number of toes, usually two or sometimes four, on each foot. One embodiment is directed to a method of making a genetically modified livestock and/or artiodactyl or a zebrafish, dogs, mice, bird, fish, avian, chicken, rats or a laboratory animal comprising introducing a TALEN-pair into a cell or an embryo that makes a genetic modification to DNA of the cell or embryo at a site that is specifically bound by the TALEN-pair, and producing the livestock animal/artiodactyl/other animal from the cell. Direct injection may be used for the cell or embryo, e.g., into a zygote, blastocyst, or embryo. Alternatively, the TALEN and/or other factors may be introduced into a cell using any of many known techniques for introduction of proteins, RNA, mRNA, DNA, or vectors. Genetically modified animals may be made from the embryos or cells according to known processes, e.g., implantation of the embryo into a gestational host, or various cloning methods. The phrase "a genetic modification to DNA of the cell at a site that is specifically bound by the TALEN", or "at a targeted chromosomal site", or the like, means that the genetic modification is made at the site cut by the nuclease on the TALEN when the TALEN is specifically bound to its target site. The nuclease does not cut exactly where the TALEN-pair binds, but rather at a defined site between the two binding sites.

Another such embodiment involves a treatment of a cell that is used for cloning the animal. The cell may be of a livestock, artiodactyl cell, fish, zebrafish, dog, mice, rat, laboratory animal, bird, fish, chicken, a cultured cell, an immortalized cell, a primary cell, a primary somatic cell, a zygote, a germ cell, a primordial germ cell, a blastocyst, or a stem cell. For example, an embodiment is a method of creating a genetic modification comprising exposing a plurality of primary cells in a culture to TALEN proteins or a nucleic acid encoding a TALEN or TALENs. The TALENs may be introduced as proteins or as nucleic acid fragments, e.g., encoded by mRNA or a DNA sequence in a vector.

Genetic modification of animals may also include transfection with a reporter (not part of the invention). As discussed above, primary cells were observed to be unstable as a result of cellular modifications mediated by the TALENs and/or TALENs introduction. As a result, success in the modification of primary cells (among other cell types), and/or the creation of new lines of livestock from such cells is not reasonably expected using conventional means. It is theorized, without being bound to a specific theory, that cells that express a gene cassette from a first vector are also likely to be successfully modified by a TALEN delivered independently by mRNA or another vector. Expression of a reporter allows for elimination of cells that do not express the reporter. Alternatively, it allows for moving cells that express the reporter from the culture for use in animals by cloning or other transgenic animal techniques, or into a second culture for further cultivation and/or expansion in number and/or addition of further vectors and/or nucleic acids and/or TALENs and/or other genetic modifications. Selecting cells based on their expression of a reporter that is independent of the gene of interest is a type of co-selection process.

The term reporter, as used herein, includes reporters and selection markers. The term selection marker, as used herein, refers to a genetically expressed biomolecule that confers a trait that permits isolation by either positive or negative survival selection criteria. The reporter may be, e.g., a fluorescent marker, e.g., green fluorescent protein and yellow fluorescent protein. The reporter may be a selection marker, e.g., puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), or xanthin-guanine phosphoribosyltransferase (XGPRT). Other phenotypic markers may be used to select animals, such markers are based on discernible physical traits (e.g., epitopes or color), growth rate, and/or viability.

Embodiments of the disclosure include introducing a reporter (for instance by use of a vector) and a TALEN (e.g., by an independent vector or mRNA) into a cell or embryo. The cell may be from a livestock or artiodactyl, bovine, avian, chicken, zebrafish, dog, mice, rats or a laboratory animal. The TALEN and/or reporter may be directly introduced, e.g., by injection, or other means, e.g., involving cell culture. A cell culture may be made comprising cultured cells (primary cells, zygotes, oocytes, immortalized cells, germ cells, primordial germ cells, stem cells), a first nucleic acid encoding a TALEN, e.g., mRNA or a vector with DNA encoding the TALEN, and an independent vector having a DNA sequence encoding a reporter. The mRNA or first vector do not encode any reporters and the second vector does not encode any TALs and does not encode any TALENs.

Vectors for the reporter, selection marker, and/or one or more TALEN may be a plasmid, transposon, transposase, viral, or other vectors, e.g., as detailed herein. Transposases may be used. One embodiment involving a transposases provides a vector that encodes a transposase. Other vectors encode a transposon that is recognized by the transposase and has a nucleic acid fragment of interest, e.g., a reporter, selection marker, exogenous nucleic acid for insertion or as a template for modification, or one or more TALENs. Accordingly, a cell or embryo may be transfected with a number of vectors between, for example, 1 and about 6; artisans will immediately appreciate that all the ranges and values within the explicitly stated ranges are contemplated, e.g., 2, 3, 4, 5, and 6. More vectors may be used. The reporter may be used to identify cells that are likely to have undergone modification by TALENs. Or a selection marker may be used to enrich the proportion of TALEN-modified cells by destroying cells or embryos that do not express the selection marker.

A disclosure is a cell or embryo culture exposed to, or injected with, a plurality of vectors. A first vector comprises a TALEN or TALEN-pair; alternatively there are two TALEN vectors that independently provide a left TALEN and a right TALEN. A second vector comprises a reporter. The reporter may provide for non-destructive identification or may be a selection marker. A vector encoding a selection marker may be used as an alternative to the reporter vector, or in addition to the reporter vector. A further vector may encode an exogenous nucleic acid.

A disclosure process for making TALEN-modified cells, embryos, or animals comprises assaying a cell or embryo exposed to a TALEN for expression of a reporter and using that cell or embryo in a method or composition for making a genetically modified livestock and/or artiodactyl or other animal (fish, zebrafish, dogs, mice, avian, chicken, rats or a laboratory animal). For instance, a primary cell may be removed from a cell culture and used for cloning. Or, a primary cell may be removed from culture and placed in a second culture to make a clonal line or for further processes. Or, an embryo or zygote expressing the reporter may be used for either implantation into a surrogate dam or can be used for cloning, while other embryos or zygotes that do not express the reporter not used for cloning. In some embodiments, the reporter is a selection marker that is used to select for cells or embryos that express the marker.

### Gross Chromosomal Deletions and Inversions; Genetically Modified Animals

Experiments were performed with TALENs directed to a plurality of DNA sites. The sites were separated by several thousand base pairs. It was observed that the DNA could be rejoined with the deletion of the entire region between the sites. Embodiments include, for example, sites separated by a distance between 1-5 megabases or between 50% and 80% of a chromosome, or between about 100 and about 1,000,000 basepairs; artisans will immediately appreciate that all the ranges and values within the explicitly stated ranges are contemplated, e.g., from about 1,000 to about 10,000 basepairs or from about 500 to about 500,000 basepairs. Alternatively, exogenous DNA may be added to the cell or embryo for insertion of the exogenous DNA, or template-driven repair of the DNA between the sites. Modification at a plurality of sites may be used to make genetically modified cells, embryos, artiodactyls, and livestock. Example 6 describes the deletion of several thousand basepairs of DNA, with rejoining of the ends verified biochemically.

Unexpectedly, TALEN-cleavage at separated sites also resulted in frequent inversion of the entire region between TALEN targets. As an additional surprise, as detailed in Example 6, these inversions were accomplished with great fidelity. Forty one out of 43 of the tested inversions were positive at both the 5' and 3' junctions. And sequencing of PCR products confirmed both deletion and inversion events with addition or deletion of very few nucleotides at their junctions (Fig. 11, 12). This result was highly surprising and unprecedented. Cells or embryos with these deletions or inversions have many uses as assay tools for genetics.

These cells are also useful for making animals, livestock, and animal models. The term animal model includes, for example, zebrafish, dogs, mice, rats or other laboratory animals. Large deletions provide for gene inactivation. Also, for instance, a deletion strain may be made of cells, livestock, or animal models. Crossing the deletion strains with an organism bearing a mutation for comparison to a wild-type helps to rapidly and conveniently localize and identify the mutation locus. Deletion strains are well known in these arts and involve sets of organisms made with a series of deletions in a gene. Deletion mapping involves crossing a strain which has a point mutation in a gene with the deletion strains. Wherever recombination occurs between the two strains to produce a wild-type (+) gene, the mutation cannot lie within the region of the deletion. If recombination cannot produce any wild-type genes, then it is reasonable to conclude that the point mutation and deletion are found within the same stretch of DNA. This can be used for example to identify causative mutations, or to identify polymorphisms underlying quantitative trait loci.

Cells, embryos, livestock, artiodactyls, and animal models with inversions are also useful for fixing a genetic trait in progeny of an organism or an animal line or animal breed. Recombinations typically occur between homologous regions of matching chromosomes during meiosis. Inversion of a chromosomal region destroys homology and suppresses meiotic recombination. Methods and compositions described herein may be used to make such organisms or animals. For example, DNA in a somatic bovine or porcine cell may be cut at a plurality of loci by TALENs, and cells with an inversion may be isolated, or cells expressing reporters may be used as likely candidates for successful inversions. The cells may be used to clone animals that harbor chromosomal regions that are incapable of meiotic recombinations. Alternatively, it is expected that inversions will also occur at reasonable frequencies in embryos treated with multiple TALEN-pairs at plurality of sites.

An embodiment of this method is identifying a DNA region encoding a genetic trait and cutting a DNA in a cell or embryo on each side of the encoded trait at sites using a plurality of TALENs. The modified cell or embryo may be used for creating a genetically modified animal. The method may comprise isolating a genetically modified animal that has the inversion.

### Movement of alleles

Some livestock traits are related to alleles such as polymorphisms (large or small), single nucleotide polymorphisms, deletions, insertions, or other variations. For instance, a myostatin allele (an 11-bp deletion) from Belgian Blue cattle is well known to cause a double-muscling phenotype. Example 7 shows, using the Belgian Blue allele, how to precisely transfer specific alleles from one livestock breed to another by homology-dependent repair (HDR). Bovine fibroblasts received the allele and may readily be used to make transgenic cattle. This allele does not interfere with normal development and the methods taught herein place the allele with precision and without disruption of other genes or the incorporation of exogenous genes. As already discussed, results presented herein show that the frequency of allele conversion in livestock fibroblasts is high when sister chromatids are used for an HDR template, therefore allele introgression into one sister chromatid can be anticipated frequently to result in homozygosity.

An embodiment of this invention is a method of transfer of an allele from a first livestock line or breed to a second livestock line or breed, comprising cutting DNA with a pair of TALENs in a cell or embryo of the second livestock line/breed in a presence of a nucleic acid that contains the allele of the first livestock line/breed. The embryo or cell may be used to create an animal of the second line/breed that has the allele of the first line/breed. The DNA that contains the allele provides a template for homology-dependent repair. As a template, it has homology to portions of the DNA on each side of the cut and also contains the desired allele.

Embodiments of the invention comprise moving a plurality of alleles from one breed to another breed. For instance, alleles may be moved from *Wagyu* or *Nelore* cattle to *Belgian Blue* cattle, or vice versa. As set forth elsewhere herein, the TALENs may be delivered a protein or encoded by a nucleic acid, e.g., an mRNA or a vector. A reporter may also be transfected into the cell or embryo and used as a basis for selecting TALEN-modified cells. The reporter may be assayed non-destructively and/or may comprise a selection marker. The term breed means a group of domestic animals or plants with a homogeneous appearance, behavior, and other characteristics that distinguish it from other animals or plants of the same species. The animals that belong to a particular breed are known to artisans that practice in these arts. Similarly, allele migration may be practiced in an animal model.

A population or species of organisms typically includes multiple alleles at each locus among various individuals. Allelic variation at a locus is measurable as the number of alleles (polymorphisms) present, or the proportion of heterozygotes in the population. For example, at the gene locus for the ABO blood type carbohydrate antigens in humans, classical genetics recognizes three alleles, IA, IB, and IO, that determine compatibility of blood transfusions. An allele is a term that means one of two or more forms of a gene.

In livestock, many alleles are known to be linked to various traits such as production traits, type traits, workability traits, and other functional traits. Artisans are accustomed to monitoring and quantifying these traits, e.g., Visscher et al., Livestock Production Science, 40 (1994) 123-137, US 7,709,206, US 2001/0016315, US 2011/0023140, and US 2005/0153317. Accordingly, the allele that is transferred may be linked to a trait or chosen from a trait in the group consisting of a production trait, a type trait, a workability trait, a fertility trait, a mothering trait, and a disease resistance trait.

The term natural allele in the context of genetic modification means an allele found in nature in the same species of organism that is being modified. The term novel allele means a non-natural allele. A human allele placed into a goat is a novel allele. The term synthetic allele means an allele that is not found in nature. Thus a natural allele is a variation already existing within a species that can be interbred. And a novel allele is one that does not exist within a species that can be interbred. Movement of an allele interspecies means from one species of animal to another and movement intraspecies means movement between animals of the same species.

Moving an allele from one breed to another by conventional breeding processes involves swapping many alleles between the breeds. Recombination during meiosis inevitably exchanges genetic loci between the breeds. In contrast, TALENs-modified livestock and other animals are free of genetic changes that result from meiotic recombination events since the cells or embryos are modified at a time when cells are exclusively mitotic. As a result, a TALEN-modified animal can easily be distinguished from an animal created by sexual reproduction.

The processes herein provide for editing the genomes of existing animals. The animal has a fixed phenotype and cloning the animal, e.g., by somatic cell cloning, effectively preserves that phenotype. Making a specific change or changes in a cellular genome during cloning allows for a known phenotype to be altered. Processes herein alternatively provide for altering a genome of an embryo that has yet to develop into an animal with fixed traits. Embryos with sound genetics may nonetheless not express all of the traits that are within the genetic potential of their genetics, i.e., animals do not always express the traits that their line is bred for. Embodiments include providing embryos having genetics known to be capable of expressing a set of traits and exposing the embryos to the TALEN (optionally without a reporter gene and/or selection marker) and screening the gestated animal for the modification and for expression of the set of traits. Accordingly, the introgression of desirable alleles into livestock can be achieved by editing the genomes of animals previously determined to be of significant genetic value by somatic cell modification and cloning, or by editing the genomes of animals prior to determining their implicit genetic value by treatment/injection of embryos. In the case of cloning, genetically superior animals could be identified and subjected to gene editing for the correction of a loss of function allele or the introgression of desirable alleles that are not already present. This approach provides for a controlled and characterized outcome at every step of the process as only cells harboring the desired changes would be cloned.

Editing could also be applied by the direct treatment of embryos. Embryos of unknown genetic merit would be treated and screening of offspring may consist of analysis for the desired change and analysis of genetic merit of the animal, e.g., analysis for the change plus analysis of various traits that the animal expresses. A beneficial aspect of this approach is it can be applied simultaneous with genetic improvement by marker assisted selection whereas cloning results in the loss of 1+ generation intervals. The efficiency of such modifications would need to be sufficiently high to offset any losses in reproductive rate engendered by embryo treatment. In the case of simple gene-inactivation, the frequency of success is very high (75%), with even homozygous modification in 10-20% of embryos (Examples 1 and 9). Embodiments include exposing embryos (or cells) to a TALEN (optionally without a reporter gene and/or without a marker gene with more than about 1% of the embryos incorporating the modification at the TALEN-targeted chromosomal site (heterozygous or homozygous); artisans will immediately appreciate that all the ranges and values within the explicitly stated ranges are contemplated, e.g., from about 1% to about 85%, or at least about 5% or at least about 10%. Cells may similarly have TALENs introduced successfully at vey high efficiencies, with the same ranges being contemplated, i.e., more than about 1%. Conventional processes achieve a lower percentage. Moreover, precision genome editing can also be used to introduce alleles that do not currently exist within a species by homology-driven allelic substitution.

### Animals genetically modified without any reporters; TALENs techniques; Allelic Migrations

Certain embodiments of the invention are directed to processes of modifying cells or embryos without the use of reporters and/or selection markers. In general, it was observed that the frequency of TALEN-modified cells decreases significantly over time in the absence of enrichment or selection methods such as the use of reporter genes. This observation lead to approaches such as the co-transfection, co-selection technique reported herein that involves reporter genes.

It has been discovered, however, that TALENs modification can be performed with an efficiency that is so great that reporters are not needed and their use merely delays the creation of transgenic animal lines. Without being bound to a particular theory, a number of factors independently contributed to the invention of the reporter-free embodiments. One is the realization that TALENs tend to act quickly and at a high efficiency. However, TALENs modifications tended to be unstable over a time frame of several days such that efficiencies can seem to be low depending on the time of sampling. Further, it is conventional wisdom that only stably modified organisms should be used to make transgenic animals so that there is little incentive to understand short-term modifications. There is an incentive to use cell survival genes to select for stable incorporation, as is conventionally done in other systems. Another factor is that TALENs mRNA is unexpectedly effective as compared to vectors that express the TALENs. Direct introduction of mRNA encoding TALENs is, in general, useful, and was used in Examples 13 to 18.

Another factor is that, when an HDR template is desired, direct introduction of ssDNA, e.g., single stranded (ss) oligonucleotides, is useful, as demonstrated in Example 12. A confounding effect is that the timing of the delivery of ssDNA was important. In Example 12, delivery of the ss oligonucleotides at the same time as the TALENs encoded from plasmid DNA was not effective, but delaying introduction of the ss oligonucleotides for 24 hours resulted in high efficiencies. On the other hand, Example 16 showed that simultaneous introduction of ss oligonucleotide templates and TALENs mRNA was effective. Since TALENs were introduced in Example 12 as plasmid DNA expression cassettes, there may have been 12 or more hours of delay between transfection and accumulation of enough TALEN protein to begin cleaving the target. Perhaps the oligonucleotides introduced with the TALENs in Example 12 were degraded by the cells or otherwise un-available (compartmentalized or in complex with proteins) to act as template for HR at the same time that TALENs were actively cleaving the target. Another confounding factor, surprisingly, was that the ss nucleotides have a biphasic effect (Example 16). That is to say, too little or too much ss oligonucleotide results in a low frequency of HDR. Embodiments of the invention include those wherein the ssDNA is introduced into the cell after a vector encoding a TALEN is introduced into the cell, for instance, between about 8 hours and about 7 days afterwards; artisans will immediately appreciate that all the ranges and values within the explicitly stated ranges are contemplated, e.g., from about 1 to about 3 days hours. Embodiments of the invention include those wherein the ssDNA is introduced into the cell at about the same time as mRNA encoding a TALENs is directly introduced, with the term "about the same time" meaning within less than about 7 hours of each other.

Another factor contributing to discovery of reporter-free embodiments was that there is an unexpected synergy between ssDNA (ss oligonucleotide) templates and TALENs activity. The basis for this synergy is not known. For example, delivery of 0.5-10 micrograms TALEN encoding mRNAs to 500,000-750,000 cells by nucleofection followed by 3 days of culture at 30 degrees Celsius results in consistent levels of modification. But supplementation of these same conditions with 0.2-1.6 nMol of ssODN led to an increase in TALENs activity, as observed by increased NHEJ as assayed by SURVEYOR assay (Example 16). Typically, a transfection consists of 1-4 micrograms of TALEN mRNA and 0.2-0.4 nMol of ssDNA. Embodiments include introducing to a cell or an embryo, an amount of TALEN mRNA that is more than about 0.05 µg per 500,000 cells, or in a range of from about 0.05 µg to about 100 µg per 500,000 cells; artisans will immediately appreciate that all the ranges and values within the explicitly stated ranges are contemplated. Embodiments include further introducing ssDNA at a concentration of more than about 0.02 nMol or in a range of from about 0.01 to about 10 nMol of ssDNA.

The term direct introduction, e.g., direct mRNA introduction, refers to introduction of mRNA material. In contrast, introduction by means of a vector encoding the mRNA is termed indirect introduction. Many processes of direct introduction are known, e.g., electroporation, transfection, lipofection, liposome, nucleofection, biolistic particles, nanoparticles, lipid transfection, electrofusion, and direct injection.

Founder animals can be immediately created from modified cells or embryos without the need to create initially modified animals that are subsequently bred to create the basis for a new transgenic line. The term founder or founder animal is used to refer to a first-generation ("F0") transgenic animal that develops directly from the cloned cell or treated/injected embryo that is modified. Methods reported herein provide for creation of founders genetically modified only at the chromosomal target site, and without intermediate steps of breeding and/or inbreeding. Moreover, embodiments include founders that are homozygous for the modification. The founders may be prepared without ever exposing cells and/or embryos to reporter genes (and/or selection marker genes).

Embodiments include a method of making a genetically modified animal, said method comprising exposing embryos or cells to an mRNA encoding a TALEN, with the TALEN specifically binding to a chromosomal target site in the embryos or cells, cloning the cells in a surrogate mother or implanting the embryos in a surrogate mother, with the surrogate mother gestating an animal that is genetically modified without a reporter gene and only at the chromosomal target site bound by the TALEN. The animal may be free of all reporter genes or may be free of selection markers, e.g., is free of selection markers but has a reporter such as a fluorescent protein. Options include directly introducing the TALENs as mRNA and/or a ss oligonucleotide that provides a template for a genetic modification, e.g., an allele.

A method of making a genetically modified animal comprises introducing TALENs and/or vectors into cultured cells, e.g., primary livestock cells. The TALENs are directed to specific chromosomal sites and cause a genetic alteration at the site. An HDR template may also be introduced into the cell, e.g., as a double stranded vector, single stranded DNA, or directly as a ss nucleotide. The cultured cells are subsequently cultured to form colonies of clonal cells. The colonies are tested by PCR and/or sequenced, or otherwise assayed for a genetic modification, preferably without a reporter gene and/or without a selection marker. Cells are taken from colonies that are genetically modified at the intended site and used in cloning. For example, from 10 to 50,000 cells are used to make from 10 to 50,000 embryos that are implanted into surrogates, e.g., in sets of 1-500 embryos per surrogate; artisans will immediately appreciate that all the ranges and values within the explicitly stated ranges are contemplated. Embodiments comprise exposing the cells to the TALEN without a reporter gene, creating colonies of clonal cells, and testing a subset of members of the colonies to identify colonies incorporating the modification at the chromosomal target site.

Processes of making colonies of clonal cells from cultured cells are known. One such method involves dispersing cells from a first culture into a second culture wherein the various cells are not in contact with each other, e.g., by diluting the cells into multiwall plates or into a plate with a relatively large surface area for the number of cells placed therein. The cells are cultured for a period of time that allows the cells to multiply. The multiplying cells are cultured in conditions where they are not likely to move far away from their original location. As a result, a user may observe the cells after the period of time and see various colonies that are all made of a single cell and its progeny. A subset of the cells in the colony may be sampled without destroying the other cells in the colony.

Assays for a genetic modification may include destructive assays, meaning an assay that destroys the cell that is tested to determine if it has a certain property. Destructive assays provide an opportunity to rapidly, thoroughly, and directly test for a medication. Destructive assays are made practical by a taking a sample of a clonal colony. Many such assays are highly efficient, particularly when the intended modification is known. For example, PCR may be performed to identify indels or mismatches in pre-existing sequences, or to detect a sequence of a HDR template. Or, for example, cellular DNA may be nucleolytically assayed, e.g., to determine if a novel nuclease target sequence has been successfully introduced or knocked-out. Example 18 uses a nucleolytic destructive assay. Other processes may be used that involve, e.g., sequencing or SDS-PAGE to find a band that is indicative of a modification. Other processes may be used that involve, e.g., sequencing or SDS-PAGE to find a band that is indicative of a modification. Testing processes may be, e.g., chosen from the group consisting of a nucleolytic assay, sequencing, PAGE, PCR, primer extension, or hybridization.

Allele migration has many important applications. The Allelic Migration Table, below, describes certain genes and their applications. Artisans reading this application will be able to make and use the migrations and resultant cells and animals. Artisans can readily apply the processes set forth herein for the use of these alleles as templates or targets for disruption. Embodiments include making a genetically modified cell or animal (for instance, a lab animal, an F0 founder, or animal line) that has a genome with a has received a gene from the Table, e.g., by insertion or template-driven allele migration. Alleles for some genes are reported to provide livestock production advantages, but are at very low frequencies or are absent in some breeds or species (see items 1-9). Introgression of these alleles can be of significant value for production traits. For example, the Polled allele (item 1) from beef breeds results in animals that do not have horns, whereas dairy breeds do not have this allele so have horns and need to be dehorned as a production practice. Allele migration from beef breeds into horned (dairy) breeds will lead to hornless dairy cattle which is has value for both production and animal welfare. Other examples relate to alleles that can increase or enhance characteristics of agricultural products such as meat (items 4-6) and milk (items 7-8). Item 9 is useful for disease resistance.

Many commercial and commonly used animal breeds have been carefully bred to establish desirable traits but, in the process of that breeding, have accumulated genetic errors that reduce their reproductive success because of losses in fertility or by increasing miscarriages. Deleterious alleles for some genes are present in animal populations. As explained elsewhere herein, the inventive techniques provide for changing alleles only at an intended location in a target animal, without other modifications resulting from genetic tools or from meiotic recombinations. Therefore, for the first time, it is possible to clean-up the genetic errors that have accumulated in livestock and animal breeds without disrupting the genome of the animals and, consequently, disrupting traits or causing unintended consequences. Alleles for some genes can be used to control animal fertility for genetic control of breeding stock (items 2-3).

Many useful animal models can be made. Certain alleles are useful, see items 10-39. Some of these are established in animals. Others of the genes are known to cause human disease, so introgressing these alleles into livestock, lab animals, or other animals is useful to create biomedical models of human disease.

Embodiments of the invention include a method of making a genetically modified animal, said method comprising exposing embryos or cells to an mRNA encoding a TALEN, with the TALEN specifically binding to a target chromosomal site in the embryos or cells, cloning the cells in a surrogate mother or implanting the embryos in a surrogate mother, with the surrogate mother thereby gestating an animal that is genetically modified without a reporter gene and only at the TALEN targeted chromosomal site wherein the allele is a member of the group consisting of (a) horn polled locus (b) a gene recessive for fertility defects, e.g., CWC15 and/or ApaFl (c) genes for enhancing a livestock trait, e.g., meat production (GDF8, IGF2, SOCS2, or a combination thereof) and/or milk production (DGAT1 and/or ABCG2) (d) a gene for resistance to African swine fever (P65/RELA) (e) a gene for reduction of animal size (GHRHR) (f) genes that potential tumor growth (e.g., TP53, APC, PTEN, RB1, Smad4, BUB1B, BRCA1, BRCA2, ST14 or a combination thereof) (g) human oncogenes for animal models of cancer (e.g., AKT1, EGF, EGFR, KRAS, PDGFRA/B or a combination thereof) (h) genes in animal models for hypercholesterolemia (to induce atherosclerosis, stroke, and Alzheimer's disease models), e.g., LDLR, ApoE, ApoB or a combination thereof (i) Inflammatory Bowel disease, e.g., NOD2 (j) spina bifida, e.g., VANGL1 and/or VANGL2 (k) pulmonary hypertension, e.g., miR-145 (l) genes for cardiac defects, e.g., BMP10, SOS1, PTPN11, Nrg1, Kir6.2, GATA4, Hand2, or a combination thereof and (1) celiac disease genes, e.g., HLA-DQA1.

**Allelic migration Table**

| item | Genes; Species | Application |
|---|---|---|
| | [Gene Reference Identification] | |
| 1 | Horn-Polled Locus; Bovine | Transfer allele into cows of various breeds to make bovine lines of those species without horns; see Medugorac, I., D. Seichter, et al. (2012). "Bovine polledness - an autosomal dominant trait with allelic heterogeneity." PloS one 7(6): e39477. |
| | [UMD3.1:1:1705490:1706389:1] | |
| 2 | CWC15 (JH1) [hs Gene ID: 51503] | Use natural allele as template to restore wildtype sequence to animal lines and breeds with defective alleles; see VanRaden, P. M., K. M. Olson, et al. (2011). "Harmful recessive effects on fertility detected by absence of homozygous haplotypes." J Dairy Sci 94(12): 6153-6161. |
| 3 | ApaF1 (HH1) | |
| | [hs Gene ID: 317] | |
| 4 | GDF8 | Enhancement of growth for meat production. |
| | [hs Gene ID:2660] | |
| 5 | IGF2 | |
| | [hs Gene ID: 3481] | |
| 6 | SOCS2 | |
| | [hs Gene ID: 8835] | |
| 7 | DGAT1 | Alleles of these genes are known to influence the amount and composition of milk. |
| | [hs Gene ID: 8694] | |
| 8 | ABCG2 | |
| | Hs Gene ID: 9429] | |
| 9 | P65/RELA | Transfer of the warthog p65 allele to commercial swine breeds for resistance to African swine fever. Palgrave, C. J., L. Gilmour, et al. (2011). "Species-specific variation in RELA underlies differences in NF-kappaB activity: a potential role in African swine fever pathogenesis." Journal of virology 85(12): 6008-6014. |
| | [hs Gene ID: 5970] | |
| 10 | GHRHR | Size reduction of animals for Biomedical modeling. |
| | [hs Gene ID: 2692] | |
| 11 | TP53 | Tumor suppressor genes; heterozygous knockout to potentiate tumor growth. |
| | [hs Gene ID: 7157] | |
| 12 | APC | |
| | [hs Gene ID: 324] | |
| 13 | PTEN | |
| | [hs Gene ID: 5728] | |
| 14 | RB1 | |
| | [hs Gene ID: 5925] | |
| 15 | Smad4 | |
| | [hs Gene Id: 4089] | |
| 16 | BUB1B | |
| | [hs Gene ID: 701] | |
| 17 | BRCA1 | |
| | [hs Gene ID: 672] | |
| 18 | BRCA2 | |
| | [hs Gene ID: 675] | |
| 19 | ST14 | |
| | [hs Gene ID: 6768] | |
| 20 | AKT1 | Oncogenes. Activated human alleles will be introgressed into pigs to model cancers. |
| | [hs Gene ID: 207] | |
| 21 | EGF | |
| | [hs Gene ID: 1950] | |
| 22 | EGFR | |
| | [hs Gene ID: 1956] | |
| 23 | KRAS | |
| | [hs Gene ID: 3845] | |
| 24 | PDGFRA/B | |
| | [hs Gene IDs: 5156/5159] | |
| 25 | LDLR | Hypercholesterolemia to induce atherosclerosis, stroke and Alzheimer's disease models. |
| | [hs Gene ID: 3949] | |
| 26 | ApoE | |
| | [hs Gene ID: 348] | |
| 27 | ApoB | |
| | [hs Gene ID: 338] | |
| 28 | NOD2 | Inflammatory Bowel disease for animal models. |
| | [hs Gene ID: 64127] | |
| 29 | VANGL1 | Spina Bifida is associated with alleles of these genes. Transfer of these alleles in livestock will generate models for biomedical research. |
| | [hs Gene ID: 81839] | |
| 30 | VANGL2 | |
| | [hs Gene ID: 57216] | |
| 31 | miR-145 | Pulmonary hypertension is associated with alleles of these genes. Transfer of these alleles in swine will generate models for biomedical research. |
| | [hs Gene ID: 611795] | |
| 32 | BMP10 | Cardiac defects associated with alleles of these genes. Transfer of these alleles will generate models for biomedical research. |
| | [hs Gene ID: 27302] | |
| 33 | SOS1 | |
| | [hs Gene ID: 6654] | |
| 34 | PTPN11 | |
| | [hs Gene ID: 5781] | |
| 35 | Nrg1 | |
| | [hs Gene ID: 3084] | |
| 36 | Kir6.2 | |
| | [hs Gene ID: 3767] | |
| 37 | GATA4 | |
| | [hs Gene ID: 2626] | |
| 38 | Hand2 | |
| | [hs Gene ID: 9464] | |
| 39 | HLA-DQA1 | Alleles associated with celiac disease will be transferred to livestock to create an animal model. |
| | [hs Gene ID: 3117] | |

### Compositions and kits

The present invention also provides compositions and kits containing, for example, nucleic acid molecules encoding TALENs, TALEN polypeptides, compositions containing such nucleic acid molecules or polypeptides, or TALEN engineered cell lines. Such items can be used, for example, as research tools, or therapeutically.

### Recombinases

Embodiments of the invention include administration of a TALEN or TALENs with a recombinase or other DNA-binding protein associated with DNA recombination. A recombinase forms a filament with a nucleic acid fragment and, in effect, searches cellular DNA to find a DNA sequence substantially homologous to the sequence. An embodiment of a TALEN-recombinase embodiment comprises combining a recombinase with a nucleic acid sequence that serves as a template for HDR. The HDR template sequence has substantial homology to a site that is targeted for cutting by the TALEN/TALEN pair. As described herein, the HDR template provides for a change to the native DNA, by placement of an allele, creation of an indel, insertion of exogenous DNA, or with other changes. The TALEN is placed in the cell or embryo by methods described herein as a protein, mRNA, or by use of a vector. The recombinase is combined with the HDR template to form a filament and placed into the cell. The recombinase and/or HDR template that combines with the recombinase may be placed in the cell or embryo as a protein, an mRNA, or with a vector that encodes the recombinase, see US Pub 2011/0059160 (U.S. Serial No. 12/869,232). The term recombinase refers to a genetic recombination enzyme that enzymatically catalyzes, in a cell, the joining of relatively short pieces of DNA between two relatively longer DNA strands. Recombinases include Cre recombinase, Hin recombinase, RecA, RAD51, Cre, and FLP. Cre recombinase is a Type I topoisomerase from P1 bacteriophage that catalyzes site-specific recombination of DNA between loxP sites. Hin recombinase is a 21kD protein composed of 198 amino acids that is found in the bacteria Salmonella. Hin belongs to the serine recombinase family of DNA invertases in which it relies on the active site serine to initiate DNA cleavage and recombination. RAD51 is a human gene. The protein encoded by this gene is a member of the RAD51 protein family which assist in repair of DNA double strand breaks. RAD51 family members are homologous to the bacterial RecA and yeast Rad51 genes. *Cre* recombinase is an enzyme that is used in experiments to delete specific sequences that are flanked by loxP sites. FLP refers to Flippase recombination enzyme (FLP or Flp) derived from the 2µ plasmid of the baker's yeast *Saccharomyces cerevisiae.*

RecA is known for its recombinase activity to catalyze strand exchange during the repair of double-strand breaks by homologous recombination (McGrew, 2003) Radding, et al., 1981; Seitz et al., 1998). RecA has also been shown to catalyze proteolysis, *e.g*., of the LexA and λ repressor proteins, and to possess DNA-dependent ATPase activity. After a double-strand break occurs from ionizing radiation or some other insult, exonucleases chew back the DNA ends 5' to 3', thereby exposing one strand of the DNA (McGrew, 2003; Cox, 1999). The single-stranded DNA becomes stabilized by single-strand binding protein (SSB). After binding of SSB, RecA binds the single-stranded (ss) DNA and forms a helical nucleoprotein filament (referred to as a filament or a presynaptic filament). During DNA repair, the homology-searching functions of RecA direct the filament to homologous DNA and catalyze homologous base pairing and strand exchange. This results in the formation of DNA heteroduplex. After strand invasion, DNA polymerase elongates the ssDNA based on the homologous DNA template to repair the DNA break, and crossover structures or Holliday junctions are formed. RecA also shows a motor function that participates in the migration of the crossover structures (Campbell and Davis, 1999).

Recombinase activity comprises a number of different functions. For example, polypeptide sequences having recombinase activity are able to bind in a non-sequence-specific fashion to single-stranded DNA to form a nucleoprotein filament. Such recombinase-bound nucleoprotein filaments are able to interact in a non-sequence-specific manner with a double-stranded DNA molecule, search for sequences in the double-stranded molecule that are homologous to sequences in the filament, and, when such sequences are found, displace one of the strands of the double-stranded molecule to allow base-pairing between sequences in the filament and complementary sequences in one of the strands of the double stranded molecule. Such steps are collectively denoted "synapsis."

RecA and RecA-like proteins (called Rad51 in many eukaryotic species) have been examined for stimulating gene targeting and homologous recombination in a variety of eukaryotic systems. In tobacco cells, expression of bacterial RecA containing a nuclear localization signal (NLS) increases the repair of mitomycin C-induced DNA damage by homologous recombination and somatic intrachromosomal recombination (recombination between homologous chromosomes) from three to ten fold (Reiss, 1996). Expression of NLSRecA in tobacco can also stimulate sister chromatid exchange 2.4-fold over wild-type levels (Reiss, 2000). In somatic mammalian cells, overexpression of NLSRecA stimulates gene-targeting by homologous recombination 10-fold (Shcherbakova, 2000). However, in human cells, overexpression of a human homologue of RecA, hRAD51, only stimulates recombination 2 to 3-fold over wild type levels under the antibiotic selection (Yanez, 1999). In zebrafish, a mutant form of the enhanced green fluorescent protein (EGFP) was corrected at low frequency by injecting ssDNA-RecA filaments directly (Cui, 2003). Rad52, a member of the Rad51 epistasis group, also promotes single-strand annealing and low level gene disruption in zebrafish using mutated oligonucleotides (Takahashi, 2005). Taken together, these studies indicate that ectopic expression of RecA or Rad51 results in a modest stimulation of homologous recombination but does not increase levels sufficiently to be useful for gene-targeting.

Thus recombinase activities include, but are not limited to, single-stranded DNA-binding, synapsis, homology searching, duplex invasion by single-stranded DNA, heteroduplex formation, ATP hydrolysis and proteolysis. The prototypical recombinase is the RecA protein from *E*. *coli.* See, for example, U.S. Patent No. 4,888,274. Prokaryotic RecA-like proteins have also been described in *Salmonella, Bacillus* and *Proteus* species. A thermostable RecA protein, from *Thermus aquaticus,* has been described in U.S. Patent No. 5,510,473. A bacteriophage T4 homologue of RecA, the UvsX protein, has been described. RecA mutants, having altered recombinase activities, have been described, for example, in U.S. Patent Nos. 6,774,213; 7,176,007 and 7,294,494. Plant RecA homologues are described in, for example, U.S. Patent Nos. 5,674,992; 6,388,169 and 6,809,183. RecA fragments containing recombinase activity have been described, for example, in U.S. Patent No. 5,731,411. Mutant RecA proteins having enhanced recombinase activity such as, for example, RecA803 have been described. *See,* for example, Madiraju et al. (1988) Proc. Natl. Acad. Sci. USA 85:6592-6596.

A eukaryotic homologue of RecA, also possessing recombinase activity, is the Rad51 protein, first identified in the yeast *Saccharomyces cerevisiae.* See Bishop et al., (1992) Cell 69: 439-56 and Shinohara et al, (1992) Cell: 457-70 Aboussekhra, et al., (1992) Mol. Cell. Biol. 72, 3224-3234. Basile et al., (1992) Mol. Cell. Biol. 12, 3235-3246.Plant Rad51 sequences are described in U.S. Patent Nos. 6,541,684; 6,720,478; 6,905,857 and 7,034,117. Another yeast protein that is homologous to RecA is the Dmcl protein. RecA/Rad51 homologues in organisms other than *E. coli* and *S. cerevisiae* have been described. Morita et al. (1993) Proc. Natl. Acad. Sci. USA 90:6577-6580; Shinohara et al. (1993) Nature Genet. 4:239-243; Heyer (1994) Experientia 50:223-233; Maeshima et al. (1995) Gene 160:195-200; U.S. Patent Nos. 6,541,684 and 6,905,857.

Herein, "RecA" or "RecA protein" refers to a family of RecA-like recombination proteins having essentially all or most of the same functions, particularly: (i) the ability to position properly oligonucleotides or polynucleotides on their homologous targets for subsequent extension by DNA polymerases; (ii) the ability topologically to prepare duplex nucleic acid for DNA synthesis; and, (iii) the ability of RecA/oligonucleotide or RecA/polynucleotide complexes efficiently to find and bind to complementary sequences. The best characterized RecA protein is from *E. coli;* in addition to the original allelic form of the protein a number of mutant RecA-like proteins have been identified, for example, RecA803. Further, many organisms have RecA-like strand-transfer proteins including, for example, yeast, *Drosophila,* mammals including humans, and plants. These proteins include, for example, Reel, Rec2, Rad51, Rad51B, Rad51C, Rad51D, Rad51E, XRCC2 and DMC1. An embodiment of the recombination protein is the RecA protein of *E. coli.* Alternatively, the RecA protein can be the mutant RecA-803 protein of *E. coli,* a RecA protein from another bacterial source or a homologous recombination protein from another organism.

Additional descriptions of proteins having recombinase activity are found, for example, in Fugisawa et al. (1985) Nucl. Acids Res. 13:7473; Hsieh et al. (1986) Cell 44:885; Hsieh et al. (1989) J. Biol. Chem. 264:5089; Fishel et al. (1988) Proc. Natl. Acad. Sci. USA 85:3683; Cassuto et al. (1987) Mol. Gen. Genet. 208:10; Ganea et al. (1987) Mol. Cell Biol. 7:3124; Moore et al. (1990) J. Biol. Chem.: 11108; Keene et al. (1984) Nucl. Acids Res. 12:3057; Kimiec (1984) Cold Spring Harbor Symp. 48:675; Kimeic (1986) Cell 44:545; Kolodner et al. (1987) Proc. Natl. Acad. Sci. USA 84:5560; Sugino et al. (1985) Proc. Natl. Acad, Sci. USA 85: 3683; Halbrook et al. (1989) J. Biol. Chem. 264:21403; Eisen et al. (1988) Proc. Natl. Acad. Sci. USA 85:7481; McCarthy et al. (1988) Proc. Natl. Acad. Sci. USA 85:5854; and Lowenhaupt et al. (1989) J. Biol. Chem. 264:20568. See also Brendel et al. (1997) J. Mol. Evol. 44:528.

Examples of proteins having recombinase activity include recA, recA803, uvsX, and other recA mutants and recA-like recombinases (Roca (1990) Crit. Rev. Biochem. Molec. Biol. 25:415), (Kolodner et al. (1987) Proc. Natl. Acad. Sci. U.S.A. 84:5560; Tishkoff et al. (1991) Molec. Cell. Biol. 11:2593), RuvC (Dunderdale et al. (1991) Nature 354:506), DST2, KEM1 and XRN1 (Dykstra et al. (1991) Molec. Cell. Biol. 11:2583), STPa/DST1 (Clark et al. (1991) Molec. Cell. Biol. 11:2576), HPP-1 (Moore et al. (1991) Proc. Natl. Acad. Sci. U.S.A. 88:9067), other eukaryotic recombinases (Bishop et al. (1992) Cell 69:439; and Shinohara et al. (1992) Cell 69:457).

*In vitro*-evolved proteins having recombinase activity have been described in U.S. Patent No. 6,686,515. Further publications relating to recombinases include, for example, U.S. Patent Nos. 7,732,585, 7,361,641, 7,144,734. For a review of recombinases, see Cox (2001) Proc. Natl. Acad. Sci. USA 98:8173-8180.

A nucleoprotein filament, or "filament" may be formed. The term filament, in the context of forming a structure with a recombinase, is a term known to artisans in these fields. The nucleoprotein filament so formed can then be, e.g., contacted with another nucleic acid or introduced into a cell. Methods for forming nucleoprotein filaments, wherein the filaments comprise polypeptide sequences having recombinase activity and a nucleic acid, are well-known in the art. See, e.g., Cui et al. (2003) Marine Biotechnol. 5:174-184 and U.S. Patent Nos. 4,888,274; 5,763,240; 5,948,653 and 7,199,281 disclosing exemplary techniques for binding recombinases to nucleic acids to form nucleoprotein filaments.

In general, a molecule having recombinase activity is contacted with a linear, single-stranded nucleic acid. The linear, single-stranded nucleic acid may be a probe. The methods of preparation of such single stranded nucleic acids are known. The reaction mixture typically contains a magnesium ion. Optionally, the reaction mixture is buffered and optionally also contains ATP, dATP or a nonhydrolyzable ATP analogue, such as, for example, γ-thio-ATP (ATP-γ-S) or γ-thio-GTP (GTP-γ-S). Reaction mixtures can also optionally contain an ATP-generating system. Double-stranded DNA molecules can be denatured (e.g., by heat or alkali) either prior to, or during, filament formation. Optimization of the molar ratio of recombinase to nucleic acid is within the skill of the art. For example, a series of different concentrations of recombinase can be added to a constant amount of nucleic acid, and filament formation assayed by mobility in an agarose or acrylamide gel. Because bound protein retards the electrophoretic mobility of a polynucleotide, filament formation is evidenced by retarded mobility of the nucleic acid. Either maximum degree of retardation, or maximum amount of nucleic acid migrating with a retarded mobility, can be used to indicate optimal recombinase:nucleic acid ratios. Protein-DNA association can also be quantitated by measuring the ability of a polynucleotide to bind to nitrocellulose.

### TALENs

The term TALEN, as used herein, is broad and includes a monomeric TALEN that can cleave double stranded DNA without assistance from another TALEN. The term TALEN is also used to refer to one or both members of a pair of TALENs that are engineered to work together to cleave DNA at the same site. TALENs that work together may be referred to as a left-TALEN and a right-TALEN, which references the handedness of DNA or a TALEN-pair.

Miller et al. (Miller et al. (2011) Nature Biotechnol 29:143) reported making TALENs for site-specific nuclease architecture by linking truncated TAL variants to the catalytic domain of FokI nuclease. The resulting TALENs were shown to induce gene modification in immortalized human cells by means of the two major eukaryotic DNA repair pathways, non-homologous end joining (NHEJ) and homology directed repair. The TALENs can be engineered for specific binding. Specific binding, as that term is commonly used in the biological arts, refers to a molecule that binds to a target with a relatively high affinity compared to non-target tissues, and generally involves a plurality of non-covalent interactions, such as electrostatic interactions, van der Waals interactions, hydrogen bonding, and the like. Specific binding interactions characterize antibody-antigen binding, enzyme-substrate binding, and specifically binding protein-receptor interactions.

The cipher for TALs has been reported (PCT Application WO 2011/072246) wherein each DNA binding repeat is responsible for recognizing one base pair in the target DNA sequence. The residues may be assembled to target a DNA sequence, with: (a) HD for recognition of C/G; (b) NI for recognition of A/T; (c) NG for recognition of T/A; (d) NS for recognition of C/G or A/T or T/A or G/C; (e) NN for 30 recognition of G/C or A/T; (f) IG for recognition of T/A; (g) N for recognition of C/G; (h) HG for recognition of C/G or T/A; (i) H for recognition of T/A; and (j) NK for recognition of G/C. In brief, a target site for binding of a TALEN is determined and a fusion molecule comprising a nuclease and a series of RVDs that recognize the target site is created. Upon binding, the nuclease cleaves the DNA so that cellular repair machinery can operate to make a genetic modification at the cut ends. The term TALEN means a protein comprising a Transcription Activator-like (TAL) effector binding domain and a nuclease domain and includes monomeric TALENs that are functional *per se* as well as others that require dimerization with another monomeric TALEN. The dimerization can result in a homodimeric TALEN when both monomeric TALEN are identical or can result in a heterodimeric TALEN when monomeric TALEN are different.

In some embodiments, a monomeric TALEN can be used. TALEN typically function as dimers across a bipartite recognition site with a spacer, such that two TAL effector domains are each fused to a catalytic domain of the *FokI* restriction enzyme, the DNA-recognition sites for each resulting TALEN are separated by a spacer sequence, and binding of each TALEN monomer to the recognition site allows *FokI* to dimerize and create a double-strand break within the spacer. Monomeric TALENs also can be constructed, however, such that single TAL effectors are fused to a nuclease that does not require dimerization to function. One such nuclease, for example, is a single-chain variant of *FokI* in which the two monomers are expressed as a single polypeptide. Other naturally occurring or engineered monomeric nucleases also can serve this role. The DNA recognition domain used for a monomeric TALEN can be derived from a naturally occurring TAL effector. Alternatively, the DNA recognition domain can be engineered to recognize a specific DNA target. Engineered single-chain TALENs may be easier to construct and deploy, as they require only one engineered DNA recognition domain. A dimeric DNA sequence-specific nuclease can be generated using two different DNA binding domains (e.g., one TAL effector binding domain and one binding domain from another type of molecule). TALENs may function as dimers across a bipartite recognition site with a spacer. This nuclease architecture also can be used for target-specific nucleases generated from, for example, one TALEN monomer and one zinc finger nuclease monomer. In such cases, the DNA recognition sites for the TALEN and zinc finger nuclease monomers can be separated by a spacer of appropriate length. Binding of the two monomers can allow *FokI* to dimerize and create a double-strand break within the spacer sequence. DNA binding domains other than zinc fingers, such as homeodomains, myb repeats or leucine zippers, also can be fused to *FokI* and serve as a partner with a TALEN monomer to create a functional nuclease.

In some embodiments, a TAL effector can be used to target other protein domains (e.g., non-nuclease protein domains) to specific nucleotide sequences. For example, a TAL effector can be linked to a protein domain from, without limitation, a DNA 20 interacting enzyme (e.g., a methylase, a topoisomerase, an integrase, a transposase, or a ligase), a transcription activators or repressor, or a protein that interacts with or modifies other proteins such as histones. Applications of such TAL effector fusions include, for example, creating or modifying epigenetic regulatory elements, making site-specific insertions, deletions, or repairs in DNA, controlling gene expression, and modifying chromatin structure.

The spacer of the target sequence can be selected or varied to modulate TALEN specificity and activity. The flexibility in spacer length indicates that spacer length can be chosen to target particular sequences with high specificity. Further, the variation in activity has been observed for different spacer lengths indicating that spacer length can be chosen to achieve a desired level of TALEN activity.

The TALENs described herein as Carlson +63 were surprisingly found to be very efficient in use. A comparison to the most similar TALENs is shown in Figures 16 and 18. Referring to Figure 16 and using the position numbers therein, there is a leading N-terminal portion from about 1 to about 42, a 5' portion from about 43 to about 178, and RVD portion from about 179 to about 197, a +63 domain from about 198 to about 261, and a FokI portion from about 262 to the end at about 400. A number of residues are different between the sequences, for instance at about 10 positions that are circled in Figure 16A. The N-terminal leader portion is also different, with the Carlson +63 TALEN being about 20 residues shorter and having about 16 other differences. Embodiments of the N-terminal leader portion are sequences of between about 10 to about 30 residues; artisans will immediately appreciate that all the ranges and values within the explicitly stated ranges are contemplated.

Figure 17 provides a sequence listing for the vector used with the Carlson +63 sequence. Some parts of the vector are indicated in the Figure: the T3 primer binding site, a 5' UTR, the TALEN 5' for Carlson +63, a LacZ- stuffer fragment (see Cermak et. al. 2011 for blue white screening of clones), a Fok I homodimer, a 3' TALEN +18-+63 (note that 3' TALEN +1-17 provided by last TALEN repeat in final cloning step), a 3' UTR-polyA, and a Poly-C that potentially protects the mRNA from degradation. In use, as is known to artisans, the amino acids that provide specific binding are inserted in between the portions labeled as the 5' portion and the half RVD sequence. Figure 17 shows the Carlson +63 TALEN scaffold with various features for production of mRNA. The vector has some features in common with a pT3TS plasmid previously described (Hyatt, T. M. & Ekker, S. C. Vectors and techniques for ectopic gene expression in zebrafish. Methods Cell Biol 59, 117-126 (1999)). A significant improvement to the Hyatt et al. vector was made by removal of a LacZ promoter that was previously located 5' of the T3 promoter sequence indicated in Figure 17. Removal of the LacZ promoter was found to be required for reliable cloning of gene specific TALENs and propagation of the plasmid. The Carlson +63 vector has a T3 site for mRNA transcription with T3 mRNA polymerase. The features include a T3 promoter binding site from which transcription can be initiated, 5' and 3' UTR sequence from the *Xenopus* β-globin gene, and a poly-C stretch. The 5' and 3' portions of the TALEN scaffold flank a LacZ stuffer fragment that is removed when the gene specific RVD sequences are cloned in as described in Cermak, T. et al. Efficient design and assembly of custom TALEN and other TAL effector-based constructs for DNA targeting. Nucleic Acids Research 39, e82 (2011).

Alternative embodiments use alternative mRNA polymerases and cognate binding sites such as T7 or SP6. Other embodiments relate to the use of any of several alterations of the UTR sequences; these could benefit translation of the mRNA. Some examples are: addition of a cytoplasmic polyadenylation element binding site in the 3' UTR, or exchanging the Xenopus β-globin UTRs with UTR sequences from human, pig, cow, sheep, goat, zebrafish, from genes including B-globin. UTRs from genes may be selected for regulation of expression in embryonic development or in cells. Some examples of UTRs that may be useful include β-actin, DEAH, TPT1, ZF42, SKP1, TKT, TP3, DDX5, EIF3A, DDX39, GAPDH, CDK1, Hsp90ab1, Ybxl f Eif4b Rps27a Stra13, Myc, Pafl and Foxo1, or CHUK. Such vector or mRNA improvements could be used to direct special or temporal expression of ectopic TALENs for study of gene depletion at desired stages of development. TALEN mRNA produced by these vectors are generally useful as described herein, including, for example, for creation of knockout or knockin cells lines or animals to generate models of disease, animal improvement or screening of for genes that interact with environmental stimuli (example; drugs, heat, cold, UV light, growth factors, stress).

Embodiments include a vector comprising a sequence having 85% to 100% identity with the Carlson +63 vector or TALEN; artisans will immediately appreciate that all the ranges and values within the explicitly stated ranges are contemplated, e.g., 85%, 90%, and 95%. Embodiments include a Carlson+63 TALEN with a number of conservative substitutions ranging from 1 to 50; artisans will immediately appreciate that all the ranges and values within the explicitly stated ranges are contemplated, e.g., 5 to 10, 1 to 20, or about 12. Artisans will immediately appreciate that the RVD portions of these sequences are to be excluded from these comparisons since the RVD sequences are to be changed according to the target intended by a user. Embodiments include a TALEN that comprises at least one portion of a Carlson +63 TALEN chosen from the group consisting of N-terminal leader portion, 5' portion, and +63 domain (and % variations/substitutions thereof). The Carlson +63 TALEN has a 22-residue N-terminal leader sequence of MASSPPKKKRKVSWKDASGWSR (SEQ ID NO: 132). Embodiments include a TALEN vector or mRNA that comprises at least one portion of a Carlson +63 TALEN vector chosen from the group consisting of 3' primer biding site, 5'UTR, lacz stuffer fragment, 3' TALEN, 3'UTR, PolyC, and nucleic acids encoding the Carlson +63 N-terminal leader portion, 5' portion, or +63 domain (and variations/substitutions thereof). Alternatively, a sequence may be assembled using one or more of the alternatives indicated above, e.g., for T7 or SP6 or any of the various alternative UTRs. Embodiments include sequences with between 85% and 100% identity to the same, as well as a number of conservative substitutions ranging from 0 to 50.

The term nuclease includes exonucleases and endonucleases. The term endonuclease refers to any wild-type or variant enzyme capable of catalyzing the hydrolysis (cleavage) of bonds between nucleic acids within a DNA or RNA molecule, preferably a DNA molecule. Non-limiting examples of endonucleases include type II restriction endonucleases such as *Fok*I, *Hha*I, *Hin*dlII*, Not*I, *Bbv*Cl, *Eco*RI, *Bgl*II*,* and *Alw*I. Endonucleases comprise also rare-cutting endonucleases when having typically a polynucleotide recognition site of about 12-45 basepairs (bp) in length, more preferably of 14-45 bp. Rare-cutting endonucleases induce DNA double-strand breaks (DSBs) at a defined locus. Rare-cutting endonucleases can for example be a homing endonuclease, a chimeric Zinc-Finger nuclease (ZFN) resulting from the fusion of engineered zinc-finger domains with the catalytic domain of a restriction enzyme such as FokI or a chemical endonuclease. In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences. Such chemical endonucleases are comprised in the term "endonuclease" according to the present invention. Examples of such endonuclease include *I-See I, I-Chu L I-Cre I, I-Csm I, PI-See L PI-Tti L PI-Mtu I, I-Ceu I, I-See IL 1- See III, HO, PI-Civ I, PI-Ctr L PI-Aae I, PI-Bsu I, PI-Dha I, PI-Dra L PI-Mav L PI-Meh I, PI-Mfu L PI-Mfl I, PI-Mga L PI-Mgo I, PI-Min L PI-Mka L PI-Mle I, PI-Mma I, PI-30 Msh L PI-Msm I, PI-Mth I, PI-Mtu I, PI-Mxe I, PI-Npu I, PI-Pfu L PI-Rma I, PI-Spb I, PI-Ssp L PI-Fae L PI-Mja I, PI-Pho L PI-Tag L PI-Thy I, PI-Tko I, PI-Tsp I, I-MsoI.*

Many working examples for TALENs introduction into cells or embryos, and the formation of animals therefrom are provided herein. Cells for treatment by TALENs include a cultured cell, an immortalized cell, a primary cell, a primary somatic cell, a zygote, a germ cell, a primordial germ cell, a blastocyst, or a stem cell. Example 19 (Figure 26) details experimental results for modifying spermatogonial stem cells. These cells offer a another method for genetic modification of animals, e.g., livestock. Genetic modification or gene edits can be executed in vitro in spermatogonial stem cells (male germ-line stem cells, herein abbreviated GSC's) isolated from donor testes. Modified cells are transplanted into germ-cell depleted testes of a recipient. Implanted spermatogonial stem cells produce sperm that carry the genetic modification(s) that can be used for breeding via artificial insemination (AI) or in vitro fertilization (IVF) to derive founder animals. This method has advantages beyond generation of genetically modified founders. One such advantage is apparent when founders for a particular disease model are unhealthy and not suitable for growth to reproductive age. The same modifications introduced into GSC's could thus be implanted into the testes of a healthy individuals allowing propagation of the line from a healthy animal to generate disease models in newborn piglets.

The possibility and efficiency of generating TALEN-mediated indels in spermatogonial stem cells was first explored by transfection of plasmids encoding TALENs targeted to exon 7 of the porcine Duchene Muscular Dystrophy locus (DMD). Testing of several nuclefection conditions, plasmid quantities and incubation temperature yielded a maximum efficiency of 19% NHEJ despite a germ cell transfection rate of 25%, as shown in Fig. 26. TALEN activity was highest in replicates cultured at 30°C. GSCs remained viable after over 5 days of culture at 30°C, though overall, germ cell survival was higher at 37°C. Transfection of TALEN encoding mRNA, versus plasmid DNA, resulted in both greater activity and viability of livestock somatic cells and GSCs. Notably, while peak activity of mRNA transfection did not exceed plasmid DNA transfection in this experiment, a significantly lower quantity of mRNA was required to achieve the same level of modification. Example 20 details successful TALEN-stimulated HDR in primordial germ cells (avian).

### Genetically modified animals

Various techniques known in the art can be used to introduce nucleic acid constructs into non-human animals to produce founder animals, in which the nucleic acid construct is integrated into the genome. Such techniques include, without limitation, pronuclear microinjection (U.S. Patent No. 4,873,191), retrovirus mediated gene transfer into germ lines (Van der Putten et al. (1985) Proc. Natl. Acad. Sci. USA 82, 6148-1652), gene targeting into embryonic stem cells (Thompson et al. (1989) Cell 56, 313-321), electroporation of embryos (Lo (1983) Mol. Cell. Biol. 3, 1803-1814), sperm-mediated gene transfer (Lavitrano et al. (2002) Proc. Natl. Acad. Sci. USA 99, 14230-14235; Lavitrano et al. (2006) Reprod. Fert. Develop. 18, 19-23), and *in vitro* transformation of somatic cells, such as cumulus or mammary cells, or adult, fetal, or embryonic stem cells, followed by nuclear transplantation (Wilmut et al. (1997) Nature 385, 810-813; and Wakayama et al. (1998) Nature 394, 369-374). Pronuclear microinjection, sperm mediated gene transfer, and somatic cell nuclear transfer are particularly useful techniques, as well as cytoplasmic injection, primordial germ cell transplantation (Brinster), and blastocyst chimera production whereby a germ cell is propagated in an embryo.

Typically, in pronuclear microinjection, a nucleic acid construct is introduced into a fertilized egg; 1 or 2 cell fertilized eggs are used as the pronuclei containing the genetic material from the sperm head and the egg are visible within the protoplasm. Pronuclear staged fertilized eggs can be obtained *in vitro* or *in vivo* (i.e., surgically recovered from the oviduct of donor animals). *In vitro* fertilized eggs can be produced as follows. For example, swine ovaries can be collected at an abattoir, and maintained at 22-28°C during transport. Ovaries can be washed and isolated for follicular aspiration, and follicles ranging from 4-8 mm can be aspirated into 50 mL conical centrifuge tubes using 18 gauge needles and under vacuum. Follicular fluid and aspirated oocytes can be rinsed through pre-filters with commercial TL-HEPES (Minitube, Verona, WI). Oocytes surrounded by a compact cumulus mass can be selected and placed into TCM-199 OOCYTE MATURATION MEDIUM (Minitube, Verona, WI) supplemented with 0.1 mg/mL cysteine, 10 ng/mL epidermal growth factor, 10% porcine follicular fluid, 50 µM 2-mercaptoethanol, 0.5 mg/ml cAMP, 10 IU/mL each of pregnant mare serum gonadotropin (PMSG) and human chorionic gonadotropin (hCG) for approximately 22 hours in humidified air at 38.7°C and 5% CO₂. Subsequently, the oocytes can be moved to fresh TCM-199 maturation medium, which will not contain cAMP, PMSG or hCG and incubated for an additional 22 hours. Matured oocytes can be stripped of their cumulus cells by vortexing in 0.1% hyaluronidase for 1 minute.

For swine, mature oocytes can be fertilized in 500 µl Minitube PORCPRO IVF MEDIUM SYSTEM (Minitube, Verona, WI) in Minitube 5-well fertilization dishes. In preparation for *in vitro* fertilization (IVF), freshly-collected or frozen boar semen can be washed and resuspended in PORCPRO IVF Medium to 4 x 10⁵ sperm. Sperm concentrations can be analyzed by computer assisted semen analysis (SPERMVISION, Minitube, Verona, WI). Final *in vitro* insemination can be performed in a 10µl volume at a final concentration of approximately 40 motile sperm/oocyte, depending on boar. Incubate all fertilizing oocytes at 38.7°C in 5.0% CO₂ atmosphere for 6 hours. Six hours post-insemination, presumptive zygotes can be washed twice in NCSU-23 and moved to 0.5 mL of the same medium. This system can produce 20-30% blastocysts routinely across most boars with a 10-30% polyspermic insemination rate.

Linearized nucleic acid constructs can be injected into one of the pronuclei, or. e.g., transposons or cytoplasmic injection may be used. Then the injected eggs can be transferred to a recipient female (e.g., into the oviducts of a recipient female) and allowed to develop in the recipient female to produce the transgenic animals. In particular, *in vitro* fertilized embryos can be centrifuged at 15,000 X g for 5 minutes to sediment lipids allowing visualization of the pronucleus. The embryos can be injected with using an Eppendorf FEMTOJET injector and can be cultured until blastocyst formation. Rates of embryo cleavage and blastocyst formation and quality can be recorded.

Embryos can be surgically transferred into uteri of asynchronous recipients. Typically, 100-200 (e.g., 150-200) embryos can be deposited into the ampulla-isthmus junction of the oviduct using a 5.5-inch TOMCAT® catheter. After surgery, real-time ultrasound examination of pregnancy can be performed.

In somatic cell nuclear transfer, a transgenic artiodactyl cell (e.g., a transgenic pig cell or bovine cell) such as an embryonic blastomere, fetal fibroblast, adult ear fibroblast, or granulosa cell that includes a nucleic acid construct described above, can be introduced into an enucleated oocyte to establish a combined cell. Oocytes can be enucleated by partial zona dissection near the polar body and then pressing out cytoplasm at the dissection area. Typically, an injection pipette with a sharp beveled tip is used to inject the transgenic cell into an enucleated oocyte arrested at meiosis 2. In some conventions, oocytes arrested at meiosis-2 are termed "eggs." After producing a porcine or bovine embryo (e.g., by fusing and activating the oocyte), the embryo is transferred to the oviducts of a recipient female, about 20 to 24 hours after activation. See, for example, Cibelli et al. (1998) Science 280, 1256-1258 and U.S. Patent No. 6,548,741. For pigs, recipient females can be checked for pregnancy approximately 20-21 days after transfer of the embryos.

Standard breeding techniques can be used to create animals that are homozygous for the target nucleic acid from the initial heterozygous founder animals. Homozygosity may not be required, however. Transgenic pigs described herein can be bred with other pigs of interest.

In some embodiments, a nucleic acid of interest and a selectable marker can be provided on separate transposons and provided to either embryos or cells in unequal amount, where the amount of transposon containing the selectable marker far exceeds (5-10 fold excess) the transposon containing the nucleic acid of interest. Transgenic cells or animals expressing the nucleic acid of interest can be isolated based on presence and expression of the selectable marker. Because the transposons will integrate into the genome in a precise and unlinked way (independent transposition events), the nucleic acid of interest and the selectable marker are not genetically linked and can easily be separated by genetic segregation through standard breeding. Thus, transgenic animals can be produced that are not constrained to retain selectable markers in subsequent generations, an issue of some concern from a public safety perspective.

Once transgenic animal have been generated, expression of a target nucleic acid can be assessed using standard techniques. Initial screening can be accomplished by Southern blot analysis to determine whether or not integration of the construct has taken place. For a description of Southern analysis, see sections 9.37-9.52 of Sambrook et al., 1989, Molecular Cloning, A Laboratory Manual, second edition, Cold Spring Harbor Press, Plainview; NY. Polymerase chain reaction (PCR) techniques also can be used in the initial screening. PCR refers to a procedure or technique in which target nucleic acids are amplified. Generally, sequence information from the ends of the region of interest or beyond is employed to design oligonucleotide primers that are identical or similar in sequence to opposite strands of the template to be amplified. PCR can be used to amplify specific sequences from DNA as well as RNA, including sequences from total genomic DNA or total cellular RNA. Primers typically are 14 to 40 nucleotides in length, but can range from 10 nucleotides to hundreds of nucleotides in length. PCR is described in, for example PCR Primer: A Laboratory Manual, ed. Dieffenbach and Dveksler, Cold Spring Harbor Laboratory Press, 1995. Nucleic acids also can be amplified by ligase chain reaction, strand displacement amplification, self-sustained sequence replication, or nucleic acid sequence-based amplified. See, for example, Lewis (1992) Genetic Engineering News 12:1; Guatelli et al. (1990) Proc. Natl. Acad. Sci. USA 87:1874; and Weiss (1991) Science 254:1292. At the blastocyst stage, embryos can be individually processed for analysis by, e.g., PCR, Southern hybridization and splinkerette PCR (see, e.g., Dupuy et al. Proc Natl Acad Sci USA (2002) 99:4495).

Expression of a nucleic acid sequence encoding a polypeptide in the tissues of transgenic pigs can be assessed using techniques that include, for example, Northern blot analysis of tissue samples obtained from the animal, *in situ* hybridization analysis, Western analysis, immunoassays such as enzyme-linked immunosorbent assays, and reverse-transcriptase PCR (RT-PCR).

### Vectors and Nucleic acids

A variety of nucleic acids may be introduced into the artiodactyl or other cells, for knockout purposes, or to obtain expression of a gene for other purposes. Nucleic acid constructs that can be used to produce transgenic animals include a target nucleic acid sequence. As used herein, the term nucleic acid includes DNA, RNA, and nucleic acid analogs, and nucleic acids that are double-stranded or single-stranded (i.e., a sense or an antisense single strand). Nucleic acid analogs can be modified at the base moiety, sugar moiety, or phosphate backbone to improve, for example, stability, hybridization, or solubility of the nucleic acid. Modifications at the base moiety include deoxyuridine for deoxythymidine, and 5-methyl-2'-deoxycytidine and 5-bromo-2'-doxycytidine for deoxycytidine. Modifications of the sugar moiety include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, Summerton and Weller (1997) Antisense Nucleic Acid Drug Dev. 7(3):187; and Hyrup et al. (1996) Bioorgan. Med. Chem. 4:5. In addition, the deoxyphosphate backbone can be replaced with, for example, a phosphorothioate or phosphorodithioate backbone, a phosphoroamidite, or an alkyl phosphotriester backbone.

The target nucleic acid sequence can be operably linked to a regulatory region such as a promoter. Regulatory regions can be porcine regulatory regions or can be from other species. As used herein, operably linked refers to positioning of a regulatory region relative to a nucleic acid sequence in such a way as to permit or facilitate transcription of the target nucleic acid.

Any type of promoter can be operably linked to a target nucleic acid sequence. Examples of promoters include, without limitation, tissue-specific promoters, constitutive promoters, and promoters responsive or unresponsive to a particular stimulus. Suitable tissue specific promoters can result in preferential expression of a nucleic acid transcript in beta cells and include, for example, the human insulin promoter. Other tissue specific promoters can result in preferential expression in, for example, hepatocytes or heart tissue and can include the albumin or alpha-myosin heavy chain promoters, respectively. In other embodiments, a promoter that facilitates the expression of a nucleic acid molecule without significant tissue- or temporal-specificity can be used (i.e., a constitutive promoter). For example, a beta-actin promoter such as the chicken beta-actin gene promoter, ubiquitin promoter, miniCAGs promoter, glyceraldehyde-3-phosphate dehydrogenase (GAPDH) promoter, or 3-phosphoglycerate kinase (PGK) promoter can be used, as well as viral promoters such as the herpes simplex virus thymidine kinase (HSV-TK) promoter, the SV40 promoter, or a cytomegalovirus (CMV) promoter. In some embodiments, a fusion of the chicken beta actin gene promoter and the CMV enhancer is used as a promoter. See, for example, Xu et al. (2001) Hum. Gene Ther. 12:563; and Kiwaki et al. (1996) Hum. Gene Ther. 7:821.

An example of an inducible promoter is the tetracycline (tet)-on promoter system, which can be used to regulate transcription of the nucleic acid. In this system, a mutated Tet repressor (TetR) is fused to the activation domain of herpes simplex virus VP16 transactivator protein to create a tetracycline-controlled transcriptional activator (tTA), which is regulated by tet or doxycycline (dox). In the absence of antibiotic, transcription is minimal, while in the presence of tet or dox, transcription is induced. Alternative inducible systems include the ecdysone or rapamycin systems. Ecdysone is an insect molting hormone whose production is controlled by a heterodimer of the ecdysone receptor and the product of the *ultraspiracle* gene (USP). Expression is induced by treatment with ecdysone or an analog of ecdysone such as muristerone A. The agent that is administered to the animal to trigger the inducible system is referred to as an induction agent.

Additional regulatory regions that may be useful in nucleic acid constructs, include, but are not limited to, polyadenylation sequences, translation control sequences (e.g., an internal ribosome entry segment, IRES), enhancers, inducible elements, or introns. Such regulatory regions may not be necessary, although they may increase expression by affecting transcription, stability of the mRNA, translational efficiency, or the like. Such regulatory regions can be included in a nucleic acid construct as desired to obtain optimal expression of the nucleic acids in the cell(s). Sufficient expression, however, can sometimes be obtained without such additional elements.

A nucleic acid construct may be used that encodes signal peptides or selectable markers. Signal peptides can be used such that an encoded polypeptide is directed to a particular cellular location (e.g., the cell surface). Non-limiting examples of selectable markers include puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), and xanthin-guanine phosphoribosyltransferase (XGPRT). Such markers are useful for selecting stable transformants in culture. Other selectable markers include fluorescent polypeptides, such as green fluorescent protein or yellow fluorescent protein.

In some embodiments, a sequence encoding a selectable marker can be flanked by recognition sequences for a recombinase such as, e.g., Cre or Flp. For example, the selectable marker can be flanked by *loxP* recognition sites (34-bp recognition sites recognized by the Cre recombinase) or FRT recognition sites such that the selectable marker can be excised from the construct. See, Orban, et al., Proc. Natl. Acad. Sci. (1992) 89:6861, for a review of Cre/lox technology, and Brand and Dymecki, Dev. Cell (2004) 6:7. A transposon containing a Cre- or Flp-activatable transgene interrupted by a selectable marker gene also can be used to obtain transgenic animals with conditional expression of a transgene. For example, a promoter driving expression of the marker/transgene can be either ubiquitous or tissue-specific, which would result in the ubiquitous or tissue-specific expression of the marker in F0 animals (e.g., pigs). Tissue specific activation of the transgene can be accomplished, for example, by crossing a pig that ubiquitously expresses a marker-interrupted transgene to a pig expressing Cre or Flp in a tissue-specific manner, or by crossing a pig that expresses a marker-interrupted transgene in a tissue-specific manner to a pig that ubiquitously expresses Cre or Flp recombinase. Controlled expression of the transgene or controlled excision of the marker allows expression of the transgene.

In some embodiments, the target nucleic acid encodes a polypeptide. A nucleic acid sequence encoding a polypeptide can include a tag sequence that encodes a "tag" designed to facilitate subsequent manipulation of the encoded polypeptide (e.g., to facilitate localization or detection). Tag sequences can be inserted in the nucleic acid sequence encoding the polypeptide such that the encoded tag is located at either the carboxyl or amino terminus of the polypeptide. Non-limiting examples of encoded tags include glutathione S-transferase (GST) and FLAG™ tag (Kodak, New Haven, CT).

In other embodiments, the target nucleic acid sequence induces RNA interference against a target nucleic acid such that expression of the target nucleic acid is reduced. For example the target nucleic acid sequence can induce RNA interference against a nucleic acid encoding a cystic fibrosis transmembrane conductance regulatory (CFTR) polypeptide. For example, double-stranded small interfering RNA (siRNA) or short hairpin RNA (shRNA) homologous to a CFTR DNA can be used to reduce expression of that DNA. Constructs for siRNA can be produced as described, for example, in Fire et al. (1998) Nature 391:806; Romano and Masino (1992) Mol. Microbiol. 6:3343; Cogoni et al. (1996) EMBO J. 15:3153; Cogoni and Masino (1999) Nature 399:166; Misquitta and Paterson (1999) Proc. Natl. Acad. Sci. USA 96:1451; and Kennerdell and Carthew (1998) Cell 95:1017. Constructs for shRNA can be produced as described by McIntyre and Fanning (2006) BMC Biotechnology 6:1. In general, shRNAs are transcribed as a single-stranded RNA molecule containing complementary regions, which can anneal and form short hairpins.

Nucleic acid constructs can be methylated using an *Sss*I CpG methylase (New England Biolabs, Ipswich, MA). In general, the nucleic acid construct can be incubated with S-adenosylmethionine and *Sss*I CpG-methylase in buffer at 37°C. Hypermethylation can be confirmed by incubating the construct with one unit of *Hin*P1I endonuclease for 1 hour at 37°C and assaying by agarose gel electrophoresis.

Nucleic acid constructs can be introduced into embryonic, fetal, or adult artiodactyl cells of any type, including, for example, germ cells such as an oocyte or an egg, a progenitor cell, an adult or embryonic stem cell, a primordial germ cell, a kidney cell such as a PK-15 cell, an islet cell, a beta cell, a liver cell, or a fibroblast such as a dermal fibroblast, using a variety of techniques. Non-limiting examples of techniques include the use of transposon systems, recombinant viruses that can infect cells, or liposomes or other non-viral methods such as electroporation, microinjection, or calcium phosphate precipitation, that are capable of delivering nucleic acids to cells.

In transposon systems, the transcriptional unit of a nucleic acid construct, i.e., the regulatory region operably linked to a target nucleic acid sequence, is flanked by an inverted repeat of a transposon. Several transposon systems, including, for example, *Sleeping Beauty* (see, U.S. Patent No. 6,613,752 and U.S. Publication No. 2005/0003542); Frog Prince (Miskey et al. (2003) Nucleic Acids Res. 31:6873); *Tol2* (Kawakami (2007) Genome Biology 8(Suppl.1):S7; *Minos* (Pavlopoulos et al. (2007) Genome Biology 8(Suppl.1):S2); *Hsmar1* (Miskey et al. (2007)) Mol Cell Biol. 27:4589); and Passport have been developed to introduce nucleic acids into cells, including mice, human, and pig cells. The *Sleeping Beauty* and *Passport* transposon is particularly useful. A transposase can be delivered as a protein, encoded on the same nucleic acid construct as the target nucleic acid, can be introduced on a separate nucleic acid construct, or provided as an mRNA (e.g., an in *vitro*-transcribed and capped mRNA).

Insulator elements also can be included in a nucleic acid construct to maintain expression of the target nucleic acid and to inhibit the unwanted transcription of host genes. See, for example, U.S. Publication No. 2004/0203158. Typically, an insulator element flanks each side of the transcriptional unit and is internal to the inverted repeat of the transposon. Non-limiting examples of insulator elements include the matrix attachment region-(MAR) type insulator elements and border-type insulator elements. See, for example, U.S. Patent Nos. 6,395,549, 5,731,178, 6,100,448 and 5,610,053, and U.S. Publication No. 2004/0203158.

Nucleic acids can be incorporated into vectors. A vector is a broad term that includes any specific DNA segment that is designed to move from a carrier into a target DNA. A vector may be referred to as an expression vector, or a vector system, which is a set of components needed to bring about DNA insertion into a genome or other targeted DNA sequence such as an episome, plasmid, or even virus/phage DNA segment. Vector systems such as viral vectors (e.g., retroviruses, adeno-associated virus and integrating phage viruses), and non-viral vectors (e.g., transposons) used for gene delivery in animals have two basic components: 1) a vector comprised of DNA (or RNA that is reverse transcribed into a cDNA) and 2) a transposase, recombinase, or other integrase enzyme that recognizes both the vector and a DNA target sequence and inserts the vector into the target DNA sequence. Vectors most often contain one or more expression cassettes that comprise one or more expression control sequences, wherein an expression control sequence is a DNA sequence that controls and regulates the transcription and/or translation of another DNA sequence or mRNA, respectively.

Many different types of vectors are known. For example, plasmids and viral vectors, e.g., retroviral vectors, are known. Mammalian expression plasmids typically have an origin of replication, a suitable promoter and optional enhancer, and also any necessary ribosome binding sites, a polyadenylation site, splice donor and acceptor sites, transcriptional termination sequences, and 5' flanking non-transcribed sequences. Examples of vectors include: plasmids (which may also be a *carrier* of another type of vector), adenovirus, adeno-associated virus (AAV), lentivirus (e.g., HIV-1, SIV or FIV), retrovirus (e.g., ASV, ALV or MoMLV), and transposons (e.g., *Sleeping Beauty,* P-elements, *Tol-2, Frog Prince, piggyBac*).

As used herein, the term nucleic acid refers to both RNA and DNA, including, for example, cDNA, genomic DNA, synthetic (e.g., chemically synthesized) DNA, as well as naturally occurring and chemically modified nucleic acids, e.g., synthetic bases or alternative backbones. A nucleic acid molecule can be double-stranded or single-stranded (i.e., a sense or an antisense single strand). The term transgenic is used broadly herein and refers to a genetically modified organism or genetically engineered organism whose genetic material has been altered using genetic engineering techniques. A knockout artiodactyl is thus transgenic regardless of whether or not exogenous genes or nucleic acids are expressed in the animal or its progeny.

The nucleic acid sequences set forth herein are intended to represent both DNA and RNA sequences, according to the conventional practice of allowing the abbreviation "T" stand for "T" or for "U", as the case may be, for DNA or RNA. Polynucleotides are nucleic acid molecules of at least three nucleotide subunits. Polynucleotide analogues or polynucleic acids are chemically modified polynucleotides or polynucleic acids. In some embodiments, polynucleotide analogues can be generated by replacing portions of the sugar-phosphate backbone of a polynucleotide with alternative functional groups. Morpholino-modified polynucleotides, referred to herein as "morpholinos," are polynucleotide analogues in which the bases are linked by a morpholino-phosphorodiamidate backbone (see, e.g., U.S. Patent Nos. 5,142,047 and 5,185,444). In addition to morpholinos, other examples of polynucleotide analogues include analogues in which the bases are linked by a polyvinyl backbone, peptide nucleic acids (PNAs) in which the bases are linked by amide bonds formed by pseudopeptide 2-aminoethyl-glycine groups, analogues in which the nucleoside subunits are linked by methylphosphonate groups, analogues in which the phosphate residues linking nucleoside subunits are replaced by phosphoroamidate groups, and phosphorothioated DNAs, analogues containing sugar moieties that have 2' O-methyl group). Polynucleotides of the invention can be produced through the well-known and routinely used technique of solid phase synthesis. Alternatively, other suitable methods for such synthesis can be used (e.g., common molecular cloning and chemical nucleic acid synthesis techniques). Similar techniques also can be used to prepare polynucleotide analogues such as morpholinos or phosphorothioate derivatives. In addition, polynucleotides and polynucleotide analogues can be obtained commercially. For oligonucleotides, examples of pharmaceutically acceptable compositions are salts that include, e.g., (a) salts formed with cations such as sodium, potassium, ammonium, etc.; (b) acid addition salts formed with inorganic acids, for example, hydrochloric acid, hydrobromic acid (c) salts formed with organic acids e.g., for example, acetic acid, oxalic acid, tartaric acid; and (d) salts formed from elemental anions e.g., chlorine, bromine, and iodine.

### Polypeptides

There are a variety of conservative changes that can generally be made to an amino acid sequence without altering activity. These changes are termed conservative substitutions or mutations; that is, an amino acid belonging to a grouping of amino acids having a particular size or characteristic can be substituted for another amino acid. Substitutes for an amino acid sequence may be selected from other members of the class to which the amino acid belongs. For example, the nonpolar (hydrophobic) amino acids include alanine, leucine, isoleucine, valine, proline, phenylalanine, tryptophan, and tyrosine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Such alterations are not expected to substantially affect apparent molecular weight as determined by polyacrylamide gel electrophoresis or isoelectric point. Exemplary conservative substitutions include, but are not limited to, Lys for Arg and vice versa to maintain a positive charge; Glu for Asp and vice versa to maintain a negative charge; Ser for Thr so that a free --OH is maintained; and Gln for Asn to maintain a free NH₂. Moreover, point mutations, deletions, and insertions of the polypeptide sequences or corresponding nucleic acid sequences may in some cases be made without a loss of function of the polypeptide or nucleic acid fragment. Substitutions may include, e.g., 1, 2, 3, or more residues. The amino acid residues described herein employ either the single letter amino acid designator or the three-letter abbreviation. Abbreviations used herein are in keeping with the standard polypeptide nomenclature, J. Biol. Chem., (1969), 243, 3552-3559. All amino acid residue sequences are represented herein by formulae with left and right orientation in the conventional direction of amino-terminus to carboxy-terminus.

In some cases a determination of the percent identity of a peptide to a sequence set forth herein may be required. In such cases, the percent identity is measured in terms of the number of residues of the peptide, or a portion of the peptide. A polypeptide of, e.g., 90% identity, may also be a portion of a larger peptide. Embodiments include such polypeptides that have the indicated identity and/or conservative substitution of sequence set forth herein.

The term purified as used herein with reference to a polypeptide refers to a polypeptide that either has no naturally occurring counterpart (e.g., a peptidomimetic), or has been chemically synthesized and is thus substantially uncontaminated by other polypeptides, or has been separated or purified from other most cellular components by which it is naturally accompanied (e.g., other cellular proteins, polynucleotides, or cellular components). An example of a purified polypeptide is one that is at least 70%, by dry weight, free from the proteins and naturally occurring organic molecules with which it naturally associates. A preparation of a purified polypeptide therefore can be, for example, at least 80%, at least 90%, or at least 99%, by dry weight, the polypeptide. Polypeptides also can be engineered to contain a tag sequence (e.g., a polyhistidine tag, a myc tag, or a FLAG® tag) that facilitates the polypeptide to be purified or marked (e.g., captured onto an affinity matrix, visualized under a microscope). Thus a purified composition that comprises a polypeptide refers to a purified polypeptide unless otherwise indicated.

Polypeptides may include a chemical modification; a term that, in this context, refers to a change in the naturally-occurring chemical structure of amino acids. Such modifications may be made to a side chain or a terminus, e.g., changing the amino-terminus or carboxyl terminus. In some embodiments, the modifications are useful for creating chemical groups that may conveniently be used to link the polypeptides to other materials, or to attach a therapeutic agent.

### Research Tools and Investigation

The processes for modifying cells and embryos herein are applicable to a wide variety of research tools. The cells or embryos themselves are also useful for research. For instance, programs for traditional breeding are often very reliant on having an understanding of the genetic components of the animals that are bred. Animals with desirable traits are bred to foster the breeds. These traits are heavily dependent on the animals' genes and gene combinations. The placement of genes into cells or embryos, or modifying their genes provides valuable information about how the genes interact to produce traits that are of interest. The cells or embryos may be cultured for a suitable time and then tested. The embryos may be destroyed prior to achieving any meaningful developmental stage while nonetheless providing useful insights.

Accordingly, various disclosures are stated as follows.
1. A method of making a genetically modified non-human animal cell or embryo comprising exposing embryos or cells of the animal *in vitro* to an mRNA encoding a TALEN, with the TALEN specifically binding to a targeted chromosomal site in the embryos or cells, with the cells or embryos being genetically modified only at the targeted chromosomal site and with the method being performed without a reporter gene. 2. The method of 1 comprising the cells, and further comprising culturing the cells and isolating colonies of the cells. 3. The method of 1 or 2 with the method being performed without additives that create a positive or a negative selection pressure to select genetically modified cells. 4. The method of any of 1-3 further comprising exposing the embryos or cells of the animal *in vitro* to a single stranded DNA that contains an exogenous sequence. 4. The method of any of 1 or 3-4 comprising the cells, and further comprising culturing the cells and isolating colonies of the cells, wherein a test cell is taken from an isolated colony and testing the test cell to determine how the cell was modified. 5. The method of 4 wherein the testing is a destructive process that destroys the test cell. 6. The method of 1 wherein the cell is a primary cell. 7. The method of 6 wherein the primary cell is a cell chosen from the group consisting of swine, cow, sheep, goat, chickens, rabbit, fish, zebrafish, dog, mouse, cat, mouse, rat, and laboratory animal. 8. The method of any of 1-7 comprising exposing a non-human primary cell in an *in vitro* culture or a non-human embryo to a nucleic acid encoding a TALEN, wherein the nucleic acid encodes an N-terminal leader portion having at least 80% homology to SEQ ID NO:132. 9. The method of any of 1-8 comprising exposing the cells to the TALEN without a reporter gene, with more than 1% of the cells incorporating the modification at the targeted chromosomal site. 10. The method of any of 1-9 exposing the cells to the TALEN without a reporter gene, creating colonies of clonal cells, and testing a subset of members of the colonies to identify colonies incorporating the modification at the targeted chromosomal site. 11. The method of any of 1-10 wherein the genetic modification is chosen from the group consisting of an insertion, deletion, inversion or translocation. 12. The method of any of 1-11 wherein the TALEN is a first TALEN and the targeted chromosomal site is a first site, with the method further comprising a second TALEN directed to a second targeted chromosomal site. 13. The method of any of 1-12 further comprising exposing the embryos or cells to single stranded DNA (ssDNA) that contains an exogenous sequence, with the genetic modification comprising the exogenous sequence. 14. The method of any of 1-13 wherein the exogenous sequence comprises an alternative allele for the TALEN targeted chromosomal site. 15. The method of 14 wherein the alternative allele comprises a myostatin allele present in *Belgian Blue* cattle. 16. The method of any of 1-15 wherein the allele is chosen from the group consisting of an insertion, a deletion, a polymorphism, and a single nucleotide polymorphism. 17. The method of any of 1-16 wherein the targeted chromosomal site is chosen for a disruption of a gene, wherein the disruption of the gene comprises an insertion, deletion, or substitution of one or more bases in a sequence encoding the gene and/or a cis-regulatory element thereof. 18. The method of any of 1-17 further comprising delivering a recombinase to the cell or embryo. 19. The method of any of 1-18 wherein the TALEN mRNA is directly introduced into the cell as mRNA. 20. The method of any of 1-19 wherein the TALEN mRNA is introduced into the cell as a plasmid that encodes the mRNA. 21. The method of 20 wherein the ssDNA is introduced into the cell after a vector encoding a TALEN is introduced into the cell. 22. The method of 20 or 21 wherein ssDNA is introduced into the cell between about 8 hours and about 3 days after the vector expressing a TALEN is introduced into the cell. 23. The method of any of 20, 21, or 22 wherein TALEN mRNA is directly introduced into the cell at about the same time as the ssDNA. 24. The method of any of 1-23 with the cells being primary somatic cells or stem cells. 25. A method of creating a genetic modification comprising: exposing a non-human primary cell in an *in vitro* culture or a non-human embryo to a nucleic acid encoding a TALEN, wherein the nucleic acid encodes an N-terminal leader portion having at least 80% homology to SEQ ID NO:132. 26. The method of 25 wherein the N-terminal leader portion has the 80% homology to the 22-residue sequence portion of SEQ ID NO:132 and a total of no more than about 30 residues. 27. The method of 25 or 26 with the nucleic acid having at least 90% homology to SEQ ID NO: 132. 28. A cell made by, or used in, a method of any process set forth within the Application.

### Examples

### Example 1 Genetically modified artiodactyl livestock (bovine) produced by direct injection of TALENs.

Three TALEN pairs were designed and assembled as described in Cermak et. al. (2011) Nuc. Acids Res. 39:e82 (Fig. 3) and mRNA was injected into the cytoplasm of bovine embryos at about 19 hours post fertilization. Injected embryos were cultured *in vitro* and collected at the blastocysts stage (Fig. 3). Individual blastocyst genomic DNA was amplified by whole genome amplification (WGA) (Carlson et al. (2011) Trangenic Res. 20:29) and screened for indels using the SURVEYOR nuclease assay (Fig. 3, 4A, and 4B). Cleavage products indicative of TALEN activity were observed in 10% of injected embryos (e.g., Fig. 3, 4A, and 4B). Mutations in the predicted region were confirmed in 2 blastocysts injected with either ACAN11 or ACAN12 TALEN pairs (Fig.3). A significant decrease in the developmental competence of TALEN-injected embryos was not observed. A second round of injections was then performed using the ACAN12 TALEN pair at mRNA dosages ranging from 10-100 ng/µl. Comparison of the blastocyst formation rate between rounds 1 (33%) and 2 (5%) (10 ng/µl conditions) revealed poor embryo quality. Despite the poor quality of the embryos, 12 putative mutants (27% of injected) were identified using the SURVEYOR assay. The genotypes of each SURVEYOR positive embryo were analyzed with 14 sequencing reads from cloned PCR products. Sequencing revealed mosaicism in gene modification. Indels were identified in 4 SURVEYOR positive embryos and of these, indel positive sequence reads accounted for 7-29% of the total reads for each embryo. Processes for the creation of animal founder lines based on embryo transfer are well known. TALEN treated embryos have were successfully transferred to surrogate cows to establish pregnancies. These results demonstrated that TALENs functioned in artiodactyl embryos. Gene modification of embryos with Zing Finger Nucleases (ZFNs) and TALENs has been reported for model organisms by direct injection of ZFN or TALEN mRNAs encoding a nuclease pair Geurts et al. (2009) Science 325:433; Carbery et al., (2010) Genetics 186:451; Mashimo et al. (2010) PLoS One 5:e8870; Tesson et al. (2011) Nature Biotechnol. 29:695.

### Example 3 Genetically modified artiodactyl livestock produced by genetic modification of bovine and swine somatic cells.

Several additional TALEN pairs were assembled for targets in pigs and cattle chosen based on either biomedical or agricultural relevance. Binding domains of six TALEN pairs were placed in the context of two previously described TALEN scaffolds (+231, Christian *et. al.* 2010 (*op cit)* and Carlson +63, see Miller *et. al.* 2011 (*op cit*)) (Fig. 5). Each TALEN pair was transfected into primary livestock fibroblasts, and genome modification was measured at day 3 by the SURVEYOR assay (Guschin, et al. (2010) Methods Mol. Biol. 649:247. The most active TALEN pairs, DMDE7.1 and ACAN12, displayed cleavage of 38% and 25% of chromosomes, respectively, and Sanger sequencing revealed an assortment of indels characteristic of NHEJ mediated DNA repair (Fig. 5 and Fig. 6). The TALENs scaffold had a significant effect on activity in fibroblasts. In total, 4 of 6 loci targeted with the +63 isoform cleaved at 3.5% or greater while only the DMDE7.1 TALEN pair cleaved above 1% in the +231 scaffold (Fig. 5). As noted in previous studies Doyon et al. (2010) Nature Methods 8:74; Miller, (2011) *op. cit.*)*,* a 72 hour incubation at 30°C after transfection also had a positive effect on activity, and was required for activity of 3 TALEN pairs. The success rate for generating active Carlson +63 TALEN pairs has been high. Data collected up to the time of filing shows that 23 of 36 (64%) TALEN pairs were detectably active (> 1.0% NHEJ) at 15 genes scattered across the pig and cow genome, on autosomes and both the X and Y chromosomes. Three quarters of the active pairs cleaved with high efficiency (19-40%) with an average modification level of 25%. Clonal processes for the creation of animal founder lines based on modified fibroblasts are well known.

### Example 4 Extended culture and indel enrichment by transposon co-transfection.

TALEN pairs were transfected into fibroblasts and cultured cells for 14+ days with or without transposon co-selection prior to measurement of modification levels. The results are summarized in Fig. 7. At day zero (D0), cells are transfected with a mixture of plasmids including an expression cassette for each TALEN (two plasmids), a transposon encoding a selection marker (a third plasmid, encoding puromycin, and a transposase-expression cassette (fourth plasmid). The TALEN plasmids are the main component (4-fold excess by mass) of each transfection. Transfected cells are cultured for 3 days at either 30 or 37 degrees Celsius prior to splitting, collection of a sample for SURVEYOR assay and re-plating for extended culture +/- selection for transposon integration. All cells are cultured at 37 degrees Celsius after day 3. Cells cultured for 14+ days are collected for SURVEYOR assay and cryopreserved for downstream applications, i.e., SCNT. For comparison, other fibroblasts were transfected by Nucleofection and percent NHEJ was measured at day 3, and in day 14+ non-selected (NS) and selected (S) populations. For comparison, fibroblasts were also transfected using cationic-lipids.

### Example 5 Isolation of mono- and bi-allelic KO clones.

Transgenic primary fibroblasts can be effectively expanded and isolated as colonies when plated with non-transgenic fibroblasts (feeder-cells) at standard densities (> 150 cells/cm2) and subjected to drug selection using the transposon co-selection technique applied above (Carlson et al. (2011) Transgenic Res. 20:1125). To evaluate this approach, puromycin resistant colonies for cells treated with six TALEN pairs were isolated and their genotypes evaluated by SURVEYOR assay or direct sequencing of PCR products spanning the target site (Fig. 8A and 8B). In general, the proportion of indel positive clones was similar to predictions made based on day 3 modification levels. Bi-alleic knockout clones were identified for 6 of 7 different TALEN pairs, occurring in up to 35 percent of indel positive cells (Table 1). In the majority of examples, indels were homozygous (same indel on each allele) rather than unique indels on each allele suggesting that sister chromatid-templated repair is common (Fig. 9). Notably, among modified clones, the frequency of bi-alleic modification (17-60 %) for the majority of TALEN pairs exceed predictions based on day 3 modification levels (10-17%) if chromosome cleavages are treated as independent events. Previous studies with ZFN (Kim et al. (2009) Genome Res. 19:1279; Lee et al. (2010) Genome Res. 20:81; Perez et al. (2008) Nature Biotechnol. 26:808) are in support of these results. These previous studies suggested that bi-allelic modification occurs in nearly one-third of modified cells independent of the nuclease activity level.

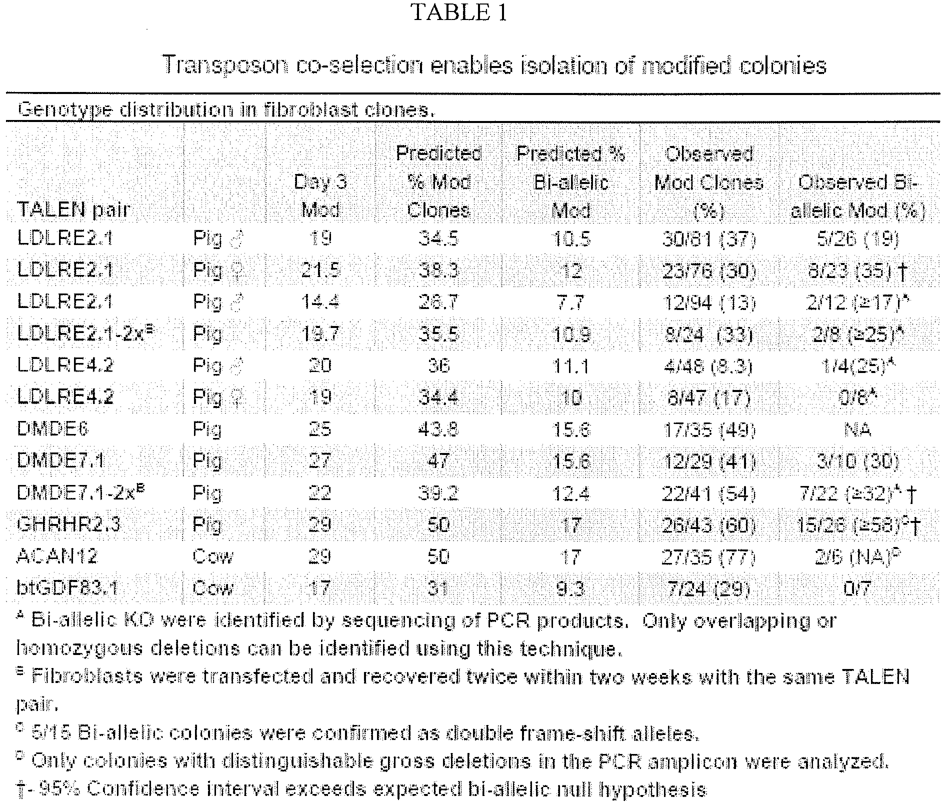

### Example 6 Chromosomal deletions and inversions with TALENs.

It was hypothesized that simultaneous delivery of two TALEN pairs targeting the same chromosome could induce large chromosomal deletions. These were achieved and, further, large inversions were incidentally discovered. The TALEN pairs, DMDE6 and DMDE7.1 were tested because of their high activity and the fact that a high percentage of Duchene's Muscular Dystrophy is caused by gross deletions (Blake, 2002) such that a porcine model would match to the human condition. The results are summarized in Fig. 10. Day 3 gene modification levels were high for each TALEN pair (24% for DMDE6 and 23% DMDE7.1), albeit slightly lower that when either TALEN pair was transfected individually (Fig. 10). To determine if the sequence between the two TALEN pairs had been deleted, PCR was attempted with primers spanning the TALEN target sites. If the 6.5 kb sequence had been removed, a band of ∼500 bp was expected, whereas the wild type band of 7 kb would not be amplified with the PCR conditions chosen. A band near 500 bp was observed in replicates where both TALEN pairs were introduced, but was absent when either TALEN pair was introduced alone (Fig. 10).

Next, the cell population was assayed for inversion events by PCR across presumptive new 5' and 3' junctions. Products were observed at the expected size for both the 5' and 3' junctions of the presumptive inversion only when both TALEN pairs were introduced (Fig. 10). To validate further that the inversions, colonies were generated using the transposon co-selection strategy and screened for deletion and inversion events. Both deletion and inversion events were recovered with high frequency (10.3% and 4.1% respectively; n > 1000). Deletion and inversion events occurred with remarkable fidelity. Forty one out of 43 of the inversion positive colonies were positive at both the 5' and 3' junctions. Finally, sequencing of PCR products confirmed both deletion and inversion events with addition or deletion of very few nucleotides at their junctions (Fig. 11, 12).

### Example 7 TALEN-induced homologous recombination eliminates need for linked selection markers.

A mutant myostatin allele (an 11 bp deletion) from Belgian Blue cattle was placed into the genome of wild-type Wagyu cattle (Grobet et al. (1997) Nature Genet. 17:71) (Fig. 13). When transfected alone, the btGDF8.1 TALEN pair cleaved up to 16% of chromosomes at the target locus (Fig. 13). TALENs (btGDF83.1) and a dsDNA template (BB-HDR) were designed to introduce an 11-bp deletion in exon-3 of bovine GDF8 (Belgium Blue mutation) by DSB induced homologous recombination. Half of the binding site for the left TALEN was missing in the BB-HDR template, to make it resistant to TALEN cleavage. A SURVEYOR assay demonstrated activity of btGDF83.1 TALENs at both 37 and 30° Celsius. The PCR product used for this assay was generated using primers b and b' (shown panel a of Fig. 13). The BB-HDR template was not included in these replicates since it would confound estimates of btGDF83.1 activity. Allele specific PCR demonstrated that HDR induction was dependent on co-transfection of TALENs and the BB-HDR template. The PCR assay was developed to specifically detect HDR modified GDF8 alleles using primers c and c' (shown panel a of Fig. 13). The 3' end of primer c' spanned the 11-bp deletion, and cannot amplify the wild type allele "wt". Five hundred cell equivalents were included in each PCR reaction including the positive control "C". Percent HDR was determined by comparative densitometry between experimental and control reactions. Co-transfection with a supercoiled DNA template harboring a 1623bp DNA fragment from Belgian Blue cattle resulted in a gene conversion frequency (HDR) of 0.5% to 5% as suggested by semi-quantitative PCR at day 3, without selection for the desired event (Fig. 13). These results demonstrated that TALENs can be used to effectively place exogenous nucleic acid sequences in livestock, including alleles - and without markers. To assess the frequency of placement in individual colonies, the transposon co-selection strategy was implemented to isolate and expand individual colonies for DNA sequencing. Gene conversion using template from Belgian Blue cattle was detected in 5 colonies out of 366 examined by PCR. Amplification with primers outside the Belgian Blue HDR template and sequencing confirmed the presence of the expected 11 bp deletion in 4 of the colonies (Fig. 14). A second repeat experiment was performed with consistent results, with about 1% of all tested colonies being positive for bi-allelic conversion and about 0.5% to about 1% of all tested colonies being heterozygous for allele conversion.

### Example 8 TALEN mediated DNA cleavage as a target for HDR in livestock cells.

A TALEN pair (LDLR4.2) targeted to the fourth exon of the swine low density lipoprotein receptor (LDLR) gene was co-transfected with the supercoiled plasmid *Ldlr*-E4N-stop, which contains homology arms corresponding to the swine LDLR gene and a gene-trap enabling expression of Neomycin phosphotransferase upon HDR (Figure 15). After 3 days of culture PCR analysis revealed that, even without antibiotic selection, a band corresponding to an HDR event can be detected at the targeted locus (lane 4) at 30°C. Selection of populations of cultured cells for 14 days with geneticin (G418) resulted in significant enrichment of HDR cells (lanes 11 & 13).

### Example 9 TALEN activity in Bovine zygotes.

This Example compares results obtained with the Carlson +63 TALENS to a previously known +231 scaffold. The methods described in Example 1 were followed. Table 2 summarizes the results using the GDF83.1 TALEN pair targeted to exon 3 of the bovine GDF8 locus, with the GDF83.1 being based on the Carlson +63 scaffold. Mutation frequency using the CARLSON +63 TALENs significantly exceeded previous injections. Six of 14 blastocysts (43%) injected with a low mRNA dosage (2ng/µl) displayed indels without a significant reduction in development rate. Three of four blastocysts in the high dosage group (10ng/µl) displayed indels, with bi-allelic modification occurring in 2 of 3 mutant blastocysts (Table 3).

**Table 2: TALEN activity in bovine zygotes.**

| Target | Trial | Scaffold | mRNA total ng/ul | Number Inj. | Blast rate | SURVEYOR Candidates/ assayed | NHEJ Confirmed | | |
|---|---|---|---|---|---|---|---|---|---|
| Non inj. | 1 | - | - | 60 | 41% | - | - | 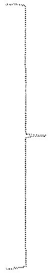 | Trial 1 |
| Buffer | 1 | - | - | 68 | 36% | - | - | | |
| ACAN11 | 1 | 231 | 10 | 67 | 22% | 2/24 | 1/2 | | |
| ACAN11 | 1 | 231 | 2 | 87 | 28% | 1/32 | 0/1 | | |
| ACAN12 | 1 | 231 | 10 | 57 | 33% | 0/22 | - | | |
| ACAN12 | 1 | 231 | 2 | 54 | 37% | 1/23 | 1/1 | | |
| PRNP3.2 | 1 | 231 | 10 | 65 | 14% | 0/19 | - | | |
| PRNP3.2 | 1 | 231 | 2 | 50 | 30% | 0/17 | - | | |
| Subtotal- | | | | 380 | | 4 (3%) | 2/4 | | |
| | | | | | | | (1.5% assayed) | | |
| ACAN12 | 2 | 231 | 10 | 59 | 5% | 1/10 | 0/1 | 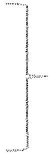 | Trial 2 |
| ACAN12 | 2 | 231 | 25 | 58 | 16% | 3/16 | 2/3 | | |
| ACAN12 | 2 | 231 | 50 | 59 | 2% | 2/9 | 1/2 | | |
| ACAN12 | 2 | 231 | 100 | 51 | 0% | 1/10 | 1/1^{a} | | |
| Subtotal- | | | | 227 | | 7 (16%) | 4/7 | | |
| | | | | | | | (9% assayed) | | |
| Non inj. | 3 | - | - | 51 | 43% | - | - | 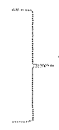 | Trial 3 |
| Buffer | 3 | - | - | 35 | 23% | - | - | | |
| GDF83.1 | 3 | GT | 2^{b} | 62 | 24% | - | 6/14 | | |
| GDF83.1 | 3 | GT | 10^{b} | 53 | 8% | - | 3/4^{c} | | |
| Subtotal- | | | | 328 | | | 9/18 | | |
| | | | | | | | (50% assayed) | | |

| | | | | | | | | | |
|---|---|---|---|---|---|---|---|---|---|
| a- 3 indels in one embryo b- eGFP mRNA was added to a final concentration of 2 ng/ul. c- two bi-allelic modification ACAN- Aggrecan, candidate for model of congenital achondroplasia. PRNP- Major prion protein, implicated in spongiform encephalopathy. GDF8- Growth differentiation factor 8 (myostatin), regulator of muscle growth. | | | | | | | | | |

**Table 3: Indels for GDF83.1 Bi-allelic modification**

| SEQ ID NOS | | SEQUENCES | |
|---|---|---|---|
| 147 | wt. | ACTCCACAGAATCTCGATGCTGTCGTTACCCTCTAACTGTGGATT | |
| 148 | 1a. | ACTCCACAGAATCTCGATGCT:::GTTACCCTCTAACTGTGGATT | X9 |
| 135 | 1b. | ACTCCACAGAATCTCGATG:::::::::::CTCTAACTGTGGATT | X2 |
| 136 | 1c. | ACTCCACAGAA TCTCGATGC::::::TACCCTCTAACTGTGGATT | X1 |
| 137 | 2a. | ACTCCACAGAAT:::::::::::::::::CCTCTAACTGTGGATT | X3 |
| 138 | 2b. | ACTCCACAGAATCTCGATGCT:::GTTACCTCTAACTGTGGATT | X1 |
| 139 | 2c. | ACTCCACAGAATCTCGATGCT:TCGTTACCCTCTAACTGTGGATT | X2 |
| | | 2/9 Bi-allelic | |

### Example 10 Cloning of TALEN-modified cells.

Cells from Example 5 that were modified with LDLR2 TALEN pairs were grown as clones. Transposon co-selected Ossabaw swine colonies with mono- and bi-allelic modification of the Class A domain 1 of the *LDLR* gene were pooled disproportionately (pools *A* - *4 genotypes, B* - *3 genotypes and C* - *5 genotypes*) and cloned by chromatin transfer. Pregnancy was established in 7/9 transfers (1/2 for pool *A,* 2/3 for pool *B,* and 4/4 for pool *C*). Seven of the 9 sows became pregnant, and 6 of the 7 pregnant sows had live births. 17 piglets were born that appear to be in good health for purposes of raising to maturity. The piglets had various genotypes, referred to as B1, B2, C1 and C2 in Table 4, below. Two of the genotypes were deletions, one was a single base insertion and one genotype had modifications of both alleles, an insertion in one allele and deletion in the other.

**Table 4**

| SEQ ID NO | SEQUENCE | |
|---|---|---|
| 140 | **Wt:** | **CTCCTACAAGTGGATT**GTGATGGGAACACCGA**GTGCAAGGACGGGTCCG** |
| | **B1:** (289_290INS34; 285_287delATG) 10 born; 9 live | |
| 141 | 1. | **CTCCTACAAGTGGATTT**GTGATGGGA i34 ACACCGA**GTGCAAGGACGGGTCCG** |
| 142 | 2. | **CTCCTACAAGTGGATTT**GTG:::GGAACACCGA**GTGCAAGGACGGGTCCG** |
| | **B2:** (211_292del128) One stillborn | |
| 143 | 1. | AGGGAGTATGGTCAC:::::::Δ128::ACCG**AGTGCAAGGACGGGTCCG** |
| | **C1:** (289_290del10) 3 born (one stillborn, one euthanized due to clonedefects) | |
| 144 | 1. | **CTCCTACAAGTGGATTTGT**GATGGG::::::::::**GCAAGGACGGGTCCG** |
| | **C2:** (289_290insA) 8 born; 8 live | |
| 145 | 1. | **CTCCTACAAGTGGATTT**GTGATGGG**A**AACACCGA**GTGCAAGGACGGGTCCG** |

### Example 11 An adeno-associated virus (AAV) is an effective template for TALEN stimulated homologous recombination (HR).

In another study, similar to Example 7, there was an experimental attempt to introgress a mutant myostatin allele (11bp deletion) from Belgian Blue cattle into the genome of wild-type *Wagyu* cattle (Grobet, 1997, Kambadur, 1997) (Fig. 18). Four micrograms of TALEN encoding plasmids were transfected into Wagyu cells and 24 hours later an adeno-associated virus (AAV-BB-HDR) harboring a 1,623bp DNA fragment from Belgian Blue cattle was added. Semi-quantitative PCR at day three suggests an allele conversion frequency of up to 5% only when both GDF8 TALENs and the AAV vector were added. To assess the frequency of introgression in individual colonies, the transposon co-selection strategy was implemented to isolate and expand individual colonies for DNA sequencing. Thirteen percent of isolated colonies were PCR positive for introgression of the BB allele. These results demonstrate that TALENs and an AAV homologous recombination template is an effective method for targeted allele introgression in livestock and represents a significant improvement over supercoiled plasmid homologous recombination template for the same locus (Example 7).

### Example 12 Single stranded DNA for templating.

Figure 19 summarizes the results. Single stranded oligodeoxynucleotides (ssODNs) were found to be an effective template for TALEN stimulated HR. It has recently been demonstrated that zinc finger nuclease induced double stand breaks can stimulate HR with a ssODNs at high frequency {Chen, 2011}. It was not known if TALENS could similarly stimulate homologous recombination in any cells, in particular primary cells, and in particular primary livestock cells with ssODNs as the HR template. The same loci as above (Examples 7 and 11) were targeted to introgress the 11 base pair Belgian Blue cattle mutation into Wagyu cells. Two 76 base pair ssODNs were designed to mimic either the sense or antisense strand of the BB GDF8 gene including the 11 base pair deletion. Four micrograms of TALEN encoding plasmids were transfected into Wagyu cells, and 0.3 nMol of ssODNs were either co-transfected with TALENS (N) or delivered 24 hours after TALEN nucleofection by either MirusLT1 (M) reagent or Lipofectamine LTX reagent (L). Semi-quantitative PCR at day three suggests an allele conversion frequency of up to 5% in conditions where ssODNs were delivered with LIPOFECTAMINE LTX reagent 24 hours after TALEN transfection. No difference in PCR signal was observed between sense and antisense ssODNs designed against the target.

### Example 13 Transfection of livestock cells with mRNAs encoding TALENs results in efficient target cleavage.

Fig. 20 summarizes the results. TALEN cDNA's (TALEN pairs p6511.1 and DMD7.1) were cloned downstream of the T3 promoter in the pT3TS cloning vector transcribed as previously described (Carlson, 2010) and purified using the MINELUTE PCR purification kit (Qiagen) prior to mRNA synthesis using the MMESSAGE MACHINE T3 kit (Applied Biosciences) according to the manufacturers protocol. Modified mRNA was synthesized from the same vectors with the MMESSAGE MACHINE T3 kit (Applied Biosciences) substituting a ribonucleotide cocktail consisting of 3'-0-Me-m⁷G(5')ppp(5')G RNA cap analog (New England Biolabs), 5-methylcytidine triphosphate pseudouridine triphosphate (TriLink Biotechnologies, San Diego, CA) and two standard ribonucleotides, adenosine triphosphate and guanosine triphosphate. mRNA synthesis reactions were DNAse treated prior to purification using the MEGACLEAR REACTION CLEANUP kit (Applied Biosciences). a) The indicated quantities of p6511.1 TALENs were transfected into pig fibroblasts (500,000-750,000 cells per replicate) using the NEON nucleofection system (Life Technologies) with the following settings: 1 pulse, 1800 v; 20 ms width and a 100 ul tip. Transfected cells were culture 3 days at either 30 or 37 degrees Celsius prior to indel analysis by the SURVEYOR assay (Transgenomic). Percent NHEJ was calculated as described in Guischin et. al., 2010, and plotted on the graph. Four micrograms of plasmid DNA (pDNA) encoding the p6511.1 TALENs was also transfected under the same conditions for comparison of %NHEJ. b) mRNA structure, composition or in vitro synthesis reaction scheme have little effect on TALEN activity. mRNA encoding the DMD7.1 TALENs was synthesized either by individually ("*I"* left and right TALENs in a separate reaction) or in the same reaction (Dual "*D*") using standard or modified ribonucleotides. The reactions were then split into two replicates, one of which an additional polyA tail was added using the Poly(A) Tailing Kit (Ambion) according to the manufacturers protocol.

Expression of TALENs from plasmid DNA has been an effective method for induction of TALEN mediated indels in livestock cells; however, integration of the TALEN encoding plasmids into the genomes of cells is possible. In contrast, mRNA cannot integrate into the genomes of host cells. To avoid the integration of TALEN encoding plasmids, an experiment was performed to determine if similar levels of TALEN activity could be achieved by transfection of mRNAs encoding TALENs. mRNA for TALENs encoding the p6511.1 TALEN pair was generated using either standard or modified ribonucleotides. Two quantities of each TALEN mRNA preparation were transfected into pig fibroblasts by nucleofection, cultured 3 days at 30 or 37 degrees Celsius prior to analysis of indels. Percent NHEJ was similar for all mRNA transfections incubated at 30 degrees Celsius while a dosage response could be observed for transfected cells incubated at 37 degrees Celsius. A significant difference in percent NHEJ between modified and standard ribonucleotides could not be detected in this replicate, however, equivalent quantities were not used. Notably, mRNA transfection in all groups incubated at 30 degrees C significantly outperformed the p6511.1 TALENs transfected as plasmid DNA under the same conditions.

Another experiment was performed to examine the influence of modified versus standard nucleotide synthesized mRNA at a second locus, porcine DMD. This experiment also evaluated whether addition of a polyA tail influenced TALEN activity, and whether each TALEN monomer (left and right monomers) could be synthesized in the same transcription reaction (Dual) or if they must be synthesized individually and mixed prior to transfection. One or four micrograms of DMD7.1 TALEN mRNA were transfected into pig fibroblasts and cultured 3 days at 30 or 37 degrees Celsius. As with the p6511.1 TALENs, little difference was observed in TALEN activity in cells cultured at 30 degrees Celsius suggesting that neither modified nucleotides, in vitro poly adenylation of mRNAs or dual transcription of mRNAs had an influence on activity. A dosage response could again be observed in the 37 degree cultured replicates as 4 µg of mRNA outperformed 1 µg transfections. Also, polyadenylated mRNAs appeared to outperform non adenlyated mRNAs in 37 degree replicates.

Notably when plasmid DNA encoding the DMD7.1 TALENs was transfected into pig fibroblasts, a significant reduction (40-60%) in %NHEJ levels measured at day 3 versus cells cultured to day 14 was noticed (Example 4). No such reduction in %NHEJ was observed for any of the mRNA transfected replicates shown here, data not shown for day 14 modification levels. Thus mRNA transfection appears to be superior to DNA transfection not only for TALEN activity, but also for maintaining a high proportion of modified cells after an extended period in culture. Without being bound to a particular theory, it is believed that this result is due to improved cell viability when transfected with mRNA versus plasmid DNA.

### Example 14 Analysis of colonies created by mRNA transfection with no selection.

The results of this Example are summarized in Table 5. One to four micrograms of mRNA encoding TALENs were added, as in Example 13, to bovine or swine primary fibroblasts. The cells were grown at 30°C for three days after exposure to TALENs and cells were enumerated and plated at a range of densities 1-20 cells/cm² on 10 cm dishes. Cells were cultured for 10-15 days until individual colonies of 3-4 mm in diameter could be observed. Colonies were aspirated with a p-200 pipettor under gentle aspiration and expelled into a well of 24-well plate with 500 µl of growth medium (Carlson, 2011). Plates with clearly defined colonies (∼10-30 / plate) were chosen for colony aspiration to limit the chance of aspirating cells from multiple colonies. Once a colony reached 70-90 percent confluent in the 24-well dish, a portion was harvested for indel analysis and the remainder was cryopreserved. The results of the indel analysis are located in the last five lines of Table 5. These results demonstrate that colonies can be readily isolated from TALEN mRNA transfected fibroblasts without the use of selection markers. Mutation frequency in analyzed clones were accurately predicted by the modification levels of the source population at day 3. Clones with bi-allelic modifications could also be readily identified.

**Table 5: Genotype distribution in fibroblast clones.**

| TALEN pair | Selection | | Day 3 Mod | Predicted % Mod Clones | Predicted % Bi-allelic Mod | Observed Mod Clones (%) | Observed Bi-allelic Mod (%) |
|---|---|---|---|---|---|---|---|
| LDLRE2.1 | Puro | Pig ♂ | 19 | 34.5 | 10.5 | 30/81 (37) | 5/26 (19) |
| LDLRE2.1 | Puro | Pig ♀ | 21.5 | 38.3 | 12 | 23/76 (30) | 8/23 (35) † |
| LDLRE2.1 | Puro | Pig ♂ | 14.4 | 26.7 | 7.7 | 12/94 (13) | 2/12 (≥17)^{A} |
| LDLRE2.1-2x^{B} | Puro | Pig | 19.7 | 35.5 | 10.9 | 8/24 (33) | 2/8 (≥25)^{A} |
| LDLRE4.2 | Puro | Pig ♂ | 20 | 36 | 11.1 | 4/48 (8.3) | ¼(25)^{A} |
| LDLRE4.2 | Puro | Pig ♀ | 19 | 34.4 | 10 | 8/47 (17) | 0/8^{A} |
| DMDE6 | Puro | Pig | 25 | 43.8 | 15.6 | 17/35 (49) | NA |
| DMDE7.1 | Puro | Pig | 27 | 47 | 15.6 | 12/29 (41) | 3/10 (30) |
| DMDE7.1-2x^{B} | Puro | Pig | 22 | 39.2 | 12.4 | 22/41 (54) | 7/22 (≥32)^{A} † |
| GHRHR2.3 | G-418 | Pig | 29 | 50 | 17 | 26/43 (60) | 15/26 (≥58)^{C}† |
| ACAN12 | Puro | Cow | 29 | 50 | 17 | 27/35 (77) | 2/6 (NA)^{D} |
| btGDF83.1 | Puro | Cow | 17 | 31 | 9.3 | 7/24 (29) | 0/7 |
| GHRHR2.3 | None | Pig ♂ | 32.5 | 55 | 19.4 | 21/25 (84) | 6/21 (≥29)^{A} |
| GHRHR2.3 | None | Pig ♀ | 35 | 58 | 21 | 13/13 (100) | 3/13 (≥23)^{A} |
| LDLR2.1 | None | Pig ♀ | 34 | 57 | 20 | 88/166 (53) | 5/16 (31%) |
| btGDF83.1 | None | Cow | 29 | 50 | 17 | 23/45 (51) | 2/23 (≥9)^{E} |
| btGDF83.1 | None | Cow | 35 | 58 | 21 | 23/41 (56) | 7/23 (≥30)^{E} |

| | | | | | | | |
|---|---|---|---|---|---|---|---|
| ^{A} Bi-allelic KO were identified by sequencing of PCR products. Only overlapping or homozygous deletions can be identified using this technique. ^{B} Fibroblasts were transfected and recovered twice within two weeks with the same TALEN pair. ^{C} 5/15 Bi-allelic colonies were confirmed as double frame-shift alleles. ^{D} Only colonies with distinguishable gross deletions in the PCR amplicon were analyzed. ^{E} Bi-allelic KO colonies were identified by high definition melt analysis. Only homozygous modifications can be identified. †- 95% Confidence interval exceeds expected bi-allelic null hypothesis | | | | | | | |

### Example 15 Co-transfection of mRNA encoded TALENs and ssODNs enhances HDR.

Figure 21 sets forth a summary of experimental results for modifying *Wagyu* cells with a combination of mRNA encoding TALENs and single-stranded oligonucleotides. The cells were *Waygu* cells and the allele was the *Belgian Blue.* Experimentation, cell type and locus assayed was the same as in Example 12 with the exception that TALEN encoding mRNA (2 ug) was delivered in place of DNA encoded TALENs (where indicated) and only the sense ssODN was used. No selection markers were introduced in these cells at any stage. Population analysis at day three revealed HDR in both DNA and mRNA transfected cells when the ssODN was introduced 24 hours after TALENs. Peak activity (∼10%) was observed in cells co-transfected with the ssODN and TALEN mRNA by nuclofection. This result contrasts the previous result with DNA encoding TALENs which were unable to stimulate HDR at a measurable frequency. Among individual colonies, prepared as in Example 13, both heterozygous and homozygous introgression of the *Belgian Blue* allele could be observed at 5 and 2 percent, respectively.

### Example 16 Introduction of single base alterations using ssODNs and mRNA encoding TALENs.

Figures 22 and 23 depict results of another study wherein a single nucleotide polymorphism was replicated. This polymorphism was known to cause a coding change to the bovine GDF8 locus, C313Y, known to cause hypermuscularity in Piedmontese cattle (Kambadur, 1997; Genome Res. 1997 7: 910-915 An additional SNP was introduced into the ssODN to create a silent EcoRI site to aid in screening and/or quantification of HDR. mRNA and ssODNs were introduced simultaneously by nucleofection as indicated in Examples 12 and 14 with relative quantities indicated. Figures 22 and 23 show results when transfected cells are incubated at 30 and 37 degrees Celsius for three days. Peak levels of homologous recombination were significantly higher at 30 degrees Celsius (11.3%) than at 37 (1.7%), despite similar activity of the TALENs. The data shows that from about 0.2 to about 0.4nmol ssDNA is effective for homologous recombination both at 30 and 37 degrees Celsius. Surprisingly, a bi-phasic effect was observed, with too great of an oligonucleotide concentration/amount abolishing the recombination. No selection markers were used in these cells at any stage.

### Example 17 Alleles introduced into pig (Ossabaw) cells using oligo HDR.

Figure 24 sets forth a summary of experimental results for modifying cells with a combination of mRNA encoded TALENs and single-stranded oligonucleotides to place an allele that occurs naturally in one species to another species (interspecific migration). Piedmontese GFD8 SNP C313Y, as in Example 16, was chosen as an example and was introduced into Ossabow swine cells. Experiments were performed as in Example 16 with Ossabaw swine cells substituted for Wagyu cells. No markers were used in these cells at any stage. A similar peak in HDR was observed between pig and cattle cells at 0.4 nmol ssODN, (Fig. 22, 23) however, HDR was not extinguished by higher concentrations of ssODN in Ossabaw fibroblasts.

### Example 18 Cloning for alleles introduced into cells using oligo HDR.

Fig. 25 sets forth a summary of experimental results for modifying cells with a combination of mRNA encoded TALENs and single-stranded oligonucleotides to place an allele into cells for cloning animals. A new allele (BamH1) was introduced into Ossabaw swine cells designed to introduce a 4 base pair insertion that would both create frame-shift allele and introduce a novel BamH1 site. Two or 1 micrograms of ssLDLR2.1 TALEN mRNA and 0.3 nmol of ssODN was introduced into Ossabaw cells as in Examples 12 and 14. Surprisingly, there was synergy between the ssODNs and the TALEN activity as the previous maximum for this TALEN pair without ssODN was 25% NHEJ. This synergy was unexpected and not predictable based on current understanding of the relevant molecular mechanisms. HDR was detected by restriction digest of PCR amplicons with BamH1. HDR levels were similar to NHEJ levels suggesting that the majority of TALEN induced breaks were repaired with the ssODN. Analysis of individual colonies, generated, as in example 14, revealed heterozygous and homozygous modification of up to 30 and 2.5 percent respectively. No selection markers were used in these animals at any stage. TALEN treated cells were cloned by chromatin transfer, implanted into surrogate sows and resulted in the establishment of pregnancies.

### Example 19 DNA and mRNA encoded TALENs are active in spermatigonial stem cells.

Results are summarized in Fig. 26. Porcine germ cells were isolated from 10 wk old boars, and enriched by differential. Plasmids encoding eGFP and DMD - specific TALENs were transfected into germ cells using the AMAXA NUCLEOFECTOR system Amaxa solutions "V"- and "L" and "B" using programs X-001 and X-005. Each transfection reaction was performed with 10⁶ of enriched germ cells, and indicated micrograms of TALEN encoding plasmid DNA. The same methods were used to deliver mRNAs encoding DMD7.1 TALENs. After nucleofection, they were cultured for 5 days in 5% CO₂ atmosphere at 37°C or 30°C. Transfection efficiency was evaluated by immunofluorescence analysis for co-localization of expression of GFP and UCH-L1. Cell viability was evaluated by trypan blue exclusion.

### Example 20 TALEN stimulated HDR in primordial germ cells.

TALEN stimulated HDR was also tested in chicken primordial germ cells (PGCs) at the chicken Ddx4 locus. Two TALEN pairs were constructed, on to intron 1 (Tal1.1) and exon 7 (Tal7.1) and their function was verified in DF1 chicken cells, see Figure 27. See also Example 8. Subsequently, each TALEN pair was co-transfected with the donor targeting vector designed to fuse GFP with Exon 2 of the Ddx4 gene (Panel b). As expected cleavage with Tal 1.1 stimulated homologous recombination (panel c) whereas Tal 7., which lies outside of the homologous sequence in the donor targeting vector, did not stimulate HDR.

### References

M. CHRISTIAN, et al., Targeting DNA double-strand breaks with TAL effector nucleases, 186 Genetics (2010).
J.C. MILLER, et al., A TALE nuclease architecture for efficient genome editing, 29 Nature Biotech. (2011).
D. A. MCGREW & K. L. KNIGHT, Molecular design and functional organization of the RecA protein, 38 Crit Rev Biochem Mol Biol (2003).
M. M. Cox, Recombinational DNA repair in bacteria and the RecA protein, 63 Prog Nucleic Acid Res Mol Biol (1999).
B. REISS, et al., RecA protein stimulates homologous recombination in plants, 93 Proc Natl Acad Sci U S A (1996).
B. REISS, et al., RecA stimulates sister chromatid exchange and the fidelity of double-strand break repair, but not gene targeting, in plants transformed by Agrobacterium, 97 Proc Natl Acad Sci U S A (2000).
O. G. SHCHERBAKOVA, et al., Overexpression of bacterial RecA protein stimulates homologous recombination in somatic mammalian cells, 459 Mutat Res (2000).
R. J. YANEZ & A. C. PORTER, Gene targeting is enhanced in human cells overexpressing hRAD51, 6 Gene Ther (1999).
Z. CUI, et al., RecA-mediated, targeted mutagenesis in zebrafish, 5 Mar Biotechnol (NY) (2003).
N. TAKAHASHI & I. B. DAWID, Characterization of zebrafish Rad52 and replication protein A for oligonucleotide-mediated mutagenesis, 33 Nucleic Acids Res (2005).
T. CERMAK, et al., Efficient design and assembly of custom TALEN and other TAL effector-based constructs for DNA targeting, (in press) Nucl. Acids Res. (2011).
D. F. CARLSON, et al., Strategies for selection marker-free swim transgenesis using the Sleeping Beauty transpason system, 20 Transgenic Res (2011).
A.M. GEURTS, et al., Knockout rats via embryo microinjection of zinc-finger nucleases, 325 Science (2009).
I.D. CARBERY, et al., Targeted genome modification in mice using zinc-finger nucleases, 186 Genetics (2010).
T. MASHIMO, et al., Generation of knockout rats with X-linked severe combined immunodeficiency (X-SCID) using zinc-finger nucleases, 5 PLoS One (2010).
L. TESSON, et al., Knockout rats generated by embryo microinjection of TALENs, 29 Nat Biotechnol (2011).
C. J. PALGRAVE, et al., Species-specific variation in RELA underlies differences in NF-kappaB activity: a potential role in African swine fever pathogenesis, 85 J Virol (2011).
C. MUSSOLINO, et al., A novel TALE nuclease scaffold enables high genome editing activity in combination with low toxicity, Nucleic Acids Res (2011).
D. Y. GUSCHIN, et al., A rapid and general assay for monitoring endogenous gene modification, 649 Methods Mol Biol (2010).
Y. DOYON, et al., Transient cold shock enhances zinc-finger nuclease-mediated gene disruption, 7 Nat Methods (2010).
H.J. KIM, et al., Targeted genome editing in human cells with zinc finger nucleases constructed via modular assembly, 19 Genome Res. (2009).
H. J. LEE, et al., Targeted chromosomal deletions in human cells using zinc finger nucleases, 20 Genome Res (2010).
E. E. PEREZ, et al., Establishment of HIV-1 resistance in CD4+ T cells by genome editing using zinc-finger nucleases, 26 Nat Biotechnol (2008).
D. J. BLAKE, et al., Function and genetics of dystrophin and dystrophin-related proteins in muscle, 82 Physiol Rev (2002).
L. GROBET, et al., A deletion in the bovine myostatin gene causes the double-muscled phenotype in cattle, 17 Nat Genet (1997).
R. KAMBADUR, et al., Mutations in myostatin (GDF8) in double-muscled Belgian Blue and Piedmontese cattle, 7 Genome Res (1997).

## Claims

1. A method of making a genetically edited livestock animal, said method comprising
exposing embryos, primary somatic cells, stem cells, germ cells to a single-stranded DNA (ssDNA) homology-dependent repair (HDR) template and an mRNA encoding a TALEN, with the TALEN specifically binding to a target chromosomal site in the embryos or cells,
cloning the cells in a surrogate mother or using the embryo for creating a genetically edited animal,
with the surrogate mother gestating an animal that is genetically edited at the target chromosomal site,
the method being performed without a reporter gene.

2. The method of claim 1 comprising exposing the embryos or the cells to the TALEN without a reporter gene, with more than about 1% of the embryos incorporating the modification at the targeted chromosomal site.

3. The method of claims 1 or 2 comprising exposing the cells to the TALEN without a reporter gene, creating colonies of clonal cells, and testing a subset of members of the colonies to identify colonies incorporating the modification at the targeted chromosomal site.

4. The method of any of claims 1 to 3 wherein the single stranded DNA (ssDNA) template contains an exogenous sequence, with the genetic modification comprising the exogenous sequence.

5. The method of claim 4 wherein the exogenous sequence comprises an alternative allele for the TALEN targeted chromosomal site.

6. The method of claim 5 wherein the alternative allele comprises a myostatin allele present in *Belgian Blue* cattle.

7. The method of any of claims 5 to 6 wherein the cell or embryo belongs to a first breed and the allele belongs to a second breed of the animal.

8. The method of claim 7 wherein the first breed is *Wagyu or Nelore* cattle and the second breed is *Belgian Blue* cattle, with the genetically edited animal having genetics to provide offspring that is a *Wagyu* or *Nelore* calf.

9. The method of any of claims 5 to 8 wherein the alternative allele
provides for an enhanced livestock trait, and is chosen from the group consisting of a horn polled locus, a gene recessive for fertility defects, a gene for enhancing meat production, a gene for enhancing dairy production, and combinations thereof; or
provides for an animal model, and is chosen from the group consisting of a gene for reduction of animal size, a gene that potentiate tumor growth, an oncogene, hypercholesterolemia genes, an inflammatory bowel disease gene, a spina bifida gene, a pulmonary hypertension gene, a gene causing a cardiac defects, and a celiac disease gene.

10. The method of any of claims 1 to 9 wherein the genetic edit is chosen from the group consisting of an insertion, a deletion, a change to an exogenous nucleic acid sequence, an inversion, a translocation, a gene conversion to natural allele, a gene conversion to a synthetic allele, interspecies allele migration, intraspecies allele migration, and a gene conversion to a novel allele.

11. The method of any of claims 1 to 10 wherein the cell is a primary somatic cell or stem cell and the method is performed without a selection step that requires either a positive or a negative survival selection criterion.

12. The method of any of claims 1 to 11 wherein the gestated animal is chosen from the group consisting of swine, cows sheep, goats, chicken, rabbit.

13. The method of any of claims 1 to 12 wherein the cell is chosen from the group consisting of a livestock cell, an artiodactyl cell, a cultured cell, a primary cell, a primary somatic cell, a zygote, a primordial germ cell, a stem cell, and a zygote, or wherein the embryo is a blastocyst.

14. The method of any of claims 1 to 13 wherein the gestated animal is a founder animal.

15. A method of making a genetically edited non-human animal cell or genetically edited non-human animal embryo comprising
exposing embryos or cells of the animal *in vitro* to a single-stranded DNA (ssDNA) homology-dependent repair (HDR) template and an mRNA encoding a TALEN, with the TALEN specifically binding to a targeted chromosomal site in the embryos or cells, with the cells or embryos being genetically modified only at the targeted chromosomal site and with the method being performed without a reporter gene.

## Patentansprüche

1. Verfahren zur Herstellung eines genetisch editierten Nutztiers, wobei das Verfahren umfasst:
das Exponieren von Embryonen, primären somatischen Zellen, Stammzellen, Keimzellen an eine einzelsträngige DNA (ssDNA)-Homologieabhängige-Reparatur (HDR)-Matrize und eine für eine TALEN codierende mRNA, wobei die TALEN spezifisch an eine chromosomale Zielstelle in den Embryonen oder Zellen bindet,
das Klonen der Zellen in einer Ersatzmutter oder das Verwenden des Embryos für die Erzeugung eines genetisch editierten Tieres,
wobei die Ersatzmutter ein Tier austrägt, das an der chromosomalen Zielstelle genetisch editiert ist,
wobei das Verfahren ohne Reportergen durchgeführt wird.

2. Verfahren von Anspruch 1, umfassend das Exponieren der Embryonen oder der Zellen an die TALEN ohne ein Reportergen, wobei bei mehr als etwa 1% der Embryonen die Modifikation an der angezielten chromosomale Stelle eingebracht wird.

3. Verfahren von Anspruch 1 oder 2, umfassend das Exponieren der Zellen an die TALEN ohne ein Reportergen, das Erzeugen von Kolonien klonaler Zellen und das Testen einer Untergruppe von Mitgliedern der Kolonien, um Kolonien zu identifizieren, bei denen die Modifikation an der angezielten chromosomalen Stelle eingebracht ist.

4. Verfahren von beliebigen der Ansprüche 1 bis 3, wobei die einzelsträngige DNA (ssDNA)-Matrize eine exogene Sequenz enthält, wobei die genetische Modifikation die exogene Sequenz umfasst.

5. Verfahren von Anspruch 4, wobei die exogene Sequenz ein alternatives Allel für die von der TALEN angezielte chromosomale Stelle umfasst.

6. Verfahren von Anspruch 5, wobei das alternative Allel ein Myostatin-Allel umfasst, das in *Belgian-Blue-*Rindern vorhanden ist.

7. Verfahren von beliebigen der Ansprüche 5 bis 6, wobei die Zelle oder das Embryo zu einer ersten Rasse gehört und das Allel zu einer zweiten Rasse des Tieres gehört.

8. Verfahren von Anspruch 7, wobei es sich bei der ersten Rasse um *Wagyu-* oder *Nelore*-Rinder handelt, und bei der zweiten Rasse um *Belgian-Blue*-Rinder, wobei das genetisch editierte Tier eine Genetik aufweist, um Nachkommen bereitzustellen, bei denen es sich um ein *Wagyu-* oder *Nelore*-Kalb handelt.

9. Verfahren von beliebigen der Ansprüche 5 bis 8, wobei das alternative Allel
ein verbessertes Nutzviehmerkmal bereitstellt und aus der Gruppe bestehend aus einem horn-polled-Locus, einem für Fertilitätsdefekte rezessiven Gen, einem Gen zur Steigerung der Fleischproduktion, einem Gen zur Steigerung der Milchproduktion und Kombinationen davon ausgewählt ist; oder
ein Tiermodell bereitstellt und aus der Gruppe bestehend aus einem Gen zur Reduktion der Tiergröße, einem Tumorwachstum potenzierenden Gen, einem Onkogen, Hypercholesterinämie-Genen, einem Gen für entzündliche Darmerkrankung, einem Spina-Bifida-Gen, einem Gen für pulmonale Hypertonie, einem Herzdefekte verursachenden Gen und einem Zöliakie-Gen ausgewählt ist.

10. Verfahren von beliebigen der Ansprüche 1 bis 9, wobei die genetische Editierung aus der Gruppe bestehend aus einer Insertion, einer Deletion, einer Änderung zu einer exogenen Nukleinsäuresequenz, einer Inversion, einer Translokation, einer Genkonversion zu einem natürlichen Allel, einer Genkonversion zu einem synthetischen Allel, einer Interspezies-Allel-Migration, einer Intraspezies-Allel-Migration und einer Genkonversion zu einem neuen Allel ausgewählt ist.

11. Verfahren von beliebigen der Ansprüche 1 bis 10, wobei die Zelle eine primäre somatische Zelle oder Stammzelle ist und das Verfahren ohne einen Selektionsschritt durchgeführt wird, der entweder ein positives oder ein negatives Überlebens-Selektionskriterium erfordert.

12. Verfahren von beliebigen der Ansprüche 1 bis 11, wobei das trächtige Tier aus der Gruppe bestehend aus Schweinen, Kühen, Schafen, Ziegen, Hühnern, Kaninchen ausgewählt ist.

13. Verfahren von beliebigen der Ansprüche 1 bis 12, wobei die Zelle aus der Gruppe bestehend aus einer Nutzvieh-Zelle, einer Paarhufer-Zelle, einer kultivierten Zelle, einer primären Zelle, einer primären somatischen Zelle, einer Zygote, einer primordialen Keimzelle, einer Stammzelle und einer Zygote ausgewählt ist, oder wobei der Embryo eine Blastozyste ist.

14. Verfahren von beliebigen der Ansprüche 1 bis 13, bei dem das trächtige Tier ein Gründertier ist.

15. Verfahren zur Herstellung einer genetisch editierten nicht-humanen Tierzelle oder eines genetisch editierten nicht-humanen Tierembryos, umfassend das In-vitro-Exponieren von Embryonen oder Zellen des Tieres an eine einzelsträngige DNA (ssDNA)-Homologieabhängige-Reparatur (HDR)-Matrize und eine für eine TALEN codierende mRNA, wobei die TALEN spezifisch an eine angezielte chromosomale Stelle in den Embryonen oder Zellen bindet, wobei die Zellen oder Embryonen nur an der angezielten chromosomalen Stelle genetisch modifiziert werden und wobei das Verfahren ohne ein Reporter-Gen durchgeführt wird.

## Revendications

1. Procédé de génération d'un animal d'élevage génétiquement modifié, ledit procédé comprenant les étapes consistant à
exposer des embryons, des cellules somatiques primaires, des cellules souches, des cellules germinales à une matrice d'ADN en simple brin (ADNsb) de réparation dépendante de l'homologie (HDR) et à un ARNm codant pour une TALEN, avec la TALEN se liant spécifiquement à un site chromosomique cible dans les embryons ou cellules,
cloner les cellules dans une mère porteuse ou en utilisant l'embryon pour créer un animal génétiquement modifié,
avec la mère porteuse portant en elle un animal qui est génétiquement modifié au niveau du site chromosomique cible,
le procédé étant exécuté sans gène rapporteur.

2. Procédé de la revendication 1 comprenant l'étape consistant à exposer les embryons ou les cellules à la TALEN sans gène rapporteur, avec plus de 1% environ des embryons incorporant la modification au niveau du site chromosomique ciblé.

3. Procédé des revendications 1 ou 2 comprenant les étapes consistant à exposer les cellules à la TALEN sans gène rapporteur, créer des colonies de cellules clonales et tester un sous-ensemble de membres des colonies afin d'identifier des colonies incorporant la modification au niveau du site chromosomique ciblé.

4. Procédé de l'une quelconque des revendications 1 à 3, dans lequel la matrice d'ADN en simple brin (ADNsb) contient une séquence exogène, avec la modification génétique comprenant la séquence exogène.

5. Procédé de la revendication 4, dans lequel la séquence exogène comprend un allèle alternatif pour le site chromosomique ciblé par TALEN.

6. Procédé de la revendication 5, dans lequel l'allèle alternatif comprend un allèle de myostatine présent dans un bovin Blanc-Bleu Belge.

7. Procédé de l'une quelconque des revendications 5 à 6, dans lequel la cellule ou l'embryon appartient à une première race et l'allèle appartient à une deuxième race de l'animal.

8. Procédé de la revendication 7, dans lequel la première race est un bovin *Wagyu* ou *Nelore* et la deuxième race est un bovin Blanc-Bleu Belge, avec l'animal génétiquement modifié ayant une génétique destinée à générer une progéniture qui est un veau *Wagyu* ou *Nelore.*

9. Procédé de l'une quelconque des revendications 5 à 8, dans lequel l'allèle alternatif
fournit un trait d'élevage renforcé, et est choisi dans le groupe constitué par un locus à cornes, un gène récessif pour des anomalies de la fertilité, un gène destiné à renforcer la production de viande, un gène destiné à renforcer la production de lait, ainsi que des combinaisons de ceux-ci ; ou
fournit un modèle animal, et est choisi dans le groupe constitué par un gène destiné à réduire la taille de l'animal, un gène qui potentialise la croissance tumorale, un oncogène, des gènes d'hypercholestérolémie, un gène de la maladie inflammatoire de l'intestin, un gène du spina bifida, un gène d'hypertension pulmonaire, un gène causant des anomalies cardiaques et un gène de la maladie cœliaque.

10. Procédé de l'une quelconque des revendications 1 à 9, dans lequel la modification génétique est choisie dans le groupe constitué par une insertion, une délétion, un changement au niveau d'une séquence exogène d'acides nucléiques, une inversion, une translocation, une conversion génique en un allèle naturel, une conversion génique en un allèle de synthèse, une migration d'allèle inter-espèces, une migration d'allèle intra-espèces ainsi qu'une conversion génique en un nouvel allèle.

11. Procédé de l'une quelconque des revendications 1 à 10, dans lequel la cellule est une cellule somatique primaire ou une cellule souche et le procédé est exécuté sans étape de sélection qui requiert un critère de sélection de survie soit positif soit négatif.

12. Procédé de l'une quelconque des revendications 1 à 11, dans lequel l'animal gestant est choisi dans le groupe constitué par un porc, des vaches, des moutons, des chèvres, un poulet, un lapin.

13. Procédé de l'une quelconque des revendications 1 à 12, dans lequel la cellule est choisie dans le groupe constitué par une cellule d'animal d'élevage, une cellule d'artiodactyle, une cellule cultivée, une cellule primaire, une cellule somatique primaire, un zygote, une cellule germinale primordiale, une cellule souche et un zygote, ou dans lequel l'embryon est un blastocyste.

14. Procédé de l'une quelconque des revendications 1 à 13, dans lequel l'animal gestant est un animal fondateur.

15. Procédé de génération d'une cellule d'animal non humain génétiquement modifié ou d'un embryon d'animal non humain génétiquement modifié, comprenant les étapes consistant à
exposer *in vitro* des embryons ou cellules de l'animal à une matrice d'ADN en simple brin (ADNsb) de réparation dépendante de l'homologie (HDR) et à un ARNm codant pour une TALEN, avec la TALEN se liant spécifiquement à un site chromosomique ciblé dans les embryons ou cellules, avec les cellules ou embryons étant génétiquement modifié(e)s seulement au niveau du site chromosomique ciblé et avec le procédé étant exécuté sans gène rapporteur.
